# EUROPEAN PATENT APPLICATION

(11) **EP 4 653 458 A1**
(43) Date of publication of application: **26.11.2025**
(21) Application number: 23917002.0
(22) Date of filing: 06.07.2023
(51) Int. Cl.: C07K 16/28, C12N 15/13, C12N 5/10, G01N 33/68, G01N 33/577, C12N 15/63

(54) **HUMAN ANTI-HUMAN PLA2R ANTIBODY STANDARD SUBSTANCE AND USE THEREOF**

(30) Priority: 18.01.2023 CN 202310061031
(71) Applicant: Chengdu Di'ao Pharmaceutical Group Co., Ltd., Chengdu, Sichuan 610041 (CN)
(72) Inventor: LAI, Bing, Chengdu, Sichuan 610041 (CN); HUANG, Yingchun, Chengdu, Sichuan 610041 (CN); YANG, Li, Chengdu, Sichuan 610041 (CN); ZHAI, Yixing, Chengdu, Sichuan 610041 (CN); XIAO, Jinfeng, Chengdu, Sichuan 610041 (CN)
(74) Representative: Dehns
(86) International application number: PCT/CN2023/106160
(87) International publication number: WO 2024/152529

(57) **Abstract**

Provided are a human anti-human PLA2R antibody or an antigen-binding fragment thereof. The antibody or the antigen-binding fragment thereof includes at least one CDR sequence selected from the following amino acid sequences or amino acid sequences having at least 80% identity thereto: a heavy chain variable region CDR sequence selected from SEQ ID NO: 1 to SEQ ID NO: 105; and a light chain variable region CDR sequence selected from SEQ ID NO: 106 to SEQ ID NO: 210.

## Description

### FIELD

The present disclosure relates to the field of bioengineering. Specifically, the present disclosure relates to a human anti-human PLA2R antibody standard and uses thereof. More specifically, the present disclosure relates to a human anti-human PLA2R antibody or an antigen-binding fragment thereof, a nucleic acid molecule, an expression vector, a recombinant cell, as well as a kit and use thereof.

### BACKGROUND

Primary membranous nephropathy is one of the important causes of kidney failure and the leading cause of the primary cause of nephrotic syndrome. Although no more than 30% of primary membranous nephropathy may spontaneously remit, about 30% of patients experience a slow progression of renal function to kidney failure within approximately 10 years, posing a serious threat to people's lives and health. Basic research in recent years has confirmed that primary membranous nephropathy is an autoimmune disease. The body's autoimmune antigens activate the immune system under disease conditions and produce autoimmune antibodies. The autoimmune antibodies bind to the autoimmune antigens on podocytes of the glomerulus, causing the immune system to attack podocytes and surrounding tissues, resulting in inflammation and damage to the glomerulus.

The autoimmune antigens of primary membranous nephropathy that have been studied more clearly include PLA2R and THSD7A. About 70% to 80% of primary membranous nephropathy is the PLA2R type, and less than 10% of primary membranous nephropathy is the THSD7A type.

The PLA2R antibody (PLA2R-Ab) plays an important role in the occurrence and development of PLA2R type membranous nephropathy. The level of PLA2R-Ab in serum can serve as a predictive marker for PLA2R-type membranous nephropathy. A higher titer of PLA2R-Ab means a lower chance of spontaneous remission. A decrease in the titer of PLA2R-Ab is often associated with spontaneous remission and becomes undetectable in patients with complete remission of the disease. The titer of PLA2R-Ab can be reduced by immunosuppressive therapy.

The concentration of the PLA2R antibody can be qualitatively or quantitatively detected by a diagnostic kit. The PLA2R antibody diagnostic kit usually uses immunological detection methods such as the ELISA method to detect the signal generated by the antibody in the sample, which is compared with the signal generated by the antibody standard to obtain the relative concentration. The standard used in the currently available PLA2R antibody diagnostic kit is diluted human serum, which has several major defects as a standard. First, the human serum standard is a relative concentration obtained by comparing it with the antibody in the standard human serum. Therefore, the absolute quantification of the chemical concentration of the antibody in the plasma is impossible, and only the relative unit RU can be obtained, which has limited value for clinical research and treatment, as well as the development of drugs and treatment methods. Secondly, even when the human serum standard has undergone several virus tests such as HIV and HCV, it cannot rule out that the human serum standard is contaminated by other viruses and pathogenic microorganisms. Therefore, there is a risk of pathogen contamination. Finally, the sources of the standard prepared from human serum are narrow and limited, leading to high production cost, and the antibodies derived from different patients may be difference, thereby likely leading to batch differences.

Therefore, it is urgent to provide a PLA2R antibody standard that is simple to prepare and less likely to have batch differences.

### SUMMARY

The present disclosure aims to solve at least one of the technical problems existing in the related art to a certain extent. To this end, the present disclosure provides an anti-human PLA2R antibody or an antigen-binding fragment thereof. The anti-human PLA2R antibody or the antigen-binding fragment thereof has a high affinity to a PLA2R protein, and has the advantages of simple preparation and being less likely to have batch differences. Therefore, it can be used as an anti-human PLA2R antibody standard.

The present disclosure is completed based on the inventors' following findings.

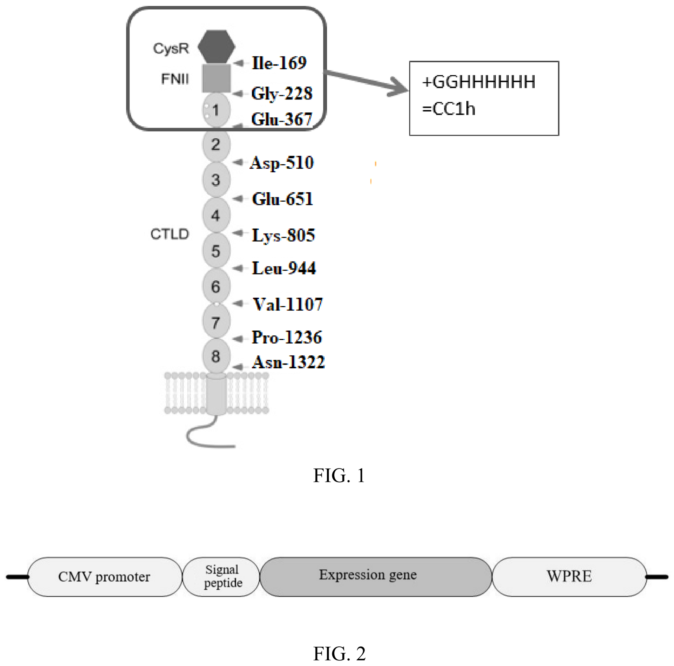

PLA2R is a membrane protein highly expressed on the surface of podocytes of the glomerulus and includes domains as illustrated in FIG. 1. Antibodies in the patient's plasma mainly recognize three domains of PLA2R, i.e., CysR, FNII, and CTLD1. Therefore, these three domains are designed and recombinantly expressed, and adopted as antigens. The human antibodies targeting these three domains are screened by using humanized recombinant phage libraries, and these antibodies are adopted as anti-human PLA2R antibody standards. By using a marketed diagnostic kit for calibration, a concentration conversion relationship between the anti-human PLA2R antibody standard and the antibody in human serum can be obtained. In addition, this antibody can be obtained by recombinant expression of mammalian cells, which can achieve a purpose of stable and large-scale production, improving the yield and quality of standard antibodies, reducing production costs, and eliminating the possibility of contamination by pathogenic microorganisms.

In an aspect of the present disclosure, the present disclosure provides an antibody or an antigen-binding fragment thereof. According to an embodiment of the present disclosure, the antibody or the antigen-binding fragment thereof includes at least one CDR sequence selected from the following amino acid sequences or amino acid sequences having at least 80% identity thereto: heavy chain variable region CDR sequences: SEQ ID NO: 1 to SEQ ID NO: 105; and light chain variable region CDR sequences: SEQ ID NO: 106 to SEQ ID NO: 210. The antibody or the antigen-binding fragment thereof has a high affinity to a PLA2R protein and can be used as an anti-human PLA2R antibody standard. This antibody or the antigen-binding fragment thereof can be obtained by cell recombination expression and has the advantages such as stable and large-scale production. Moreover, the antibody or the antigen-binding fragment thereof can be prevented from being contaminated by pathogenic microorganisms during a preparation process and the batch differences can be avoided, ensuring the purity and stability of the anti-human PLA2R antibody standard.

In another aspect of the present disclosure, the present disclosure provides a nucleic acid molecule. According to an embodiment of the present disclosure, the nucleic acid molecule encodes the above-mentioned antibody or antigen-binding fragment thereof. The nucleic acid molecule of the present disclosure can effectively express the above-mentioned antibody or antigen-binding fragment thereof.

In yet another aspect of the present disclosure, the present disclosure provides an expression vector. According to an embodiment of the present disclosure, the expression vector carries the above-mentioned nucleic acid molecule. The expression vector of the present disclosure can effectively express the above-mentioned antibody or antigen-binding fragment thereof.

In yet another aspect of the present disclosure, the present disclosure provides a recombinant cell. According to an embodiment of the present disclosure, the recombinant cell carries the above-mentioned nucleic acid molecule, or the recombinant cell expresses the above-mentioned antibody or antigen-binding fragment thereof. The recombinant cell according to the embodiment of the present disclosure can be used for *in vitro* expression and large-scale acquisition of the above-mentioned antibody or antigen-binding fragment thereof.

In yet another aspect of the present disclosure, the present disclosure provides use of the above-mentioned antibody or antigen-binding fragment thereof, the above-mentioned nucleic acid molecule, the above-mentioned expression vector, or the above-mentioned recombinant cell as a PLA2R antibody standard. As mentioned above, the above-mentioned antibody or antigen-binding fragment thereof has a high affinity to a PLA2R protein, can be obtained by recombinant expression of mammalian cells, and has the advantages such as stable and large-scale production, eliminating the possibility of contamination by pathogenic microorganisms during a preparation process. In this way, the above-mentioned antibody or antigen-binding fragment thereof can be used as an anti-human PLA2R antibody standard to improve the accuracy of PLA2R antibody detection.

In yet another aspect of the present disclosure, the present disclosure provides a kit. According to an embodiment of the present disclosure, the kit includes: the above-mentioned antibody or antigen-binding fragment thereof; the above-mentioned nucleic acid molecule; the above-mentioned expression vector; or the above-mentioned recombinant cell. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the kit containing the above-mentioned antibody or antigen-binding fragment thereof has the advantages such as high accuracy in the detection of the PLA2R antibody.

In yet another aspect of the present disclosure, the present disclosure provides use of the above-mentioned antibody or antigen-binding fragment thereof, the above-mentioned nucleic acid molecule, the above-mentioned expression vector, or the above-mentioned recombinant cell in the preparation of a kit for detecting a PLA2R antibody or diagnosing PLA2R antibody-positive membranous nephropathy. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the kit containing the above-mentioned antibody or antigen-binding fragment thereof has the advantages such as high accuracy in the detection of the PLA2R antibody.

In yet another aspect of the present disclosure, the present disclosure provides use of the above-mentioned antibody or antigen-binding fragment thereof, the above-mentioned nucleic acid molecule, the above-mentioned expression vector, or the above-mentioned recombinant cell in the detection of a PLA2R antibody or in the diagnosis of PLA2R antibody-positive membranous nephropathy. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the kit containing the above-mentioned antibody or antigen-binding fragment thereof has the advantages such as high accuracy in the detection of the PLA2R antibody.

In yet another aspect of the present disclosure, the present disclosure provides the above-mentioned antibody or antigen-binding fragment thereof, the above-mentioned nucleic acid molecule, the above-mentioned expression vector, or the above-mentioned recombinant cell, for use in the detection of a PLA2R antibody or the diagnosis of PLA2R antibody-positive membranous nephropathy. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the kit containing the above-mentioned antibody or antigen-binding fragment thereof has the advantages such as high accuracy in the detection of the PLA2R antibody.

In yet another aspect of the present disclosure, the present disclosure provides a method for detecting a PLA2R antibody. According to an embodiment of the present disclosure, the method includes: determining, based on a detection result of the PLA2R antibody, a content of the PLA2R antibody in a sample to be tested by using the above-mentioned antibody or antigen-binding fragment thereof as a PLA2R antibody standard. In this way, the accuracy of PLA2R antibody detection can be improved. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the method of the present disclosure has the advantages such as high accuracy in the detection of the PLA2R antibody.

In yet another aspect of the present disclosure, the present disclosure provides a method for diagnosing PLA2R antibody-positive membranous nephropathy. According to an embodiment of the present disclosure, the method includes: determining, based on a detection result of the PLA2R antibody in a sample to be tested, a content of the PLA2R antibody in the sample to be tested by using the above-mentioned antibody or antigen-binding fragment thereof as a PLA2R antibody standard; and determining, based on the content of the PLA2R antibody, whether a patient corresponding to the sample to be tested suffers from the PLA2R antibody-positive membranous nephropathy. In this way, the accuracy of PLA2R antibody detection can be improved. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard and can accurately detect the content of the PLA2R antibody. In this way, the method of the present disclosure has a high diagnostic accuracy for PLA2R antibody-positive membranous nephropathy.

Additional aspects and advantages of the present disclosure will be provided at least in part in the following description, or will become apparent at least in part from the following description, or can be learned from practicing of the present disclosure.

The amino acid sequence table of the present disclosure is as follows:

| Name | Amino acid sequence | SEQ ID NO: |
|---|---|---|
| VH-A2 | QVQLQQSGPGLVKPSQTLSLTCTVSGGSISSGGYYWSWIRQHPGKGLEWIGYIYYSGSTY YNPSLKSRVTISVDTSKNQFSLKLSSVTAADTAVYYCARQRRANGYSSSWEFDYWGQGT LVTVSS | 211 |
| VH-A5 | QVQLVQSGGGVVQPGNSLRLSCVASGFTFSRYTIHWVRQAPGKGLEWVAVISYDGSNKY YADSVKGRFTISRDNSKNTLYLQMNSLRAEDTAVYYCARGGPTPDWGQGTLVTVSS | 212 |
| VH-A8 | QVQLVQSGAEVKKPGSSVKVSCKASGGTFSSYAISWVRQAPGQGLEWMGGIIPIFGTANY AQKFQGRVTMTRDTSTSTVYMELSSLRSEDTAVYYCARDSDFWSGYSSMDVWGQGTTV TVSS | 213 |
| VH-A13 | QVQLLESGGGLVKPGGSLRLSCAASGFTFSSYSMNWVRQAPGKGLEWVSSISSGSSYIYY ADSVKGRFTISRDNAKNSLYLQMNSLRAEDTAVYYCARDQGASVTGTHTHWGQGTLVT VSS | 214 |
| VH-A21 | EVHLVESGPELVRPGASVTLSCKASGYTFTDYEMHWVKQTPVHGLEWIGAIDPETGGTA YNQKFKGKATLTADKSSSTAYMELRSLTSEDSAVYYCTTTYGNYWYFDVWGAGTLVTVS S | 215 |
| VH-A22 | EVQLVESGAEVKKPGSSVKVSCKASGGTFSSYAISWVRQAPGQGLEWMGGIIPIFGTANY AQKFQGRVTITADESTSTAYMELSSLRSEDTAVYYCAFTSGSYYYFDYWGQGTLVTVSS | 216 |
| VH-A23 | QVQLVQSGAEVKKPGSSVKVSCKASGGTFSSYAISWVRQAPGQGLEWMGGIIPIFGTANY AQKFQGRVTITADESTSTAYMELSSLRSEDTAVYYCARGGGGRGMTSDWGQGTLVTVSS | 217 |
| VH-A26 | QVQLLESGGGLVQPGGSLRLSCAASGFTFSSYAMSWVRQAPGKGLEWVSAISGSGGSTY YADSVKGRFTISRDNSKNTLYLQMNSLRAEDTAVYYCAKGTKMATIRLGYYYYGMDVW GQGTMVTVSS | 218 |
| VH-A44 | QVQLQESGPGLVKPSQTLSLTCTVSGGSISSGGYYWSWIRQHPGKGLEWIGYIYYSGSTY YNPSLKSRVTISVDTSKNQFSLKLSSVTAADTAVYYCARWGRGGSSWRFDIWGQGTMVT VSS | 219 |
| VH-A53 | EVQLLESGPGLVKPSQTLSLTCTVSGGSISSGGYYWSWIRQHPGKGLEWIGYIYYSGSTY YNPSLKSRVTISVDTSKNQFSLKLSSVTAADTAVYYCARDSRGRSYGMDVWGQGTMVT VSS | 220 |
| VH-A61 | QVQLVQSGAEVKKPGSSVKVSCKASGGTFSSYTISWVRQAPGQGLEWMGRIIPILGIANY AQKFQGRVTITADKSTSTAYMELSSLRSEDTAVYYCATGVGMVRGDPWGQGTLVTVSS | 221 |
| VH-A67 | QVQLVQSGGGVVQPGRSLRLPCAASGFTFSNYAMHWVRQAPGKGLEWVAVISYDGSKK YYADSVKGRFTISRDNSKNTLYLQVNSLRAEDTAVYYCATGYSSPWYGMDVWGQGTTV TVSS | 222 |
| VH-A143 | EVQLVETGGGVVQPGRSLRLSCAASGFTFSSYAMHWVRQAPGKGLEWVAVISYDGSNK YYADSVKGRFTISRDNSKNTLYLQMNSLRAEDTAVYYCARAAGHGILDYWGQGTLVTV SS | 223 |
| VH-A156 | RLQLQESGPGLVKPSQTLSLTCTVSGGSISSGGYYWSWIRQHPGKGLEWIGYIYYSGSTY YNPSLKSRVTISVDTSKNQFSLKLSSVTAADTAVYYCARIHHSTAGPYNNWYFDYWGQG TLVTVSS | 224 |
| VH-A187 | QVQLLDSGGGLVKPGGSLRLSCAASGFTFSSYGMHWVRQAPGKGLEWVAFISNDGSNK YYADSVKGRFTISRDNSKNTLYLQMNSLRAEDTAVYYCARLLRGPWNFDLWGRGTLVT VSS | 225 |
| VH-A378 | EVQLLESGGGLVQPGGSLRLSCAASGFTFSSYAMSWVRQAPGKGLEWVSAISGSGGSTY YADSVKGRFTISRDNSKNTLYLQMNSLRAEDTAVYYCATSSGPRIDAFDIWGQGTMVTVS S | 226 |
| VH-A382 | QVQLVQSGAEVKKPGASVKVSCKASGYTFTSYAMHWVRQAPGQRLEWMGWINAGNG | 227 |
| | NTKYSQKFQGRVTITRDTSASTAYMELSSLRSEDTAVYYCATSKSVWNFDYWGQGTLVT VSS | |
| VH-B16 | QVQLVQSGGGLVQPGGSLRLSCAASGFTFSSYAMSWVRQAPGKGLEWVSAISGSGGSTY YADSVKGRFTISRDNPKNTLFLQMTSLRSEDTAMYYCARSYGNYFTYWGQGTLVTVSS | 228 |
| VH-B20 | QVQLLESGPGLVKPSETLSLTCTVSGDSISNYFWSWIRQSPGKGLEWIGYIHYSGTVSYNP SLRSRVTISIDTSKNQFSLKLSSVTAADTAVYYCARRGRIAAAGTGYYYYGMDVWGQGT TVTVSS | 229 |
| VH-B24 | EVQLVETGGGLVKPGGSLRLSCAASGFTFSSYSMNWVRQAPGKGLEWVSSISSSSSYIYY ADSVKGRFTISRDNAKNSLYLQMNSLRAEDTAVYYCARGRRRDFDYWGQGTLVTVSS | 230 |
| VH-B29 | QVQLVQSGAEVKKPGSSVKVSCKASGDTFSSFAISWVRQAPGQGLEWMGTIIPLFTTPNY AQKFQGRVTITADESTRTAYMELSSLRSEDTAVYYCARGPERIAAAGYYYYYGMDVWG QGTTVTVSS | 231 |
| VH-B44 | QVQLVQSGAEVKKPGASVKVSCKASGYTFTSYYMHWVRQAPGQGLEWMGIINPSGGST SYAQKFQGRVTMTRDTSTSTVYMELSSLRSEDTAVYYCARGVSRRGAFDIWGQGTMVT VSS | 232 |
| VH-B53 | EVQLVETGGGVVQPGRSLRLSCAASGFTFSSYAMHWVRQAPGKGLEWVAVISYDGSNK YYADSVKGRFTISRDNSKNTLYLQMNSLRAEDTAVYYCARNRYGATDYWGQGTLVTVS S | 233 |
| VH-B67 | QITLKESGPGLVKPSGTLSLTCAVSGGSISSSNWWSWVRQPPGKGLEWIGEIYHSGSTNYN PSLKSRVTISVDKSKNQFSLKLSSVTAADTAVYYCARDDGWRYGSVYYYYMDVWGKGT TVTVSS | 234 |
| VH-B83 | EVQLLESGGGLVQPGGSLRLSCAASGFTFSSYAMSWVRQAPGKGLEWVSAISGSGGRTY YADSVKGRFTISRDNSKNTLYLQMNSLRAEDTAVYYCARVLQYSGTDDLDVWGRGTLV TVSS | 235 |
| VH-B88 | QVQLVQSGAEVKKPGSSVKVSCKASGGTFSSYTISWVRQAPGQGLEWMGRIIPILGIANY AQKFQGRVTITADKSTSTAYMELSSLRSEDTAVYYCATGVGMVRGDPWGQGTLVTVSS | 236 |
| VH-B90 | EVQLLESGGGLVKPGGSLRLSCAASGLIFKNYGMNWVRLAPGKGLEWVASISGNSNYIS YADSVKGRFTISRDNANNSLFLQMSSLRADDTAVYYCSSGYFYFDLWGPGTLVTVSS | 237 |
| VH-B109 | EVQLLESGGGLVKPGGSLRLSCAASGFTFSSYSMNWVRQAPGKGLEWVSSISSSSSYIYY ADSVKGRFTISRDNAKNSLYLQMNSLRDGDTAIYYCVRRLWEAPGIGPFDVWGHGTMV TVSS | 238 |
| VH-B114 | EVQLLESGGGLVQPGGSLKLSCAASGFTFSGYAMHWVRQAPGKGLEWVAVISYDGSNK YYADSVKGRFTISRDNSKNTLYLQMNSLRAEDTAVYYCARGRGRGVFDYWGQGTLVTV SS | 239 |
| VH-B117 | EVQLLESGGGLVQPGGSLRLSCAASGFTFSSYSMNWVRQAPGKGLEWVSYISSSSSTIYY ADSVKGRFTISRDNAKNSLYLQMNSLRDEDTAVYYCARDRTLGYWGQGTLVTVSS | 240 |
| VH-B123 | QVQLQQWGAGLLKPSQTLSLTCTVSGGSISSGGYYWSWIRQHPGKGLEWIGYIYYSGST YYNPSLKSRVTISVDTSKNQFSLKLSSVTAADTAVYYCARWDRVREGGRFSNAFDIWGQ GTMVTVSS | 241 |
| VH-B145 | QLQLQESGPGLVKPSQTLSLTCTVSGGSISSGGYYWSWIRQHPGKGLEWIGYIYYSGSTY YNPSLKSRVTISVDTSKNQFSLKLSSVTAADTAVYYCARGKRDGYNYYFDYWGQGTLVT VSS | 242 |
| VH-B156 | QVQLQQSGPGLVKPSQTLSLTCTVSGGSISSGGYYWSWIRQHPGKGLEWIGYIYYSGSTY YNPSLKSRVTISVDTSKNQFSLKLSSVTAADTAVYYCARQRRANGYSSSWEFDYWGQGT LVTVSS | 243 |
| VH-B201 | EVQLVESGGGLVQPGGSLRLSCAASGFTFSSYAMHWVRQAPGKGLEWVSAISGSGDSTY YADSVKGRFSISRDNSKNTLYLQMNSLRAEDTAVYYCAKGSRRSGFNLFDYWGQGTLVT VSS | 244 |
| VH-B307 | QLQLQESGPGLVKPSQTLSLTCTVSGGSISSGGYYWSWIRQHPGKGLEWIGYIYYSGSTY YNPSLKSRVTISVDTSKNQFSLKLSSVTAADTAVYYCARMGRIAASNYYGMDVWGQGTT VTVSS | 245 |
| VL-A2 | DIVMTQSPSSLSASVGDRVTITCRASQGISSALAWYQQKPGKAPKLLIYDASSLESGVPSR FSGSGSGTDFTLTISSLQPEDFATYYCQQFNSYPLTFGGGTKVEIK | 246 |
| VL-A5 | DIQMTQSPSSLSASVGDRVTITCRASQGIRNYLAWYQQKPGKVPRLLIFAASTLQSGVPSR FSGSGSGTDFTLTISSLQPEDVATYYCQKYYSAPFTFGPGTKVDIK | 247 |
| VL-A8 | DIVMTQSPLSLPVTLGQPASISCRSSQSLVHSDGNTYLNWFQQRPGQSPRRLIYKVSNRDS GVPDRFSGSGSGTDFTLKISRVEAEDVGVYYCMQGTQWPDTFGQGTKLEIK | 248 |
| VL-A13 | EIVMTQSPATLSLSPGERATLSCRASQSVSSYLAWYQQKPGQAPRLLIYDASNRATGIPAR FSGSGSGTDFTLTISSLEPEDFAVYYCQQRSNWPPMYTFGQGTKLEIK | 249 |
| VL-A21 | DIQLTQSPSSVSASVGDRVTITCRASQNIENWLAWYQQKPGKAPKLLIYATSRLENDVPSR FSGSRSGTEFILTISSLQPEDFATYYCHQANGFPWTFGPGTKVDIK | 250 |
| VL-A22 | DVVMTQSPLSLPVTLGQPASISCRSSQSLVHSDGNTYLNWFQQRPGQSPRRLIYKVSNRD SGVPDRFSGSGSGTDFTLKISRVEAEDVGVYYCMQGTHWPPRYTFGQGTKLEIK | 251 |
| VL-A23 | NIQLTQSPSSLSASVGDRVTITCRASQDISNYLAWFQQKPGKAPKSLIYAASSLQSGVPSRF SGSWSGTDFTLTISSLQPEDIATYYCQQYKRYPFIFGQGTKLEIK | 252 |
| VL-A26 | DIVMTQSPLSLPVTPGEPASISCRSSQSLLHSNGRNYLDWYLQKPGQSPQLLIYLGSNRAS | 253 |
| | GVSDRFSASGSGTDFTLQISRVEAEDVGIYYCMQALQTPWTFGQGTKVEVK | |
| VL-A44 | AIQMTQSPSSLSASVGDRVTITCRASQALTYTLAWYQQKPGKAPRLLIHDASSLESGVPSR FSGSGSGTDFTLTITSLQPEDFATYYCQQFRSYPLTFGGGTKVEIK | 254 |
| VL-A53 | DIQMTQSPSSLSASVGDRVTITCRASQSINNYLNWYQEKPGKAPKLLIYAASSLQSGVPSR FSGSGSGTDFTLTISSLQPEDFATYYCQQSYSYPFTFGPGTKVDIK | 255 |
| VL-A61 | DIVMTQSPLSLPVTLGQPASISCRSSQSLVKSDGNTYLSWFQQRPGQSPRRLIYKVSNRDS GVPDRFSGSGSGTDFTLKISRVEAEDVGVYYCMQGTHWPPTFGPGTKVDIK | 256 |
| VL-A67 | NIQMTQSPSSLSASVGDRITITCRASQSVNSYVNWYQQTPGQAPKLLIYAASSVHNGVPV RFSGSRSGTEFILTISSLQPEDFVTYFCQQSFTSPWTFGQGTKLEMK | 257 |
| VL-A143 | EIVLTQSPATLSLSPGERATLSCRASQSIARRIAWYQQQPGQAPRLLIYDASNRATGIPARFS GSGSGTDFTLTISSLEPEDFAIYYCQQRANWPLTFGPGTKVDIK | 258 |
| VL-A156 | AIQLTQSPSSLSASVGDRVTITCRASQGISSYLAWYQQKPGKAPKLLIFAASLLQRGVPSRF SGSGFGTDFTLTISGLQPEDFATYFCQQFNTYPITFGGGTKVEIK | 259 |
| VL-A187 | EIVMTQSPGTLSLSPGERATLSCRASQSVSSSYLAWYQQKPGQAPRLLIYGASSRATGIPD RFSGSGSGTDFTLTISRLEPEDFAVYYCQQYGSSPPITFGQGTRLEIK | 260 |
| VL-A378 | NIQMTQSPSSLTASVGDRITITCRASQGIRNSLAWYQQKPGTAPKLLVSAASKLQTGVPSR FSGSGSGTDYTLTISSLQTEDFATYYCQQYYGTPPTFGQGTKLEIK | 261 |
| VL-A382 | EIVLTQSPATLSLSPGERATLSCRASQGISSYLAWYQQKPGQAPRLLIYDASNRATGIPARF SGSGSGTDFTLTISNLEPEDFAVYYCQQRKNWPLTFGGGSKVEIK | 262 |
| VL-B16 | EIVLTQSPATLSLSPGERATLSCRASQSVSSYLAWYQQKPGQAPRLLIYDASNRATGIPARF SGSGSGTDFTLTISSLEPEDFAVYYCQQRSNWPPELTFGGGTKVEIK | 263 |
| VL-B20 | EIVMTQSPATLSLSPGERATLSCRASQSVSNYLAWYQQKPGQAPRLLIYGASSRATGIPDR FSGSGSGTDFTLTISRLEPEDFAVYYCQQYGSSLWTFGQGTKVEIK | 264 |
| VL-B24 | DIQMTQSPSSLPASVGDRVTITCRASQGIGTFLAWYQQHLGKAPKLLISASSTVQRGVPSR FSGSGSGTEFTLTINSLQPEDFATYYCQQLNNYPRTFGQGTKVEIK | 265 |
| VL-B29 | EIVMTQSPGTLSLSPGQRATLSCRASQSVSSSSLAWYQQKPGQAPRLLIHGASSRATGIPD RFSGSGSGTDFTLTISRLEPEDFAVYYCQQYGTSWTFGQGTKVEIK | 266 |
| VL-B44 | DVVMTQSPLSLPVTPGEPASISCRSSQSLLHRSGYNYLDWYVQKPGQSPQLLIYLGSIRAV GVPDRFSGSASGTDFTLRISRVEAEDVGIYYCMQALQTPPAFGGGTKVEIK | 267 |
| VL-B53 | NIQLTQSPSSLSASVGDRVTITCRASQGINSWLAWYQQKPGKAPKLLIYAASTLQSGVPSR FSGSGSGTDFTLTINSLQPEDFATYYCQQLNSFPFTFGPGTKVHIK | 268 |
| VL-B67 | DIQLTQSPSSLSASVGDRVTITCRASQSIDDHLNWYQQKPGKVPKLLISSASTLQSGAPSRF SGSGYGTDFTLTISSLQVEDFATYYCQQSYSPPQTFGQGTKVEIK | 269 |
| VL-B83 | DIQLTQSPSSLSASVGDRVTITCRASQSISSYLNWYQQKPGKAPKLLIYAASSLQSGVPSRF SGSGSGTDFTLTISSLQPEDFATYYCQQSYSTPRTFGQGTRLEIK | 270 |
| VL-B88 | DVVMTQSPLSLPVTLGQPASISCRSSQSLVYSDGNTYLNWFQQRPGQSPRRLIYKVSNRD SGVPDRFSGSGSGTDFTLKISRVEAEDVGVYYCMQGTHWPPTFGQGTKLEIK | 271 |
| VL-B90 | DIQMTQSPSILSASVGDRVTLTCRASQSISSWLAWYQQTPGKAPRVLIYEASTLEVGVPSR FSGSGSGTDFTLTISSLQPEDFGTYFCQQYHTDSRTFGQGTILEIK | 272 |
| VL-B109 | DIVMTQSPSSLSASVGDRVTITCRASQSISSYLNWYQQKPGKAPKLLIYAASSLQSGVPSR FSGSGSGTDFTLTISSLQPEDFATYYCQQSYSTLTFGGGTKVEIK | 273 |
| VL-B 114 | EIVLTQSPGTLSLSPGERATLSCRASQSVSKYLAWYQQKPGQAPRLLIYDVSNRATGIPAR FSGSGSGTDFTLTISSLEPEDFAVYYCQQRRSWPLTFGGGTKVEIR | 274 |
| VL-B 117 | NIQLTQSPSSVSASVGDRVTISCRASQGIGRWLAWYQQKPGKAPKLLIYAASNLQSGVPSR FSGSGSGTDFTLTISSLQPEDFATYYCQQANSFPRTFGPGTKVDIK | 275 |
| VL-B123 | NIQLTQSPSSLSASVGDRVTITCRASQGISTYLAWYQQKPGLAPKLLIYGASTLQSGVPSK FSGFGSGRDFTLTISSLQPEDIATYYCQQLNTYPPTFGQGTKLEIK | 276 |
| VL-B145 | DIVMTQSPSSLSASVGDRVTITCQASQDISNYLNWYQQKPGKAPKLLIYDASSLESGVPS KFSGSRSGTDFTLTISSLQPEDSATYYCQQYRSYPITFGGGTKVEIK | 277 |
| VL-B156 | DIQLTQSPSSLSASIGDRVTITCRASQGISSFLAWYQQKPGKAPQLLIYAASTLQSGVPSRFS GSGSGTEFTLTISSLQPEDFATYYCQQFKIYPLTFGGGTKVEVK | 278 |
| VL-B201 | EIVMTQSPGTLSLSPGERATLSCRASQSVSSSYLAWYQQKPGQAPRLLIYGASSRATGIPD RFSGSGSGTDFTLTISRLEPEDFAVYYCQQYGSSPPITFGQGTRLEIK | 279 |
| VL-B307 | DIQLTQSPSFLSASVGDRVTITCRASQGISSQLAWYQQEPGKAPKLLIYAASTLQSGVPSRF SGSGSGTEFTLTISSLQPEDFATYYCQQVNAYPLTFGPGTKVDIK | 280 |
| Heavy chain constant region-wild-type humanized IgG4 | ASTKGPSVFPLAPCSRSTSESTAALGCLVKDYFPEPVTVSWNSGALTSGVHTFPAVLQSSG LYSLSSVVTVPSSSLGTKTYTCNVDHKPSNTKVDKRVESKYGPPCPSCPAPEFLGGPSVFL FPPKPKDTLMISRTPEVTCVVVDVSQEDPEVQFNWYVDGVEVHNAKTKPREEQFNSTYR VVSVLTVLHQDWLNGKEYKCKVSNKGLPSSIEKTISKAKGQPREPQVYTLPPSQEEMTK NQVSLTCLVKGFYPSDIAVEWESNGQPENNYKTTPPVLDSDGSFFLYSRLTVDKSRWQEG NVFSCSVMHEALHNHYTQKSLSLSLGK | 281 |
| Heavy chain constant region-humanized IgG4 mutant | ASTKGPSVFPLAPCSRSTSESTAALGCLVKDYFPEPVTVSWNSGALTSGVHTFPAVLQSSG LYSLSSVVTVPSSSLGTKTYTCNVDHKPSNTKVDKRVESKYGPPCPPCPAPEFLGGPSVFL FPPKPKDTLMISRTPEVTCVVVDVSQEDPEVQFNWYVDGVEVHNAKTKPREEQFNSTYR VVSVLTVLHQDWLNGKEYKCKVSNKGLPSSIEKTISKAKGQPREPQVYTLPPSQEEMTK NQVSLTCLVKGFYPSDIAVEWESNGQPENNYKTTPPVLDSDGSFFLYSRLTVDKSRWQEG | 282 |
| | NVFSCSVMHEALHNHYTQKSLSLSLGK | |
| Heavy chain-A2 | QVQLQQSGPGLVKPSQTLSLTCTVSGGSISSGGYYWSWIRQHPGKGLEWIGYIYYSGSTY YNPSLKSRVTISVDTSKNQFSLKLSSVTAADTAVYYCARQRRANGYSSSWEFDYWGQGT LVTVSSASTKGPSVFPLAPCSRSTSESTAALGCLVKDYFPEPVTVSWNSGALTSGVHTFPA VLQSSGLYSLSSVVTVPSSSLGTKTYTCNVDHKPSNTKVDKRVESKYGPPCPSCPAPEFLG GPSVFLFPPKPKDTLMISRTPEVTCVVVDVSQEDPEVQFNWYVDGVEVHNAKTKPREEQ FNSTYRVVSVLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFLYSRLTVDKS RWQEGNVFSCSVMHEALHNHYTQKSLSLSLGK | 283 |
| Heavy chain-A5 | QVQLVQSGGGVVQPGNSLRLSCVASGFTFSRYTIHWVRQAPGKGLEWVAVISYDGSNKY YADSVKGRFTISRDNSKNTLYLQMNSLRAEDTAVYYCARGGPTPDWGQGTLVTVSSAST KGPSVFPLAPCSRSTSESTAALGCLVKDYFPEPVTVSWNSGALTSGVHTFPAVLQSSGLYS LSSVVTVPSSSLGTKTYTCNVDHKPSNTKVDKRVESKYGPPCPSCPAPEFLGGPSVFLFPP KPKDTLMISRTPEVTCVVVDVSQEDPEVQFNWYVDGVEVHNAKTKPREEQFNSTYRVV SVLTVLHQDWLNGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRLTVDKSRWQEGNV FSCSVMHEALHNHYTQKSLSLSLGK | 284 |
| Heavy chain-A8 | QVQLVQSGAEVKKPGSSVKVSCKASGGTFSSYAISWVRQAPGQGLEWMGGIIPIFGTANY AQKFQGRVTMTRDTSTSTVYMELSSLRSEDTAVYYCARDSDFWSGYSSMDVWGQGTTV TVSSASTKGPSVFPLAPCSRSTSESTAALGCLVKDYFPEPVTVSWNSGALTSGVHTFPAVL QSSGLYSLSSVVTVPSSSLGTKTYTCNVDHKPSNTKVDKRVESKYGPPCPSCPAPEFLGGP SVFLFPPKPKDTLMISRTPEVTCVVVDVSQEDPEVQFNWYVDGVEVHNAKTKPREEQFN STYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 285 |
| Heavy chain-A13 | QVQLLESGGGLVKPGGSLRLSCAASGFTFSSYSMNWVRQAPGKGLEWVSSISSGSSYIYY ADSVKGRFTISRDNAKNSLYLQMNSLRAEDTAVYYCARDQGASVTGTHTHWGQGTLVT VSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPSCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 286 |
| Heavy chain-A21 | EVHLVESGPELVRPGASVTLSCKASGYTFTDYEMHWVKQTPVHGLEWIGAIDPETGGTA YNQKFKGKATLTADKSSSTAYMELRSLTSEDSAVYYCTTTYGNYWYFDVWGAGTLVTVS SASTKGPSVFPLAPCSRSTSESTAALGCLVKDYFPEPVTVSWNSGALTSGVHTFPAVLQSS GLYSLSSVVTVPSSSLGTKTYTCNVDHKPSNTKVDKRVESKYGPPCPSCPAPEFLGGPSVF LFPPKPKDTLMISRTPEVTCVVVDVSQEDPEVQFNWYVDGVEVHNAKTKPREEQFNSTY RVVSVLTVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTVDKSRWQE GNVFSCSVMHEALHNHYTQKSLSLSLGK | 287 |
| Heavy chain-A22 | EVQLVESGAEVKKPGSSVKVSCKASGGTFSSYAISWVRQAPGQGLEWMGGIIPIFGTANY AQKFQGRVTITADESTSTAYMELSSLRSEDTAVYYCAFTSGSYYYFDYWGQGTLVTVSSA STKGPSVFPLAPCSRSTSESTAALGCLVKDYFPEPVTVSWNSGALTSGVHTFPAVLQSSGL YSLSSVVTVPSSSLGTKTYTCNVDHKPSNTKVDKRVESKYGPPCPSCPAPEFLGGPSVFLF PPKPKDTLMISRTPEVTCVVVDVSQEDPEVQFNWYVDGVEVHNAKTKPREEQFNSTYRV VSVLTVLHQDWLNGKEYKCKVSNKGLPSSIEKTISKAKGQPREPQVYTLPPSQEEMTKN QVSLTCLVKGFYPSDIAVEWESNGQPENNYKTTPPVLDSDGSFFLYSRLTVDKSRWQEGN VFSCSVMHEALHNHYTQKSLSLSLGK | 288 |
| Heavy chain-A23 | QVQLVQSGAEVKKPGSSVKVSCKASGGTFSSYAISWVRQAPGQGLEWMGGIIPIFGTANY AQKFQGRVTITADESTSTAYMELSSLRSEDTAVYYCARGGGGRGMTSDWGQGTLVTVSS ASTKGPSVFPLAPCSRSTSESTAALGCLVKDYFPEPVTVSWNSGALTSGVHTFPAVLQSSG LYSLSSVVTVPSSSLGTKTYTCNVDHKPSNTKVDKRVESKYGPPCPSCPAPEFLGGPSVFL FPPKPKDTLMISRTPEVTCVVVDVSQEDPEVQFNWYVDGVEVHNAKTKPREEQFNSTYR VVSVLTVLHQDWLNGKEYKCKVSNKGLPSSIEKTISKAKGQPREPQVYTLPPSQEEMTK NQVSLTCLVKGFYPSDIAVEWESNGQPENNYKTTPPVLDSDGSFFLYSRLTVDKSRWQEG NVFSCSVMHEALHNHYTQKSLSLSLGK | 289 |
| Heavy chain-A26 | QVQLLESGGGLVQPGGSLRLSCAASGFTFSSYAMSWVRQAPGKGLEWVSAISGSGGSTY YADSVKGRFTISRDNSKNTLYLQMNSLRAEDTAVYYCAKGTKMATIRLGYYYYGMDVW GQGTMVTVSSASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVESKYGPPCPSCPA PEFLGGPSVFLFPPKPKDTLMISRTPEVTCVVVDVSQEDPEVQFNWYVDGVEVHNAKTK PREEQFNSTYRVVSVLTVLHQDWLNGKEYKCKVSNKGLPSSIEKTISKAKGQPREPQVYT LPPSQEEMTKNQVSLTCLVKGFYPSDIAVEWESNGQPENNYKTTPPVLDSDGSFFLYSRLT VDKSRWQEGNVFSCSVMHEALHNHYTQKSLSLSLGK | 290 |
| Heavy chain-A44 | QVQLQESGPGLVKPSQTLSLTCTVSGGSISSGGYYWSWIRQHPGKGLEWIGYIYYSGSTY YNPSLKSRVTISVDTSKNQFSLKLSSVTAADTAVYYCARWGRGGSSWRFDIWGQGTMVT | 291 |
| | VSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPSCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | |
| Heavy chain-A53 | EVQLLESGPGLVKPSQTLSLTCTVSGGSISSGGYYWSWIRQHPGKGLEWIGYIYYSGSTY YNPSLKSRVTISVDTSKNQFSLKLSSVTAADTAVYYCARDSRGRSYGMDVWGQGTMVT VSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPSCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 292 |
| Heavy chain-A61 | QVQLVQSGAEVKKPGSSVKVSCKASGGTFSSYTISWVRQAPGQGLEWMGRIIPILGIANY AQKFQGRVTITADKSTSTAYMELSSLRSEDTAVYYCATGVGMVRGDPWGQGTLVTVSSA STKGPSVFPLAPCSRSTSESTAALGCLVKDYFPEPVTVSWNSGALTSGVHTFPAVLQSSGL YSLSSVVTVPSSSLGTKTYTCNVDHKPSNTKVDKRVESKYGPPCPSCPAPEFLGGPSVFLF PPKPKDTLMISRTPEVTCVVVDVSQEDPEVQFNWYVDGVEVHNAKTKPREEQFNSTYRV VSVLTVLHQDWLNGKEYKCKVSNKGLPSSIEKTISKAKGQPREPQVYTLPPSQEEMTKN QVSLTCLVKGFYPSDIAVEWESNGQPENNYKTTPPVLDSDGSFFLYSRLTVDKSRWQEGN VFSCSVMHEALHNHYTQKSLSLSLGK | 293 |
| Heavy chain-A67 | QVQLVQSGGGVVQPGRSLRLPCAASGFTFSNYAMHWVRQAPGKGLEWVAVISYDGSKK YYADSVKGRFTISRDNSKNTLYLQVNSLRAEDTAVYYCATGYSSPWYGMDVWGQGTTV TVSSASTKGPSVFPLAPCSRSTSESTAALGCLVKDYFPEPVTVSWNSGALTSGVHTFPAVL QSSGLYSLSSVVTVPSSSLGTKTYTCNVDHKPSNTKVDKRVESKYGPPCPSCPAPEFLGGP SVFLFPPKPKDTLMISRTPEVTCVVVDVSQEDPEVQFNWYVDGVEVHNAKTKPREEQFN STYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 294 |
| Heavy chain-A143 | EVQLVETGGGVVQPGRSLRLSCAASGFTFSSYAMHWVRQAPGKGLEWVAVISYDGSNK YYADSVKGRFTISRDNSKNTLYLQMNSLRAEDTAVYYCARAAGHGILDYWGQGTLVTV SSASTKGPSVFPLAPCSRSTSESTAALGCLVKDYFPEPVTVSWNSGALTSGVHTFPAVLQS SGLYSLSSVVTVPSSSLGTKTYTCNVDHKPSNTKVDKRVESKYGPPCPSCPAPEFLGGPSV FLFPPKPKDTLMISRTPEVTCVVVDVSQEDPEVQFNWYVDGVEVHNAKTKPREEQFNST YRVVSVLTVLHQDWLNGKEYKCKVSNKGLPSSIEKTISKAKGQPREPQVYTLPPSQEEM TKNQVSLTCLVKGFYPSDIAVEWESNGQPENNYKTTPPVLDSDGSFFLYSRLTVDKSRWQ EGNVFSCSVMHEALHNHYTQKSLSLSLGK | 295 |
| Heavy chain-A156 | RLQLQESGPGLVKPSQTLSLTCTVSGGSISSGGYYWSWIRQHPGKGLEWIGYIYYSGSTY YNPSLKSRVTISVDTSKNQFSLKLSSVTAADTAVYYCARIHHSTAGPYNNWYFDYWGQG TLVTVSSASTKGPSVFPLAPCSRSTSESTAALGCLVKDYFPEPVTVSWNSGALTSGVHTFP AVLQSSGLYSLSSVVTVPSSSLGTKTYTCNVDHKPSNTKVDKRVESKYGPPCPSCPAPEFL GGPSVFLFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQVYTLPPS QEEMTKNQVSLTCLVKGFYPSDIAVEWESNGQPENNYKTTPPVLDSDGSFFLYSRLTVDK SRWQEGNVFSCSVMHEALHNHYTQKSLSLSLGK | 296 |
| Heavy chain-A187 | QVQLLDSGGGLVKPGGSLRLSCAASGFTFSSYGMHWVRQAPGKGLEWVAFISNDGSNK YYADSVKGRFTISRDNSKNTLYLQMNSLRAEDTAVYYCARLLRGPWNFDLWGRGTLVT VSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPSCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 297 |
| Heavy chain-A378 | EVQLLESGGGLVQPGGSLRLSCAASGFTFSSYAMSWVRQAPGKGLEWVSAISGSGGSTY YADSVKGRFTISRDNSKNTLYLQMNSLRAEDTAVYYCATSSGPRIDAFDIWGQGTMVTVS SASTKGPSVFPLAPCSRSTSESTAALGCLVKDYFPEPVTVSWNSGALTSGVHTFPAVLQSS GLYSLSSVVTVPSSSLGTKTYTCNVDHKPSNTKVDKRVESKYGPPCPSCPAPEFLGGPSVF LFPPKPKDTLMISRTPEVTCVVVDVSQEDPEVQFNWYVDGVEVHNAKTKPREEQFNSTY RVVSVLTVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTVDKSRWQE GNVFSCSVMHEALHNHYTQKSLSLSLGK | 298 |
| Heavy chain-A382 | QVQLVQSGAEVKKPGASVKVSCKASGYTFTSYAMHWVRQAPGQRLEWMGWINAGNG NTKYSQKFQGRVTITRDTSASTAYMELSSLRSEDTAVYYCATSKSVWNFDYWGQGTLVT VSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPSCPAPEFLGGPS VFLFPPKPKDTLMISRTPEVTCVVVDVSQEDPEVQFNWYVDGVEVHNAKTKPREEQFNS | 299 |
| | TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | |
| Heavy chain-B16 | QVQLVQSGGGLVQPGGSLRLSCAASGFTFSSYAMSWVRQAPGKGLEWVSAISGSGGSTY YADSVKGRFTISRDNPKNTLFLQMTSLRSEDTAMYYCARSYGNYFTYWGQGTLVTVSSA STKGPSVFPLAPCSRSTSESTAALGCLVKDYFPEPVTVSWNSGALTSGVHTFPAVLQSSGL YSLSSVVTVPSSSLGTKTYTCNVDHKPSNTKVDKRVESKYGPPCPSCPAPEFLGGPSVFLF PPKPKDTLMISRTPEVTCVVVDVSQEDPEVQFNWYVDGVEVHNAKTKPREEQFNSTYRV VSVLTVLHQDWLNGKEYKCKVSNKGLPSSIEKTISKAKGQPREPQVYTLPPSQEEMTKN QVSLTCLVKGFYPSDIAVEWESNGQPENNYKTTPPVLDSDGSFFLYSRLTVDKSRWQEGN VFSCSVMHEALHNHYTQKSLSLSLGK | 300 |
| Heavy chain-B20 | QVQLLESGPGLVKPSETLSLTCTVSGDSISNYFWSWIRQSPGKGLEWIGYIHYSGTVSYNP SLRSRVTISIDTSKNQFSLKLSSVTAADTAVYYCARRGRIAAAGTGYYYYGMDVWGQGT TVTVSSASTKGPSVFPLAPCSRSTSESTAALGCLVKDYFPEPVTVSWNSGALTSGVHTFPA VLQSSGLYSLSSVVTVPSSSLGTKTYTCNVDHKPSNTKVDKRVESKYGPPCPSCPAPEFLG GPSVFLFPPKPKDTLMISRTPEVTCVVVDVSQEDPEVQFNWYVDGVEVHNAKTKPREEQ FNSTYRVVSVLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFLYSRLTVDKS RWQEGNVFSCSVMHEALHNHYTQKSLSLSLGK | 301 |
| Heavy chain-B24 | EVQLVETGGGLVKPGGSLRLSCAASGFTFSSYSMNWVRQAPGKGLEWVSSISSSSSYIYY ADSVKGRFTISRDNAKNSLYLQMNSLRAEDTAVYYCARGRRRDFDYWGQGTLVTVSSA STKGPSVFPLAPCSRSTSESTAALGCLVKDYFPEPVTVSWNSGALTSGVHTFPAVLQSSGL YSLSSVVTVPSSSLGTKTYTCNVDHKPSNTKVDKRVESKYGPPCPSCPAPEFLGGPSVFLF PPKPKDTLMISRTPEVTCVVVDVSQEDPEVQFNWYVDGVEVHNAKTKPREEQFNSTYRV VSVLTVLHQDWLNGKEYKCKVSNKGLPSSIEKTISKAKGQPREPQVYTLPPSQEEMTKN QVSLTCLVKGFYPSDIAVEWESNGQPENNYKTTPPVLDSDGSFFLYSRLTVDKSRWQEGN VFSCSVMHEALHNHYTQKSLSLSLGK | 302 |
| Heavy chain-B29 | QVQLVQSGAEVKKPGSSVKVSCKASGDTFSSFAISWVRQAPGQGLEWMGTIIPLFTTPNY AQKFQGRVTITADESTRTAYMELSSLRSEDTAVYYCARGPERIAAAGYYYYYGMDVWG QGTTVTVSSASTKGPSVFPLAPCSRSTSESTAALGCLVKDYFPEPVTVSWNSGALTSGVH TFPAVLQSSGLYSLSSVVTVPSSSLGTKTYTCNVDHKPSNTKVDKRVESKYGPPCPSCPAP EFLGGPSVFLFPPKPKDTLMISRTPEVTCVVVDVSQEDPEVQFNWYVDGVEVHNAKTKP REEQFNSTYRVVSVLTVLHQDWLNGKEYKCKVSNKGLPSSIEKTISKAKGQPREPQVYTL PPSQEEMTKNQVSLTCLVKGFYPSDIAVEWESNGQPENNYKTTPPVLDSDGSFFLYSRLTV DKSRWQEGNVFSCSVMHEALHNHYTQKSLSLSLGK | 303 |
| Heavy chain-B44 | QVQLVQSGAEVKKPGASVKVSCKASGYTFTSYYMHWVRQAPGQGLEWMGIINPSGGST SYAQKFQGRVTMTRDTSTSTVYMELSSLRSEDTAVYYCARGVSRRGAFDIWGQGTMVT VSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPSCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 304 |
| Heavy chain-B53 | EVQLVETGGGVVQPGRSLRLSCAASGFTFSSYAMHWVRQAPGKGLEWVAVISYDGSNK YYADSVKGRFTISRDNSKNTLYLQMNSLRAEDTAVYYCARNRYGATDYWGQGTLVTVS SASTKGPSVFPLAPCSRSTSESTAALGCLVKDYFPEPVTVSWNSGALTSGVHTFPAVLQSS GLYSLSSVVTVPSSSLGTKTYTCNVDHKPSNTKVDKRVESKYGPPCPSCPAPEFLGGPSVF LFPPKPKDTLMISRTPEVTCVVVDVSQEDPEVQFNWYVDGVEVHNAKTKPREEQFNSTY RVVSVLTVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTVDKSRWQE GNVFSCSVMHEALHNHYTQKSLSLSLGK | 305 |
| Heavy chain-B67 | QITLKESGPGLVKPSGTLSLTCAVSGGSISSSNWWSWVRQPPGKGLEWIGEIYHSGSTNYN PSLKSRVTISVDKSKNQFSLKLSSVTAADTAVYYCARDDGWRYGSVYYYYMDVWGKGT TVTVSSASTKGPSVFPLAPCSRSTSESTAALGCLVKDYFPEPVTVSWNSGALTSGVHTFPA VLQSSGLYSLSSVVTVPSSSLGTKTYTCNVDHKPSNTKVDKRVESKYGPPCPSCPAPEFLG GPSVFLFPPKPKDTLMISRTPEVTCVVVDVSQEDPEVQFNWYVDGVEVHNAKTKPREEQ FNSTYRVVSVLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFLYSRLTVDKS RWQEGNVFSCSVMHEALHNHYTQKSLSLSLGK | 306 |
| Heavy chain-B83 | EVQLLESGGGLVQPGGSLRLSCAASGFTFSSYAMSWVRQAPGKGLEWVSAISGSGGRTY YADSVKGRFTISRDNSKNTLYLQMNSLRAEDTAVYYCARVLQYSGTDDLDVWGRGTLV TVSSASTKGPSVFPLAPCSRSTSESTAALGCLVKDYFPEPVTVSWNSGALTSGVHTFPAVL QSSGLYSLSSVVTVPSSSLGTKTYTCNVDHKPSNTKVDKRVESKYGPPCPSCPAPEFLGGP SVFLFPPKPKDTLMISRTPEVTCVVVDVSQEDPEVQFNWYVDGVEVHNAKTKPREEQFN STYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 307 |
| Heavy chain-B88 | QVQLVQSGAEVKKPGSSVKVSCKASGGTFSSYTISWVRQAPGQGLEWMGRIIPILGIANY AQKFQGRVTITADKSTSTAYMELSSLRSEDTAVYYCATGVGMVRGDPWGQGTLVTVSSA STKGPSVFPLAPCSRSTSESTAALGCLVKDYFPEPVTVSWNSGALTSGVHTFPAVLQSSGL YSLSSVVTVPSSSLGTKTYTCNVDHKPSNTKVDKRVESKYGPPCPSCPAPEFLGGPSVFLF PPKPKDTLMISRTPEVTCVVVDVSQEDPEVQFNWYVDGVEVHNAKTKPREEQFNSTYRV VSVLTVLHQDWLNGKEYKCKVSNKGLPSSIEKTISKAKGQPREPQVYTLPPSQEEMTKN QVSLTCLVKGFYPSDIAVEWESNGQPENNYKTTPPVLDSDGSFFLYSRLTVDKSRWQEGN VFSCSVMHEALHNHYTQKSLSLSLGK | 308 |
| Heavy chain-B90 | EVQLLESGGGLVKPGGSLRLSCAASGLIFKNYGMNWVRLAPGKGLEWVASISGNSNYIS YADSVKGRFTISRDNANNSLFLQMSSLRADDTAVYYCSSGYFYFDLWGPGTLVTVSSAST KGPSVFPLAPCSRSTSESTAALGCLVKDYFPEPVTVSWNSGALTSGVHTFPAVLQSSGLYS LSSVVTVPSSSLGTKTYTCNVDHKPSNTKVDKRVESKYGPPCPSCPAPEFLGGPSVFLFPP KPKDTLMISRTPEVTCVVVDVSQEDPEVQFNWYVDGVEVHNAKTKPREEQFNSTYRVV SVLTVLHQDWLNGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRLTVDKSRWQEGNV FSCSVMHEALHNHYTQKSLSLSLGK | 309 |
| Heavy chain-B109 | EVQLLESGGGLVKPGGSLRLSCAASGFTFSSYSMNWVRQAPGKGLEWVSSISSSSSYIYY ADSVKGRFTISRDNAKNSLYLQMNSLRDGDTAIYYCVRRLWEAPGIGPFDVWGHGTMV TVSSASTKGPSVFPLAPCSRSTSESTAALGCLVKDYFPEPVTVSWNSGALTSGVHTFPAVL QSSGLYSLSSVVTVPSSSLGTKTYTCNVDHKPSNTKVDKRVESKYGPPCPSCPAPEFLGGP SVFLFPPKPKDTLMISRTPEVTCVVVDVSQEDPEVQFNWYVDGVEVHNAKTKPREEQFN STYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 310 |
| Heavy chain-B114 | EVQLLESGGGLVQPGGSLKLSCAASGFTFSGYAMHWVRQAPGKGLEWVAVISYDGSNK YYADSVKGRFTISRDNSKNTLYLQMNSLRAEDTAVYYCARGRGRGVFDYWGQGTLVTV SSASTKGPSVFPLAPCSRSTSESTAALGCLVKDYFPEPVTVSWNSGALTSGVHTFPAVLQS SGLYSLSSVVTVPSSSLGTKTYTCNVDHKPSNTKVDKRVESKYGPPCPSCPAPEFLGGPSV FLFPPKPKDTLMISRTPEVTCVVVDVSQEDPEVQFNWYVDGVEVHNAKTKPREEQFNST YRVVSVLTVLHQDWLNGKEYKCKVSNKGLPSSIEKTISKAKGQPREPQVYTLPPSQEEM TKNQVSLTCLVKGFYPSDIAVEWESNGQPENNYKTTPPVLDSDGSFFLYSRLTVDKSRWQ EGNVFSCSVMHEALHNHYTQKSLSLSLGK | 311 |
| Heavy chain-B117 | EVQLLESGGGLVQPGGSLRLSCAASGFTFSSYSMNWVRQAPGKGLEWVSYISSSSSTIYY ADSVKGRFTISRDNAKNSLYLQMNSLRDEDTAVYYCARDRTLGYWGQGTLVTVSSASTK GPSVFPLAPCSRSTSESTAALGCLVKDYFPEPVTVSWNSGALTSGVHTFPAVLQSSGLYSL SSVVTVPSSSLGTKTYTCNVDHKPSNTKVDKRVESKYGPPCPSCPAPEFLGGPSVFLFPPK PKDTLMISRTPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQEEMTKNQV SLTCLVKGFYPSDIAVEWESNGQPENNYKTTPPVLDSDGSFFLYSRLTVDKSRWQEGNVF SCSVMHEALHNHYTQKSLSLSLGK | 312 |
| Heavy chain-B123 | QVQLQQWGAGLLKPSQTLSLTCTVSGGSISSGGYYWSWIRQHPGKGLEWIGYIYYSGST YYNPSLKSRVTISVDTSKNQFSLKLSSVTAADTAVYYCARWDRVREGGRFSNAFDIWGQ GTMVTVSSASTKGPSVFPLAPCSRSTSESTAALGCLVKDYFPEPVTVSWNSGALTSGVHT FPAVLQSSGLYSLSSVVTVPSSSLGTKTYTCNVDHKPSNTKVDKRVESKYGPPCPSCPAPE FLGGPSVFLFPPKPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKGQPREPQVYTLP PSQEEMTKNQVSLTCLVKGFYPSDIAVEWESNGQPENNYKTTPPVLDSDGSFFLYSRLTV DKSRWQEGNVFSCSVMHEALHNHYTQKSLSLSLGK | 313 |
| Heavy chain-B145 | QLQLQESGPGLVKPSQTLSLTCTVSGGSISSGGYYWSWIRQHPGKGLEWIGYIYYSGSTY YNPSLKSRVTISVDTSKNQFSLKLSSVTAADTAVYYCARGKRDGYNYYFDYWGQGTLVT VSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPSCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 314 |
| Heavy chain-B156 | QVQLQQSGPGLVKPSQTLSLTCTVSGGSISSGGYYWSWIRQHPGKGLEWIGYIYYSGSTY YNPSLKSRVTISVDTSKNQFSLKLSSVTAADTAVYYCARQRRANGYSSSWEFDYWGQGT LVTVSSASTKGPSVFPLAPCSRSTSESTAALGCLVKDYFPEPVTVSWNSGALTSGVHTFPA VLQSSGLYSLSSVVTVPSSSLGTKTYTCNVDHKPSNTKVDKRVESKYGPPCPSCPAPEFLG GPSVFLFPPKPKDTLMISRTPEVTCVVVDVSQEDPEVQFNWYVDGVEVHNAKTKPREEQ FNSTYRVVSVLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFLYSRLTVDKS RWQEGNVFSCSVMHEALHNHYTQKSLSLSLGK | 315 |
| Heavy chain-B201 | EVQLVESGGGLVQPGGSLRLSCAASGFTFSSYAMHWVRQAPGKGLEWVSAISGSGDSTY YADSVKGRFSISRDNSKNTLYLQMNSLRAEDTAVYYCAKGSRRSGFNLFDYWGQGTLVT VSSASTKGPSVFPLAPCSRSTSESTAALGCLVKDYFPEPVTVSWNSGALTSGVHTFPAVLQ | 316 |
| | SSGLYSLSSVVTVPSSSLGTKTYTCNVDHKPSNTKVDKRVESKYGPPCPSCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | |
| Heavy chain-B307 | QLQLQESGPGLVKPSQTLSLTCTVSGGSISSGGYYWSWIRQHPGKGLEWIGYIYYSGSTY YNPSLKSRVTISVDTSKNQFSLKLSSVTAADTAVYYCARMGRIAASNYYGMDVWGQGTT VTVSSASTKGPSVFPLAPCSRSTSESTAALGCLVKDYFPEPVTVSWNSGALTSGVHTFPAV LQSSGLYSLSSVVTVPSSSLGTKTYTCNVDHKPSNTKVDKRVESKYGPPCPSCPAPEFLGG PSVFLFPPKPKDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPREPQVYTLPPSQE EMTKNQVSLTCLVKGFYPSDIAVEWESNGQPENNYKTTPPVLDSDGSFFLYSRLTVDKSR WQEGNVFSCSVMHEALHNHYTQKSLSLSLGK | 317 |
| Light chain-A2 (light chain-A2 (IgG4SP)) | DIVMTQSPSSLSASVGDRVTITCRASQGISSALAWYQQKPGKAPKLLIYDASSLESGVPSR FSGSGSGTDFTLTISSLQPEDFATYYCQQFNSYPLTFGGGTKVEIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 318 |
| Light chain-A5 (light chain-A5 (IgG4SP)) | DIQMTQSPSSLSASVGDRVTITCRASQGIRNYLAWYQQKPGKVPRLLIFAASTLQSGVPSR FSGSGSGTDFTLTISSLQPEDVATYYCQKYYSAPFTFGPGTKVDIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 319 |
| Light chain-A8 | DIVMTQSPLSLPVTLGQPASISCRSSQSLVHSDGNTYLNWFQQRPGQSPRRLIYKVSNRDS GVPDRFSGSGSGTDFTLKISRVEAEDVGVYYCMQGTQWPDTFGQGTKLEIKRTVAAPSV FIFPPSDEQLKSGTASVVCLLNNFYPREAKVQWKVDNALQSGNSQESVTEQDSKDSTYSL SSTLTLSKADYEKHKVYACEVTHQGLSSPVTKSFNRGEC | 320 |
| Light chain-A13 (light chain-A13 (IgG4SP)) | EIVMTQSPATLSLSPGERATLSCRASQSVSSYLAWYQQKPGQAPRLLIYDASNRATGIPAR FSGSGSGTDFTLTISSLEPEDFAVYYCQQRSNWPPMYTFGQGTKLEIKRTVAAPSVFIFPPS DEQLKSGTASVVCLLNNFYPREAKVQWKVDNALQSGNSQESVTEQDSKDSTYSLSSTLT LSKADYEKHKVYACEVTHQGLSSPVTKSFNRGEC | 321 |
| Light chain-A21 | DIQLTQSPSSVSASVGDRVTITCRASQNIENWLAWYQQKPGKAPKLLIYATSRLENDVPSR FSGSRSGTEFILTISSLQPEDFATYYCHQANGFPWTFGPGTKVDIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 322 |
| Light chain-A22 | DVVMTQSPLSLPVTLGQPASISCRSSQSLVHSDGNTYLNWFQQRPGQSPRRLIYKVSNRD SGVPDRFSGSGSGTDFTLKISRVEAEDVGVYYCMQGTHWPPRYTFGQGTKLEIKRTVAAP SVFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC | 323 |
| Light chain-A23 | NIQLTQSPSSLSASVGDRVTITCRASQDISNYLAWFQQKPGKAPKSLIYAASSLQSGVPSRF SGSWSGTDFTLTISSLQPEDIATYYCQQYKRYPFIFGQGTKLEIKRTVAAPSVFIFPPSDEQL KSGTASVVCLLNNFYPREAKVQWKVDNALQSGNSQESVTEQDSKDSTYSLSSTLTLSKA DYEKHKVYACEVTHQGLSSPVTKSFNRGEC | 324 |
| Light chain-A26 | DIVMTQSPLSLPVTPGEPASISCRSSQSLLHSNGRNYLDWYLQKPGQSPQLLIYLGSNRAS GVSDRFSASGSGTDFTLQISRVEAEDVGIYYCMQALQTPWTFGQGTKVEVKRTVAAPSV FIFPPSDEQLKSGTASVVCLLNNFYPREAKVQWKVDNALQSGNSQESVTEQDSKDSTYSL SSTLTLSKADYEKHKVYACEVTHQGLSSPVTKSFNRGEC | 325 |
| Light chain-A44 | AIQMTQSPSSLSASVGDRVTITCRASQALTYTLAWYQQKPGKAPRLLIHDASSLESGVPSR FSGSGSGTDFTLTITSLQPEDFATYYCQQFRSYPLTFGGGTKVEIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 326 |
| Light chain-A53 | DIQMTQSPSSLSASVGDRVTITCRASQSINNYLNWYQEKPGKAPKLLIYAASSLQSGVPSR FSGSGSGTDFTLTISSLQPEDFATYYCQQSYSYPFTFGPGTKVDIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 327 |
| Light chain-A61 (light chain-A61 (IgG4SP)) | DIVMTQSPLSLPVTLGQPASISCRSSQSLVKSDGNTYLSWFQQRPGQSPRRLIYKVSNRDS GVPDRFSGSGSGTDFTLKISRVEAEDVGVYYCMQGTHWPPTFGPGTKVDIKRTVAAPSVF IFPPSDEQLKSGTASVVCLLNNFYPREAKVQWKVDNALQSGNSQESVTEQDSKDSTYSLS STLTLSKADYEKHKVYACEVTHQGLSSPVTKSFNRGEC | 328 |
| Light chain-A67 | NIQMTQSPSSLSASVGDRITITCRASQSVNSYVNWYQQTPGQAPKLLIYAASSVHNGVPV RFSGSRSGTEFILTISSLQPEDFVTYFCQQSFTSPWTFGQGTKLEMKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC | 329 |
| Light chain-A143 | EIVLTQSPATLSLSPGERATLSCRASQSIARRIAWYQQQPGQAPRLLIYDASNRATGIPARFS GSGSGTDFTLTISSLEPEDFAIYYCQQRANWPLTFGPGTKVDIKRTVAAPSVFIFPPSDEQL KSGTASVVCLLNNFYPREAKVQWKVDNALQSGNSQESVTEQDSKDSTYSLSSTLTLSKA | 330 |
| | DYEKHKVYACEVTHQGLSSPVTKSFNRGEC | |
| Light chain-A156 (light chain-A156 (IgG4SP)) | AIQLTQSPSSLSASVGDRVTITCRASQGISSYLAWYQQKPGKAPKLLIFAASLLQRGVPSRF SGSGFGTDFTLTISGLQPEDFATYFCQQFNTYPITFGGGTKVEIKRTVAAPSVFIFPPSDEQL KSGTASVVCLLNNFYPREAKVQWKVDNALQSGNSQESVTEQDSKDSTYSLSSTLTLSKA DYEKHKVYACEVTHQGLSSPVTKSFNRGEC | 331 |
| Light chain-A187 (light chain-A187 (IgG4SP)) | EIVMTQSPGTLSLSPGERATLSCRASQSVSSSYLAWYQQKPGQAPRLLIYGASSRATGIPD RFSGSGSGTDFTLTISRLEPEDFAVYYCQQYGSSPPITFGQGTRLEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC | 332 |
| Light chain-A378 | NIQMTQSPSSLTASVGDRITITCRASQGIRNSLAWYQQKPGTAPKLLVSAASKLQTGVPSR FSGSGSGTDYTLTISSLQTEDFATYYCQQYYGTPPTFGQGTKLEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC | 333 |
| Light chain-A382 | EIVLTQSPATLSLSPGERATLSCRASQGISSYLAWYQQKPGQAPRLLIYDASNRATGIPARF SGSGSGTDFTLTISNLEPEDFAVYYCQQRKNWPLTFGGGSKVEIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 334 |
| Light chain-B16 | EIVLTQSPATLSLSPGERATLSCRASQSVSSYLAWYQQKPGQAPRLLIYDASNRATGIPARF SGSGSGTDFTLTISSLEPEDFAVYYCQQRSNWPPELTFGGGTKVEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC | 335 |
| Light chain-B20 | EIVMTQSPATLSLSPGERATLSCRASQSVSNYLAWYQQKPGQAPRLLIYGASSRATGIPDR FSGSGSGTDFTLTISRLEPEDFAVYYCQQYGSSLWTFGQGTKVEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC | 336 |
| Light chain-B24 | DIQMTQSPSSLPASVGDRVTITCRASQGIGTFLAWYQQHLGKAPKLLISASSTVQRGVPSR FSGSGSGTEFTLTINSLQPEDFATYYCQQLNNYPRTFGQGTKVEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC | 337 |
| Light chain-B29 | EIVMTQSPGTLSLSPGQRATLSCRASQSVSSSSLAWYQQKPGQAPRLLIHGASSRATGIPD RFSGSGSGTDFTLTISRLEPEDFAVYYCQQYGTSWTFGQGTKVEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC | 338 |
| Light chain-B44 | DVVMTQSPLSLPVTPGEPASISCRSSQSLLHRSGYNYLDWYVQKPGQSPQLLIYLGSIRAV GVPDRFSGSASGTDFTLRISRVEAEDVGIYYCMQALQTPPAFGGGTKVEIKRTVAAPSVFI FPPSDEQLKSGTASVVCLLNNFYPREAKVQWKVDNALQSGNSQESVTEQDSKDSTYSLS STLTLSKADYEKHKVYACEVTHQGLSSPVTKSFNRGEC | 339 |
| Light chain-B53 | NIQLTQSPSSLSASVGDRVTITCRASQGINSWLAWYQQKPGKAPKLLIYAASTLQSGVPSR FSGSGSGTDFTLTINSLQPEDFATYYCQQLNSFPFTFGPGTKVHIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 340 |
| Light chain-B67 | DIQLTQSPSSLSASVGDRVTITCRASQSIDDHLNWYQQKPGKVPKLLISSASTLQSGAPSRF SGSGYGTDFTLTISSLQVEDFATYYCQQSYSPPQTFGQGTKVEIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 341 |
| Light chain-B83 | DIQLTQSPSSLSASVGDRVTITCRASQSISSYLNWYQQKPGKAPKLLIYAASSLQSGVPSRF SGSGSGTDFTLTISSLQPEDFATYYCQQSYSTPRTFGQGTRLEIKRTVAAPSVFIFPPSDEQL KSGTASVVCLLNNFYPREAKVQWKVDNALQSGNSQESVTEQDSKDSTYSLSSTLTLSKA DYEKHKVYACEVTHQGLSSPVTKSFNRGEC | 342 |
| Light chain-B88 (light chain-B88(IgG4SP )) | DVVMTQSPLSLPVTLGQPASISCRSSQSLVYSDGNTYLNWFQQRPGQSPRRLIYKVSNRD SGVPDRFSGSGSGTDFTLKISRVEAEDVGVYYCMQGTHWPPTFGQGTKLEIKRTVAAPSV FIFPPSDEQLKSGTASVVCLLNNFYPREAKVQWKVDNALQSGNSQESVTEQDSKDSTYSL SSTLTLSKADYEKHKVYACEVTHQGLSSPVTKSFNRGEC | 343 |
| Light chain-B90 (light chain- B90 (IgG4SP)) | DIQMTQSPSILSASVGDRVTLTCRASQSISSWLAWYQQTPGKAPRVLIYEASTLEVGVPSR FSGSGSGTDFTLTISSLQPEDFGTYFCQQYHTDSRTFGQGTILEIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 344 |
| Light chain-B109 | DIVMTQSPSSLSASVGDRVTITCRASQSISSYLNWYQQKPGKAPKLLIYAASSLQSGVPSR FSGSGSGTDFTLTISSLQPEDFATYYCQQSYSTLTFGGGTKVEIKRTVAAPSVFIFPPSDEQL KSGTASVVCLLNNFYPREAKVQWKVDNALQSGNSQESVTEQDSKDSTYSLSSTLTLSKA DYEKHKVYACEVTHQGLSSPVTKSFNRGEC | 345 |
| Light chain-B114 | EIVLTQSPGTLSLSPGERATLSCRASQSVSKYLAWYQQKPGQAPRLLIYDVSNRATGIPAR FSGSGSGTDFTLTISSLEPEDFAVYYCQQRRSWPLTFGGGTKVEIRRTVAAPSVFIFPPSDEQ LKSGTASVVCLLNNFYPREAKVQWKVDNALQSGNSQESVTEQDSKDSTYSLSSTLTLSK | 346 |
| | ADYEKHKVYACEVTHQGLSSPVTKSFNRGEC | |
| Light chain-B117 | NIQLTQSPSSVSASVGDRVTISCRASQGIGRWLAWYQQKPGKAPKLLIYAASNLQSGVPSR FSGSGSGTDFTLTISSLQPEDFATYYCQQANSFPRTFGPGTKVDIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 347 |
| Light chain-B123 | NIQLTQSPSSLSASVGDRVTITCRASQGISTYLAWYQQKPGLAPKLLIYGASTLQSGVPSK FSGFGSGRDFTLTISSLQPEDIATYYCQQLNTYPPTFGQGTKLEIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC | 348 |
| Light chain-B145 | DIVMTQSPSSLSASVGDRVTITCQASQDISNYLNWYQQKPGKAPKLLIYDASSLESGVPS KFSGSRSGTDFTLTISSLQPEDSATYYCQQYRSYPITFGGGTKVEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC | 349 |
| Light chain-B156 (light chain- B156 (IgG4SP)) | DIQLTQSPSSLSASIGDRVTITCRASQGISSFLAWYQQKPGKAPQLLIYAASTLQSGVPSRFS GSGSGTEFTLTISSLQPEDFATYYCQQFKIYPLTFGGGTKVEVKRTVAAPSVFIFPPSDEQLK SGTASVVCLLNNFYPREAKVQWKVDNALQSGNSQESVTEQDSKDSTYSLSSTLTLSKAD YEKHKVYACEVTHQGLSSPVTKSFNRGEC | 350 |
| Light chain-B201 | EIVMTQSPGTLSLSPGERATLSCRASQSVSSSYLAWYQQKPGQAPRLLIYGASSRATGIPD RFSGSGSGTDFTLTISRLEPEDFAVYYCQQYGSSPPITFGQGTRLEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC | 351 |
| Light chain-B307 | DIQLTQSPSFLSASVGDRVTITCRASQGISSQLAWYQQEPGKAPKLLIYAASTLQSGVPSRF SGSGSGTEFTLTISSLQPEDFATYYCQQVNAYPLTFGPGTKVDIKRTVAAPSVFIFPPSDEQL KSGTASVVCLLNNFYPREAKVQWKVDNALQSGNSQESVTEQDSKDSTYSLSSTLTLSKA DYEKHKVYACEVTHQGLSSPVTKSFNRGEC | 352 |
| Light chain constant region | | 357 |
| Heavy chain-A2 (IgG4SP) | QVQLQQSGPGLVKPSQTLSLTCTVSGGSISSGGYYWSWIRQHPGKGLEWIGYIYYSGSTY YNPSLKSRVTISVDTSKNQFSLKLSSVTAADTAVYYCARQRRANGYSSSWEFDYWGQGT LVTVSSASTKGPSVFPLAPCSRSTSESTAALGCLVKDYFPEPVTVSWNSGALTSGVHTFPA VLQSSGLYSLSSVVTVPSSSLGTKTYTCNVDHKPSNTKVDKRVESKYGPPCPPCPAPEFLG GPSVFLFPPKPKDTLMISRTPEVTCVVVDVSQEDPEVQFNWYVDGVEVHNAKTKPREEQ FNSTYRVVSVLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFLYSRLTVDKS RWQEGNVFSCSVMHEALHNHYTQKSLSLSLGK | 358 |
| Heavy chain-A5 (IgG4SP) | QVQLVQSGGGVVQPGNSLRLSCVASGFTFSRYTIHWVRQAPGKGLEWVAVISYDGSNKY YADSVKGRFTISRDNSKNTLYLQMNSLRAEDTAVYYCARGGPTPDWGQGTLVTVSSAST KGPSVFPLAPCSRSTSESTAALGCLVKDYFPEPVTVSWNSGALTSGVHTFPAVLQSSGLYS LSSVVTVPSSSLGTKTYTCNVDHKPSNTKVDKRVESKYGPPCPPCPAPEFLGGPSVFLFPP KPKDTLMISRTPEVTCVVVDVSQEDPEVQFNWYVDGVEVHNAKTKPREEQFNSTYRVV SVLTVLHQDWLNGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRLTVDKSRWQEGNV FSCSVMHEALHNHYTQKSLSLSLGK | 359 |
| Heavy chain-A13 (IgG4SP) | QVQLLESGGGLVKPGGSLRLSCAASGFTFSSYSMNWVRQAPGKGLEWVSSISSGSSYIYY ADSVKGRFTISRDNAKNSLYLQMNSLRAEDTAVYYCARDQGASVTGTHTHWGQGTLVT VSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPPCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 360 |
| Heavy chain-A61 (IgG4SP) | QVQLVQSGAEVKKPGSSVKVSCKASGGTFSSYTISWVRQAPGQGLEWMGRIIPILGIANY AQKFQGRVTITADKSTSTAYMELSSLRSEDTAVYYCATGVGMVRGDPWGQGTLVTVSSA STKGPSVFPLAPCSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPAPEFLGGPSVFLF PPKPKDTLMISRTPEVTCVVVDVSQEDPEVQFNWYVDGVEVHNAKTKPREEQFNSTYRV VSVLTVLHQDWLNGKEYKCKVSNKGLPSSIEKTISKAKGQPREPQVYTLPPSQEEMTKN QVSLTCLVKGFYPSDIAVEWESNGQPENNYKTTPPVLDSDGSFFLYSRLTVDKSRWQEGN VFSCSVMHEALHNHYTQKSLSLSLGK | 361 |
| Heavy chain-A156 (IgG4SP) | RLQLQESGPGLVKPSQTLSLTCTVSGGSISSGGYYWSWIRQHPGKGLEWIGYIYYSGSTY YNPSLKSRVTISVDTSKNQFSLKLSSVTAADTAVYYCARIHHSTAGPYNNWYFDYWGQG TLVTVSSASTKGPSVFPLAPCSRSTSESTAALGCLVKDYFPEPVTVSWNSGALTSGVHTFP AVLQSSGLYSLSSVVTVPSSSLGTKTYTCNVDHKPSNTKVDKRVESKYGPPCPPCPAPEFL GGPSVFLFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQVYTLPPS | 362 |
| | QEEMTKNQVSLTCLVKGFYPSDIAVEWESNGQPENNYKTTPPVLDSDGSFFLYSRLTVDK SRWQEGNVFSCSVMHEALHNHYTQKSLSLSLGK | |
| Heavy chain-A187 (IgG4SP) | QVQLLDSGGGLVKPGGSLRLSCAASGFTFSSYGMHWVRQAPGKGLEWVAFISNDGSNK YYADSVKGRFTISRDNSKNTLYLQMNSLRAEDTAVYYCARLLRGPWNFDLWGRGTLVT VSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPPCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK | 363 |
| Heavy chain-B88 (IgG4SP) | QVQLVQSGAEVKKPGSSVKVSCKASGGTFSSYTISWVRQAPGQGLEWMGRIIPILGIANY AQKFQGRVTITADKSTSTAYMELSSLRSEDTAVYYCATGVGMVRGDPWGQGTLVTVSSA STKGPSVFPLAPCSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPAPEFLGGPSVFLF PPKPKDTLMISRTPEVTCVVVDVSQEDPEVQFNWYVDGVEVHNAKTKPREEQFNSTYRV VSVLTVLHQDWLNGKEYKCKVSNKGLPSSIEKTISKAKGQPREPQVYTLPPSQEEMTKN QVSLTCLVKGFYPSDIAVEWESNGQPENNYKTTPPVLDSDGSFFLYSRLTVDKSRWQEGN VFSCSVMHEALHNHYTQKSLSLSLGK | 364 |
| Heavy chain-B90 (IgG4SP) | EVQLLESGGGLVKPGGSLRLSCAASGLIFKNYGMNWVRLAPGKGLEWVASISGNSNYIS YADSVKGRFTISRDNANNSLFLQMSSLRADDTAVYYCSSGYFYFDLWGPGTLVTVSSAST KGPSVFPLAPCSRSTSESTAALGCLVKDYFPEPVTVSWNSGALTSGVHTFPAVLQSSGLYS LSSVVTVPSSSLGTKTYTCNVDHKPSNTKVDKRVESKYGPPCPPCPAPEFLGGPSVFLFPP KPKDTLMISRTPEVTCVVVDVSQEDPEVQFNWYVDGVEVHNAKTKPREEQFNSTYRVV SVLTVLHQDWLNGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRLTVDKSRWQEGNV FSCSVMHEALHNHYTQKSLSLSLGK | 365 |
| Heavy chain-B156 (IgG4SP) | QVQLQQSGPGLVKPSQTLSLTCTVSGGSISSGGYYWSWIRQHPGKGLEWIGYIYYSGSTY YNPSLKSRVTISVDTSKNQFSLKLSSVTAADTAVYYCARQRRANGYSSSWEFDYWGQGT LVTVSSASTKGPSVFPLAPCSRSTSESTAALGCLVKDYFPEPVTVSWNSGALTSGVHTFPA VLQSSGLYSLSSVVTVPSSSLGTKTYTCNVDHKPSNTKVDKRVESKYGPPCPPCPAPEFLG GPSVFLFPPKPKDTLMISRTPEVTCVVVDVSQEDPEVQFNWYVDGVEVHNAKTKPREEQ FNSTYRVVSVLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFLYSRLTVDKS RWQEGNVFSCSVMHEALHNHYTQKSLSLSLGK | 366 |

The nucleotide sequence table of the present disclosure is as follows:

| Name | Nucleotide sequence | SEQ ID NO: |
|---|---|---|
| Heavy chain-A2 | CAGGTACAGCTGCAGCAGTCGGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACCGCCGCAGACACGGCTGTGTATT ACTGTGCGAGACAGAGGCGTGCCAACGGGTATAGCAGCAGCTGGGAATTTGACTACTGGGGCCAGGGCAC CCTGGTCACCGTCTCATCAGCTTCCACCAAGGGCCCCAGCGTGTTCCCCCTGGCCCCTTGTTCCAGGAGCAC CTCCGAGAGCACCGCCGCTCTGGGCTGCCTGGTGAAGGACTACTTCCCTGAGCCTGTGACCGTGAGCTGGA ACTCCGGCGCTCTGACCTCCGGCGTGCACACCTTCCCCGCCGTGCTGCAGAGCAGCGGCCTGTACAGCCTG AGCTCCGTGGTGACAGTGCCCAGCTCCAGCCTGGGCACCAAGACCTACACCTGCAATGTGGATCACAAGCC CAGCAATACCAAGGTGGACAAGAGGGTGGAGTCCAAGTACGGCCCTCCCTGCCCCTCCTGTCCTGCTCCTG AGTTCCTGGGCGGCCCTTCCGTGTTCCTGTTCCCCCCCAAGCCCAAGGATACCCTGATGATCAGCCGGACCC CTGAGGTGACATGTGTGGTGGTGGATGTGAGCCAGGAGGACCCTGAGGTGCAGTTCAACTGGTACGTGGAT GGCGTGGAGGTGCACAACGCCAAGACCAAGCCTCGGGAGGAGCAGTTCAATAGCACCTACCGGGTGGTGA GCGTGCTGACCGTGCTGCACCAGGATTGGCTGAACGGCAAGGAGTACAAGTGCAAGGTGAGCAACAAGGG CCTGCCCAGCAGCATCGAGAAGACCATCTCCAAGGCTAAGGGCCAGCCACGGGAGCCTCAGGTGTACACCC TGCCCCCTAGCCAGGAGGAGATGACCAAGAACCAGGTGAGCCTGACCTGTCTGGTGAAGGGCTTCTACCCC TCCGATATCGCTGTGGAGTGGGAGTCCAACGGCCAGCCCGAGAACAACTATAAGACCACCCCCCCTGTGCT GGACAGCGACGGCAGCTTCTTCCTGTATTCCAGGCTGACCGTGGACAAGAGCCGGTGGCAGGAGGGCAAT GTGTTCTCCTGCAGCGTGATGCACGAGGCCCTGCACAACCACTACACCCAGAAGTCCCTGTCCCTGTCCCTC GGCAAG | 367 |
| Heavy chain-A5 | CAGGTGCAGCTGGTACAGTCTGGGGGAGGCGTGGTCCAGCCTGGGAACTCCCTGAGACTCTCCTGTGTAGC CTCTGGATTCACCTTCAGTAGGTACACCATCCACTGGGTCCGCCAGGCTCCAGGCAAGGGGCTGGAGTGGG TGGCAGTTATATCATATGATGGAAGCAATAAATACTACGCAGACTCCGTGAAGGGCCGATTCACCATCTCCAG AGACAATTCCAAGAACACGCTGTATCTGCAAATGAACAGCCTGAGAGCTGAGGACACGGCTGTGTATTACT GTGCGAGAGGAGGCCCAACTCCAGACTGGGGCCAGGGCACCCTGGTCACCGTCTCATCAGCTTCCACCAA GGGCCCCAGCGTGTTCCCCCTGGCCCCTTGTTCCAGGAGCACCTCCGAGAGCACCGCCGCTCTGGGCTGCC TGGTGAAGGACTACTTCCCTGAGCCTGTGACCGTGAGCTGGAACTCCGGCGCTCTGACCTCCGGCGTGCAC ACCTTCCCCGCCGTGCTGCAGAGCAGCGGCCTGTACAGCCTGAGCTCCGTGGTGACAGTGCCCAGCTCCAG CCTGGGCACCAAGACCTACACCTGCAATGTGGATCACAAGCCCAGCAATACCAAGGTGGACAAGAGGGTG GAGTCCAAGTACGGCCCTCCCTGCCCCTCCTGTCCTGCTCCTGAGTTCCTGGGCGGCCCTTCCGTGTTCCTG TTCCCCCCCAAGCCCAAGGATACCCTGATGATCAGCCGGACCCCTGAGGTGACATGTGTGGTGGTGGATGTG AGCCAGGAGGACCCTGAGGTGCAGTTCAACTGGTACGTGGATGGCGTGGAGGTGCACAACGCCAAGACCA AGCCTCGGGAGGAGCAGTTCAATAGCACCTACCGGGTGGTGAGCGTGCTGACCGTGCTGCACCAGGATTGG CTGAACGGCAAGGAGTACAAGTGCAAGGTGAGCAACAAGGGCCTGCCCAGCAGCATCGAGAAGACCATCT CCAAGGCTAAGGGCCAGCCACGGGAGCCTCAGGTGTACACCCTGCCCCCTAGCCAGGAGGAGATGACCAA GAACCAGGTGAGCCTGACCTGTCTGGTGAAGGGCTTCTACCCCTCCGATATCGCTGTGGAGTGGGAGTCCA ACGGCCAGCCCGAGAACAACTATAAGACCACCCCCCCTGTGCTGGACAGCGACGGCAGCTTCTTCCTGTAT TCCAGGCTGACCGTGGACAAGAGCCGGTGGCAGGAGGGCAATGTGTTCTCCTGCAGCGTGATGCACGAGG CCCTGCACAACCACTACACCCAGAAGTCCCTGTCCCTGTCCCTCGGCAAG | 368 |
| Heavy chain-A8 | CAGGTTCAGCTGGTACAGTCTGGGGCTGAGGTGAAGAAGCCTGGGTCCTCGGTGAAGGTCTCCTGCAAGG CTTCTGGAGGCACCTTCAGCAGCTATGCTATCAGCTGGGTGCGACAGGCCCCTGGACAAGGGCTTGAGTGG ATGGGAGGGATCATCCCTATCTTTGGTACAGCAAACTACGCACAGAAGTTCCAGGGCAGAGTCACCATGACC AGGGACACGTCCACGAGCACAGTCTACATGGAGCTGAGCAGCCTGAGATCTGAGGACACGGCCGTGTATTA CTGTGCGAGAGATTCGGATTTTTGGAGTGGTTATTCATCAATGGACGTCTGGGGCCAAGGGACCACGGTCAC CGTCTCATCAGCTTCCACCAAGGGCCCCAGCGTGTTCCCCCTGGCCCCTTGTTCCAGGAGCACCTCCGAGA GCACCGCCGCTCTGGGCTGCCTGGTGAAGGACTACTTCCCTGAGCCTGTGACCGTGAGCTGGAACTCCGGC GCTCTGACCTCCGGCGTGCACACCTTCCCCGCCGTGCTGCAGAGCAGCGGCCTGTACAGCCTGAGCTCCGT GGTGACAGTGCCCAGCTCCAGCCTGGGCACCAAGACCTACACCTGCAATGTGGATCACAAGCCCAGCAATA CCAAGGTGGACAAGAGGGTGGAGTCCAAGTACGGCCCTCCCTGCCCCTCCTGTCCTGCTCCTGAGTTCCTG GGCGGCCCTTCCGTGTTCCTGTTCCCCCCCAAGCCCAAGGATACCCTGATGATCAGCCGGACCCCTGAGGTG ACATGTGTGGTGGTGGATGTGAGCCAGGAGGACCCTGAGGTGCAGTTCAACTGGTACGTGGATGGCGTGGA GGTGCACAACGCCAAGACCAAGCCTCGGGAGGAGCAGTTCAATAGCACCTACCGGGTGGTGAGCGTGCTG ACCGTGCTGCACCAGGATTGGCTGAACGGCAAGGAGTACAAGTGCAAGGTGAGCAACAAGGGCCTGCCCA GCAGCATCGAGAAGACCATCTCCAAGGCTAAGGGCCAGCCACGGGAGCCTCAGGTGTACACCCTGCCCCCT AGCCAGGAGGAGATGACCAAGAACCAGGTGAGCCTGACCTGTCTGGTGAAGGGCTTCTACCCCTCCGATAT CGCTGTGGAGTGGGAGTCCAACGGCCAGCCCGAGAACAACTATAAGACCACCCCCCCTGTGCTGGACAGC GACGGCAGCTTCTTCCTGTATTCCAGGCTGACCGTGGACAAGAGCCGGTGGCAGGAGGGCAATGTGTTCTC CTGCAGCGTGATGCACGAGGCCCTGCACAACCACTACACCCAGAAGTCCCTGTCCCTGTCCCTCGGCAAG | 369 |
| Heavy chain-A13 | CAGGTGCAGCTGTTGGAGTCTGGGGGAGGCCTGGTCAAGCCTGGGGGGTCCCTGAGACTCTCCTGTGCAGC CTCTGGATTCACCTTCAGTAGCTATAGCATGAACTGGGTCCGCCAGGCTCCAGGGAAGGGGCTGGAGTGGG TCTCATCCATTAGTAGTGGTAGTAGTTACATATACTACGCAGACTCAGTGAAGGGCCGATTCACCATCTCCAG AGACAACGCCAAGAACTCACTGTATCTGCAAATGAACAGCCTGAGAGCCGAGGACACGGCTGTGTATTACT GTGCGAGAGATCAAGGGGCGTCCGTAACTGGAACCCACACCCACTGGGGCCAGGGAACCCTGGTCACCGT CTCATCAGCTTCCACCAAGGGCCCCAGCGTGTTCCCCCTGGCCCCTTGTTCCAGGAGCACCTCCGAGAGCA CCGCCGCTCTGGGCTGCCTGGTGAAGGACTACTTCCCTGAGCCTGTGACCGTGAGCTGGAACTCCGGCGCT CTGACCTCCGGCGTGCACACCTTCCCCGCCGTGCTGCAGAGCAGCGGCCTGTACAGCCTGAGCTCCGTGGT GACAGTGCCCAGCTCCAGCCTGGGCACCAAGACCTACACCTGCAATGTGGATCACAAGCCCAGCAATACCA AGGTGGACAAGAGGGTGGAGTCCAAGTACGGCCCTCCCTGCCCCTCCTGTCCTGCTCCTGAGTTCCTGGGC GGCCCTTCCGTGTTCCTGTTCCCCCCCAAGCCCAAGGATACCCTGATGATCAGCCGGACCCCTGAGGTGACA TGTGTGGTGGTGGATGTGAGCCAGGAGGACCCTGAGGTGCAGTTCAACTGGTACGTGGATGGCGTGGAGGT GCACAACGCCAAGACCAAGCCTCGGGAGGAGCAGTTCAATAGCACCTACCGGGTGGTGAGCGTGCTGACC GTGCTGCACCAGGATTGGCTGAACGGCAAGGAGTACAAGTGCAAGGTGAGCAACAAGGGCCTGCCCAGCA GCATCGAGAAGACCATCTCCAAGGCTAAGGGCCAGCCACGGGAGCCTCAGGTGTACACCCTGCCCCCTAGC CAGGAGGAGATGACCAAGAACCAGGTGAGCCTGACCTGTCTGGTGAAGGGCTTCTACCCCTCCGATATCGC TGTGGAGTGGGAGTCCAACGGCCAGCCCGAGAACAACTATAAGACCACCCCCCCTGTGCTGGACAGCGAC GGCAGCTTCTTCCTGTATTCCAGGCTGACCGTGGACAAGAGCCGGTGGCAGGAGGGCAATGTGTTCTCCTG CAGCGTGATGCACGAGGCCCTGCACAACCACTACACCCAGAAGTCCCTGTCCCTGTCCCTCGGCAAG | 370 |
| Heavy chain-A21 | GAGGTGCATCTGGTGGAGTCTGGGCCTGAGCTGGTGAGGCCTGGGGCTTCAGTGACGCTGTCCTGCAAGGC TTCGGGCTACACATTTACTGACTATGAAATGCACTGGGTGAAGCAGACACCTGTGCATGGCCTGGAATGGAT TGGAGCTATTGATCCTGAAACTGGTGGTACTGCCTACAATCAGAAGTTCAAGGGCAAGGCCACACTGACTG CAGACAAATCCTCCAGCACAGCCTACATGGAGCTCCGCAGCCTGACATCTGAGGACTCTGCCGTCTATTACT GTACAACCACTTATGGTAACTACTGGTACTTCGATGTCTGGGGCGCAGGAACCCTGGTCACCGTCTCATCAG CTTCCACCAAGGGCCCCAGCGTGTTCCCCCTGGCCCCTTGTTCCAGGAGCACCTCCGAGAGCACCGCCGCT CTGGGCTGCCTGGTGAAGGACTACTTCCCTGAGCCTGTGACCGTGAGCTGGAACTCCGGCGCTCTGACCTC CGGCGTGCACACCTTCCCCGCCGTGCTGCAGAGCAGCGGCCTGTACAGCCTGAGCTCCGTGGTGACAGTGC CCAGCTCCAGCCTGGGCACCAAGACCTACACCTGCAATGTGGATCACAAGCCCAGCAATACCAAGGTGGAC AAGAGGGTGGAGTCCAAGTACGGCCCTCCCTGCCCCTCCTGTCCTGCTCCTGAGTTCCTGGGCGGCCCTTCC GTGTTCCTGTTCCCCCCCAAGCCCAAGGATACCCTGATGATCAGCCGGACCCCTGAGGTGACATGTGTGGTG GTGGATGTGAGCCAGGAGGACCCTGAGGTGCAGTTCAACTGGTACGTGGATGGCGTGGAGGTGCACAACG CCAAGACCAAGCCTCGGGAGGAGCAGTTCAATAGCACCTACCGGGTGGTGAGCGTGCTGACCGTGCTGCAC CAGGATTGGCTGAACGGCAAGGAGTACAAGTGCAAGGTGAGCAACAAGGGCCTGCCCAGCAGCATCGAGA AGACCATCTCCAAGGCTAAGGGCCAGCCACGGGAGCCTCAGGTGTACACCCTGCCCCCTAGCCAGGAGGA GATGACCAAGAACCAGGTGAGCCTGACCTGTCTGGTGAAGGGCTTCTACCCCTCCGATATCGCTGTGGAGT GGGAGTCCAACGGCCAGCCCGAGAACAACTATAAGACCACCCCCCCTGTGCTGGACAGCGACGGCAGCTT CTTCCTGTATTCCAGGCTGACCGTGGACAAGAGCCGGTGGCAGGAGGGCAATGTGTTCTCCTGCAGCGTGA TGCACGAGGCCCTGCACAACCACTACACCCAGAAGTCCCTGTCCCTGTCCCTCGGCAAG | 371 |
| Heavy chain-A22 | GAAGTGCAGCTGGTGGAGTCTGGGGCTGAGGTGAAGAAGCCTGGGTCCTCGGTGAAGGTCTCCTGCAAGG CTTCTGGAGGCACCTTCAGCAGCTATGCTATCAGCTGGGTGCGACAGGCCCCTGGACAAGGGCTTGAGTGG ATGGGAGGGATCATCCCTATCTTTGGTACAGCAAACTACGCACAGAAGTTCCAGGGCAGAGTCACGATTACC GCGGACGAATCCACGAGCACAGCCTACATGGAGCTGAGCAGCCTGAGATCTGAGGACACGGCCGTGTATTA CTGTGCGTTCACCAGTGGGAGCTACTACTACTTTGACTACTGGGGCCAGGGCACCCTGGTCACCGTCTCATC AGCTTCCACCAAGGGCCCCAGCGTGTTCCCCCTGGCCCCTTGTTCCAGGAGCACCTCCGAGAGCACCGCCG CTCTGGGCTGCCTGGTGAAGGACTACTTCCCTGAGCCTGTGACCGTGAGCTGGAACTCCGGCGCTCTGACC TCCGGCGTGCACACCTTCCCCGCCGTGCTGCAGAGCAGCGGCCTGTACAGCCTGAGCTCCGTGGTGACAGT GCCCAGCTCCAGCCTGGGCACCAAGACCTACACCTGCAATGTGGATCACAAGCCCAGCAATACCAAGGTGG ACAAGAGGGTGGAGTCCAAGTACGGCCCTCCCTGCCCCTCCTGTCCTGCTCCTGAGTTCCTGGGCGGCCCT TCCGTGTTCCTGTTCCCCCCCAAGCCCAAGGATACCCTGATGATCAGCCGGACCCCTGAGGTGACATGTGTG GTGGTGGATGTGAGCCAGGAGGACCCTGAGGTGCAGTTCAACTGGTACGTGGATGGCGTGGAGGTGCACA ACGCCAAGACCAAGCCTCGGGAGGAGCAGTTCAATAGCACCTACCGGGTGGTGAGCGTGCTGACCGTGCT GCACCAGGATTGGCTGAACGGCAAGGAGTACAAGTGCAAGGTGAGCAACAAGGGCCTGCCCAGCAGCATC GAGAAGACCATCTCCAAGGCTAAGGGCCAGCCACGGGAGCCTCAGGTGTACACCCTGCCCCCTAGCCAGG AGGAGATGACCAAGAACCAGGTGAGCCTGACCTGTCTGGTGAAGGGCTTCTACCCCTCCGATATCGCTGTG GAGTGGGAGTCCAACGGCCAGCCCGAGAACAACTATAAGACCACCCCCCCTGTGCTGGACAGCGACGGCA GCTTCTTCCTGTATTCCAGGCTGACCGTGGACAAGAGCCGGTGGCAGGAGGGCAATGTGTTCTCCTGCAGC GTGATGCACGAGGCCCTGCACAACCACTACACCCAGAAGTCCCTGTCCCTGTCCCTCGGCAAG | 372 |
| Heavy chain-A23 | CAGGTCCAGCTTGTGCAATCTGGGGCTGAGGTGAAGAAGCCTGGGTCCTCGGTGAAGGTCTCCTGCAAGGC TTCTGGAGGCACCTTCAGCAGCTATGCTATCAGCTGGGTGCGACAGGCCCCTGGACAAGGGCTTGAGTGGA TGGGAGGGATCATCCCTATCTTTGGTACAGCAAACTACGCACAGAAGTTCCAGGGCAGAGTCACGATTACCG CGGACGAATCCACGAGCACAGCCTACATGGAGCTGAGCAGCCTGAGATCTGAGGACACGGCCGTGTATTAC TGTGCGAGAGGGGGAGGGGGGAGGGGTATGACGAGCGACTGGGGCCAGGGAACCCTGGTCACCGTCTCAT CAGCTTCCACCAAGGGCCCCAGCGTGTTCCCCCTGGCCCCTTGTTCCAGGAGCACCTCCGAGAGCACCGCC GCTCTGGGCTGCCTGGTGAAGGACTACTTCCCTGAGCCTGTGACCGTGAGCTGGAACTCCGGCGCTCTGAC CTCCGGCGTGCACACCTTCCCCGCCGTGCTGCAGAGCAGCGGCCTGTACAGCCTGAGCTCCGTGGTGACAG TGCCCAGCTCCAGCCTGGGCACCAAGACCTACACCTGCAATGTGGATCACAAGCCCAGCAATACCAAGGTG GACAAGAGGGTGGAGTCCAAGTACGGCCCTCCCTGCCCCTCCTGTCCTGCTCCTGAGTTCCTGGGCGGCCC TTCCGTGTTCCTGTTCCCCCCCAAGCCCAAGGATACCCTGATGATCAGCCGGACCCCTGAGGTGACATGTGT GGTGGTGGATGTGAGCCAGGAGGACCCTGAGGTGCAGTTCAACTGGTACGTGGATGGCGTGGAGGTGCAC AACGCCAAGACCAAGCCTCGGGAGGAGCAGTTCAATAGCACCTACCGGGTGGTGAGCGTGCTGACCGTGC TGCACCAGGATTGGCTGAACGGCAAGGAGTACAAGTGCAAGGTGAGCAACAAGGGCCTGCCCAGCAGCAT CGAGAAGACCATCTCCAAGGCTAAGGGCCAGCCACGGGAGCCTCAGGTGTACACCCTGCCCCCTAGCCAGG AGGAGATGACCAAGAACCAGGTGAGCCTGACCTGTCTGGTGAAGGGCTTCTACCCCTCCGATATCGCTGTG GAGTGGGAGTCCAACGGCCAGCCCGAGAACAACTATAAGACCACCCCCCCTGTGCTGGACAGCGACGGCA GCTTCTTCCTGTATTCCAGGCTGACCGTGGACAAGAGCCGGTGGCAGGAGGGCAATGTGTTCTCCTGCAGC GTGATGCACGAGGCCCTGCACAACCACTACACCCAGAAGTCCCTGTCCCTGTCCCTCGGCAAG | 373 |
| Heavy chain-A26 | CAGGTGCAGCTGTTGGAGTCTGGGGGAGGCTTGGTACAGCCTGGGGGGTCCCTGAGACTCTCCTGTGCAGC CTCTGGATTCACCTTTAGCAGCTATGCCATGAGCTGGGTCCGCCAGGCTCCAGGGAAGGGGCTGGAGTGGG TCTCAGCTATTAGTGGTAGTGGTGGTAGCACATACTACGCAGACTCCGTGAAGGGCCGGTTCACCATCTCCA GAGACAATTCCAAGAACACGCTGTATCTGCAAATGAACAGCCTGAGAGCCGAGGACACGGCCGTATATTAC TGTGCGAAAGGAACGAAGATGGCTACAATCCGCCTCGGCTACTACTACTACGGTATGGACGTCTGGGGCCAA GGGACAATGGTCACCGTCTCATCAGCTTCCACCAAGGGCCCCAGCGTGTTCCCCCTGGCCCCTTGTTCCAGG AGCACCTCCGAGAGCACCGCCGCTCTGGGCTGCCTGGTGAAGGACTACTTCCCTGAGCCTGTGACCGTGAG CTGGAACTCCGGCGCTCTGACCTCCGGCGTGCACACCTTCCCCGCCGTGCTGCAGAGCAGCGGCCTGTACA GCCTGAGCTCCGTGGTGACAGTGCCCAGCTCCAGCCTGGGCACCAAGACCTACACCTGCAATGTGGATCAC AAGCCCAGCAATACCAAGGTGGACAAGAGGGTGGAGTCCAAGTACGGCCCTCCCTGCCCCTCCTGTCCTGC TCCTGAGTTCCTGGGCGGCCCTTCCGTGTTCCTGTTCCCCCCCAAGCCCAAGGATACCCTGATGATCAGCCG GACCCCTGAGGTGACATGTGTGGTGGTGGATGTGAGCCAGGAGGACCCTGAGGTGCAGTTCAACTGGTACG TGGATGGCGTGGAGGTGCACAACGCCAAGACCAAGCCTCGGGAGGAGCAGTTCAATAGCACCTACCGGGT GGTGAGCGTGCTGACCGTGCTGCACCAGGATTGGCTGAACGGCAAGGAGTACAAGTGCAAGGTGAGCAAC AAGGGCCTGCCCAGCAGCATCGAGAAGACCATCTCCAAGGCTAAGGGCCAGCCACGGGAGCCTCAGGTGT ACACCCTGCCCCCTAGCCAGGAGGAGATGACCAAGAACCAGGTGAGCCTGACCTGTCTGGTGAAGGGCTT CTACCCCTCCGATATCGCTGTGGAGTGGGAGTCCAACGGCCAGCCCGAGAACAACTATAAGACCACCCCCC CTGTGCTGGACAGCGACGGCAGCTTCTTCCTGTATTCCAGGCTGACCGTGGACAAGAGCCGGTGGCAGGAG GGCAATGTGTTCTCCTGCAGCGTGATGCACGAGGCCCTGCACAACCACTACACCCAGAAGTCCCTGTCCCT GTCCCTCGGCAAG | 374 |
| Heavy chain-A44 | CAGGTACAGCTGCAGGAGTCGGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACTGCCGCGGACACGGCCGTGTATT ACTGTGCGAGATGGGGGCGAGGGGGCAGCAGCTGGCGGTTTGATATCTGGGGCCAAGGGACAATGGTCAC CGTCTCATCAGCTTCCACCAAGGGCCCCAGCGTGTTCCCCCTGGCCCCTTGTTCCAGGAGCACCTCCGAGA GCACCGCCGCTCTGGGCTGCCTGGTGAAGGACTACTTCCCTGAGCCTGTGACCGTGAGCTGGAACTCCGGC GCTCTGACCTCCGGCGTGCACACCTTCCCCGCCGTGCTGCAGAGCAGCGGCCTGTACAGCCTGAGCTCCGT GGTGACAGTGCCCAGCTCCAGCCTGGGCACCAAGACCTACACCTGCAATGTGGATCACAAGCCCAGCAATA CCAAGGTGGACAAGAGGGTGGAGTCCAAGTACGGCCCTCCCTGCCCCTCCTGTCCTGCTCCTGAGTTCCTG GGCGGCCCTTCCGTGTTCCTGTTCCCCCCCAAGCCCAAGGATACCCTGATGATCAGCCGGACCCCTGAGGTG ACATGTGTGGTGGTGGATGTGAGCCAGGAGGACCCTGAGGTGCAGTTCAACTGGTACGTGGATGGCGTGGA GGTGCACAACGCCAAGACCAAGCCTCGGGAGGAGCAGTTCAATAGCACCTACCGGGTGGTGAGCGTGCTG ACCGTGCTGCACCAGGATTGGCTGAACGGCAAGGAGTACAAGTGCAAGGTGAGCAACAAGGGCCTGCCCA GCAGCATCGAGAAGACCATCTCCAAGGCTAAGGGCCAGCCACGGGAGCCTCAGGTGTACACCCTGCCCCCT AGCCAGGAGGAGATGACCAAGAACCAGGTGAGCCTGACCTGTCTGGTGAAGGGCTTCTACCCCTCCGATAT CGCTGTGGAGTGGGAGTCCAACGGCCAGCCCGAGAACAACTATAAGACCACCCCCCCTGTGCTGGACAGC GACGGCAGCTTCTTCCTGTATTCCAGGCTGACCGTGGACAAGAGCCGGTGGCAGGAGGGCAATGTGTTCTC CTGCAGCGTGATGCACGAGGCCCTGCACAACCACTACACCCAGAAGTCCCTGTCCCTGTCCCTCGGCAAG | 375 |
| Heavy chain-A53 | GAGGTGCAGCTGTTGGAGTCTGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACTGCCGCGGACACGGCCGTGTATT ACTGTGCGAGAGATTCGAGGGGGCGAAGCTACGGTATGGACGTCTGGGGCCAAGGGACAATGGTCACCGTC TCATCAGCTTCCACCAAGGGCCCCAGCGTGTTCCCCCTGGCCCCTTGTTCCAGGAGCACCTCCGAGAGCAC CGCCGCTCTGGGCTGCCTGGTGAAGGACTACTTCCCTGAGCCTGTGACCGTGAGCTGGAACTCCGGCGCTC TGACCTCCGGCGTGCACACCTTCCCCGCCGTGCTGCAGAGCAGCGGCCTGTACAGCCTGAGCTCCGTGGTG ACAGTGCCCAGCTCCAGCCTGGGCACCAAGACCTACACCTGCAATGTGGATCACAAGCCCAGCAATACCAA GGTGGACAAGAGGGTGGAGTCCAAGTACGGCCCTCCCTGCCCCTCCTGTCCTGCTCCTGAGTTCCTGGGCG GCCCTTCCGTGTTCCTGTTCCCCCCCAAGCCCAAGGATACCCTGATGATCAGCCGGACCCCTGAGGTGACAT GTGTGGTGGTGGATGTGAGCCAGGAGGACCCTGAGGTGCAGTTCAACTGGTACGTGGATGGCGTGGAGGTG CACAACGCCAAGACCAAGCCTCGGGAGGAGCAGTTCAATAGCACCTACCGGGTGGTGAGCGTGCTGACCG TGCTGCACCAGGATTGGCTGAACGGCAAGGAGTACAAGTGCAAGGTGAGCAACAAGGGCCTGCCCAGCAG CATCGAGAAGACCATCTCCAAGGCTAAGGGCCAGCCACGGGAGCCTCAGGTGTACACCCTGCCCCCTAGCC AGGAGGAGATGACCAAGAACCAGGTGAGCCTGACCTGTCTGGTGAAGGGCTTCTACCCCTCCGATATCGCT GTGGAGTGGGAGTCCAACGGCCAGCCCGAGAACAACTATAAGACCACCCCCCCTGTGCTGGACAGCGACG GCAGCTTCTTCCTGTATTCCAGGCTGACCGTGGACAAGAGCCGGTGGCAGGAGGGCAATGTGTTCTCCTGC AGCGTGATGCACGAGGCCCTGCACAACCACTACACCCAGAAGTCCCTGTCCCTGTCCCTCGGCAAG | 376 |
| Heavy chain-A61 | CAGGTGCAGCTGGTGCAATCTGGGGCTGAGGTGAAGAAGCCTGGGTCCTCGGTGAAGGTCTCCTGCAAGG CTTCTGGAGGCACCTTCAGCAGCTATACTATCAGCTGGGTGCGACAGGCCCCTGGACAAGGGCTTGAGTGG ATGGGAAGGATCATCCCTATCCTTGGTATAGCAAACTACGCACAGAAGTTCCAGGGCAGAGTCACGATTACC GCGGACAAATCCACGAGCACAGCCTACATGGAGCTGAGCAGCCTGAGATCTGAGGACACGGCCGTGTATTA CTGTGCGACGGGGGTTGGTATGGTTCGGGGAGACCCTTGGGGCCAGGGAACCCTGGTCACCGTCTCATCAG CTTCCACCAAGGGCCCCAGCGTGTTCCCCCTGGCCCCTTGTTCCAGGAGCACCTCCGAGAGCACCGCCGCT CTGGGCTGCCTGGTGAAGGACTACTTCCCTGAGCCTGTGACCGTGAGCTGGAACTCCGGCGCTCTGACCTC CGGCGTGCACACCTTCCCCGCCGTGCTGCAGAGCAGCGGCCTGTACAGCCTGAGCTCCGTGGTGACAGTGC CCAGCTCCAGCCTGGGCACCAAGACCTACACCTGCAATGTGGATCACAAGCCCAGCAATACCAAGGTGGAC AAGAGGGTGGAGTCCAAGTACGGCCCTCCCTGCCCCTCCTGTCCTGCTCCTGAGTTCCTGGGCGGCCCTTCC GTGTTCCTGTTCCCCCCCAAGCCCAAGGATACCCTGATGATCAGCCGGACCCCTGAGGTGACATGTGTGGTG GTGGATGTGAGCCAGGAGGACCCTGAGGTGCAGTTCAACTGGTACGTGGATGGCGTGGAGGTGCACAACG CCAAGACCAAGCCTCGGGAGGAGCAGTTCAATAGCACCTACCGGGTGGTGAGCGTGCTGACCGTGCTGCAC CAGGATTGGCTGAACGGCAAGGAGTACAAGTGCAAGGTGAGCAACAAGGGCCTGCCCAGCAGCATCGAGA AGACCATCTCCAAGGCTAAGGGCCAGCCACGGGAGCCTCAGGTGTACACCCTGCCCCCTAGCCAGGAGGA GATGACCAAGAACCAGGTGAGCCTGACCTGTCTGGTGAAGGGCTTCTACCCCTCCGATATCGCTGTGGAGT GGGAGTCCAACGGCCAGCCCGAGAACAACTATAAGACCACCCCCCCTGTGCTGGACAGCGACGGCAGCTT CTTCCTGTATTCCAGGCTGACCGTGGACAAGAGCCGGTGGCAGGAGGGCAATGTGTTCTCCTGCAGCGTGA TGCACGAGGCCCTGCACAACCACTACACCCAGAAGTCCCTGTCCCTGTCCCTCGGCAAG | 377 |
| Heavy chain-A67 | CAGGTGCAGCTGGTGCAATCTGGGGGAGGCGTGGTCCAGCCTGGGAGGTCCCTGAGACTCCCCTGTGCAGC CTCTGGATTCACCTTCAGTAATTATGCTATGCACTGGGTCCGCCAGGCTCCAGGCAAGGGGCTGGAGTGGGT GGCAGTTATATCATATGATGGAAGTAAGAAATACTACGCAGACTCCGTGAAGGGCCGATTCACCATCTCCAG AGACAATTCCAAGAACACGCTGTATCTGCAAGTGAACAGCCTGAGAGCTGAGGACACGGCTGTGTATTACT GTGCGACCGGTTATAGCAGTCCCTGGTACGGTATGGACGTCTGGGGCCAAGGGACCACGGTCACCGTCTCAT CAGCTTCCACCAAGGGCCCCAGCGTGTTCCCCCTGGCCCCTTGTTCCAGGAGCACCTCCGAGAGCACCGCC GCTCTGGGCTGCCTGGTGAAGGACTACTTCCCTGAGCCTGTGACCGTGAGCTGGAACTCCGGCGCTCTGAC CTCCGGCGTGCACACCTTCCCCGCCGTGCTGCAGAGCAGCGGCCTGTACAGCCTGAGCTCCGTGGTGACAG TGCCCAGCTCCAGCCTGGGCACCAAGACCTACACCTGCAATGTGGATCACAAGCCCAGCAATACCAAGGTG GACAAGAGGGTGGAGTCCAAGTACGGCCCTCCCTGCCCCTCCTGTCCTGCTCCTGAGTTCCTGGGCGGCCC TTCCGTGTTCCTGTTCCCCCCCAAGCCCAAGGATACCCTGATGATCAGCCGGACCCCTGAGGTGACATGTGT GGTGGTGGATGTGAGCCAGGAGGACCCTGAGGTGCAGTTCAACTGGTACGTGGATGGCGTGGAGGTGCAC AACGCCAAGACCAAGCCTCGGGAGGAGCAGTTCAATAGCACCTACCGGGTGGTGAGCGTGCTGACCGTGC TGCACCAGGATTGGCTGAACGGCAAGGAGTACAAGTGCAAGGTGAGCAACAAGGGCCTGCCCAGCAGCAT CGAGAAGACCATCTCCAAGGCTAAGGGCCAGCCACGGGAGCCTCAGGTGTACACCCTGCCCCCTAGCCAGG AGGAGATGACCAAGAACCAGGTGAGCCTGACCTGTCTGGTGAAGGGCTTCTACCCCTCCGATATCGCTGTG GAGTGGGAGTCCAACGGCCAGCCCGAGAACAACTATAAGACCACCCCCCCTGTGCTGGACAGCGACGGCA GCTTCTTCCTGTATTCCAGGCTGACCGTGGACAAGAGCCGGTGGCAGGAGGGCAATGTGTTCTCCTGCAGC GTGATGCACGAGGCCCTGCACAACCACTACACCCAGAAGTCCCTGTCCCTGTCCCTCGGCAAG | 378 |
| Heavy chain-A143 | GAGGTGCAGCTGGTGGAGACTGGGGGAGGCGTGGTCCAGCCTGGGAGGTCCCTGAGACTCTCCTGTGCAG CCTCTGGATTCACCTTCAGTAGCTATGCTATGCACTGGGTCCGCCAGGCTCCAGGCAAGGGGCTGGAGTGGG TGGCAGTTATATCATATGATGGAAGTAATAAATACTACGCAGACTCCGTGAAGGGCCGATTCACCATCTCCAG AGACAATTCCAAGAACACGCTGTATCTGCAAATGAACAGCCTGAGAGCTGAGGACACGGCTGTGTATTACT GTGCGAGAGCCGCAGGTCACGGGATTTTAGACTACTGGGGCCAGGGAACCCTGGTCACCGTCTCATCAGCT TCCACCAAGGGCCCCAGCGTGTTCCCCCTGGCCCCTTGTTCCAGGAGCACCTCCGAGAGCACCGCCGCTCT GGGCTGCCTGGTGAAGGACTACTTCCCTGAGCCTGTGACCGTGAGCTGGAACTCCGGCGCTCTGACCTCCG GCGTGCACACCTTCCCCGCCGTGCTGCAGAGCAGCGGCCTGTACAGCCTGAGCTCCGTGGTGACAGTGCCC AGCTCCAGCCTGGGCACCAAGACCTACACCTGCAATGTGGATCACAAGCCCAGCAATACCAAGGTGGACAA GAGGGTGGAGTCCAAGTACGGCCCTCCCTGCCCCTCCTGTCCTGCTCCTGAGTTCCTGGGCGGCCCTTCCGT GTTCCTGTTCCCCCCCAAGCCCAAGGATACCCTGATGATCAGCCGGACCCCTGAGGTGACATGTGTGGTGGT GGATGTGAGCCAGGAGGACCCTGAGGTGCAGTTCAACTGGTACGTGGATGGCGTGGAGGTGCACAACGCC AAGACCAAGCCTCGGGAGGAGCAGTTCAATAGCACCTACCGGGTGGTGAGCGTGCTGACCGTGCTGCACC AGGATTGGCTGAACGGCAAGGAGTACAAGTGCAAGGTGAGCAACAAGGGCCTGCCCAGCAGCATCGAGAA GACCATCTCCAAGGCTAAGGGCCAGCCACGGGAGCCTCAGGTGTACACCCTGCCCCCTAGCCAGGAGGAG ATGACCAAGAACCAGGTGAGCCTGACCTGTCTGGTGAAGGGCTTCTACCCCTCCGATATCGCTGTGGAGTG GGAGTCCAACGGCCAGCCCGAGAACAACTATAAGACCACCCCCCCTGTGCTGGACAGCGACGGCAGCTTCT TCCTGTATTCCAGGCTGACCGTGGACAAGAGCCGGTGGCAGGAGGGCAATGTGTTCTCCTGCAGCGTGATG CACGAGGCCCTGCACAACCACTACACCCAGAAGTCCCTGTCCCTGTCCCTCGGCAAG | 379 |
| Heavy chain-A156 | CGGCTGCAGCTGCAGGAGTCGGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACTGCCGCGGACACGGCCGTGTATT ACTGTGCGAGGATTCACCATAGTACCGCGGGGCCATACAACAACTGGTACTTTGACTACTGGGGCCAGGGA ACCCTGGTCACCGTCTCATCAGCTTCCACCAAGGGCCCCAGCGTGTTCCCCCTGGCCCCTTGTTCCAGGAGC ACCTCCGAGAGCACCGCCGCTCTGGGCTGCCTGGTGAAGGACTACTTCCCTGAGCCTGTGACCGTGAGCTG GAACTCCGGCGCTCTGACCTCCGGCGTGCACACCTTCCCCGCCGTGCTGCAGAGCAGCGGCCTGTACAGCC TGAGCTCCGTGGTGACAGTGCCCAGCTCCAGCCTGGGCACCAAGACCTACACCTGCAATGTGGATCACAAG CCCAGCAATACCAAGGTGGACAAGAGGGTGGAGTCCAAGTACGGCCCTCCCTGCCCCTCCTGTCCTGCTCC TGAGTTCCTGGGCGGCCCTTCCGTGTTCCTGTTCCCCCCCAAGCCCAAGGATACCCTGATGATCAGCCGGAC CCCTGAGGTGACATGTGTGGTGGTGGATGTGAGCCAGGAGGACCCTGAGGTGCAGTTCAACTGGTACGTGG ATGGCGTGGAGGTGCACAACGCCAAGACCAAGCCTCGGGAGGAGCAGTTCAATAGCACCTACCGGGTGGT GAGCGTGCTGACCGTGCTGCACCAGGATTGGCTGAACGGCAAGGAGTACAAGTGCAAGGTGAGCAACAAG GGCCTGCCCAGCAGCATCGAGAAGACCATCTCCAAGGCTAAGGGCCAGCCACGGGAGCCTCAGGTGTACA CCCTGCCCCCTAGCCAGGAGGAGATGACCAAGAACCAGGTGAGCCTGACCTGTCTGGTGAAGGGCTTCTAC CCCTCCGATATCGCTGTGGAGTGGGAGTCCAACGGCCAGCCCGAGAACAACTATAAGACCACCCCCCCTGT GCTGGACAGCGACGGCAGCTTCTTCCTGTATTCCAGGCTGACCGTGGACAAGAGCCGGTGGCAGGAGGGC AATGTGTTCTCCTGCAGCGTGATGCACGAGGCCCTGCACAACCACTACACCCAGAAGTCCCTGTCCCTGTCC CTCGGCAAG | 380 |
| Heavy chain-A187 | CAGGTGCAGCTGTTGGACTCTGGGGGAGGCTTGGTCAAGCCTGGAGGGTCCCTGAGACTCTCCTGTGCAGC CTCTGGATTCACCTTCAGTAGTTATGGCATGCACTGGGTCCGCCAGGCTCCAGGCAAGGGGCTGGAGTGGGT GGCATTTATATCAAATGATGGAAGTAATAAATACTATGCAGACTCCGTGAAGGGCCGATTCACCATCTCCAGA GACAATTCCAAGAACACGTTGTATCTGCAAATGAACAGCCTGAGAGCTGAGGACACGGCTGTGTATTACTGT GCGAGACTTCTCCGTGGACCCTGGAACTTCGATCTCTGGGGCCGTGGCACCCTGGTCACCGTCTCATCAGCT TCCACCAAGGGCCCCAGCGTGTTCCCCCTGGCCCCTTGTTCCAGGAGCACCTCCGAGAGCACCGCCGCTCT GGGCTGCCTGGTGAAGGACTACTTCCCTGAGCCTGTGACCGTGAGCTGGAACTCCGGCGCTCTGACCTCCG GCGTGCACACCTTCCCCGCCGTGCTGCAGAGCAGCGGCCTGTACAGCCTGAGCTCCGTGGTGACAGTGCCC AGCTCCAGCCTGGGCACCAAGACCTACACCTGCAATGTGGATCACAAGCCCAGCAATACCAAGGTGGACAA GAGGGTGGAGTCCAAGTACGGCCCTCCCTGCCCCTCCTGTCCTGCTCCTGAGTTCCTGGGCGGCCCTTCCGT GTTCCTGTTCCCCCCCAAGCCCAAGGATACCCTGATGATCAGCCGGACCCCTGAGGTGACATGTGTGGTGGT GGATGTGAGCCAGGAGGACCCTGAGGTGCAGTTCAACTGGTACGTGGATGGCGTGGAGGTGCACAACGCC AAGACCAAGCCTCGGGAGGAGCAGTTCAATAGCACCTACCGGGTGGTGAGCGTGCTGACCGTGCTGCACC AGGATTGGCTGAACGGCAAGGAGTACAAGTGCAAGGTGAGCAACAAGGGCCTGCCCAGCAGCATCGAGAA GACCATCTCCAAGGCTAAGGGCCAGCCACGGGAGCCTCAGGTGTACACCCTGCCCCCTAGCCAGGAGGAG ATGACCAAGAACCAGGTGAGCCTGACCTGTCTGGTGAAGGGCTTCTACCCCTCCGATATCGCTGTGGAGTG GGAGTCCAACGGCCAGCCCGAGAACAACTATAAGACCACCCCCCCTGTGCTGGACAGCGACGGCAGCTTCT TCCTGTATTCCAGGCTGACCGTGGACAAGAGCCGGTGGCAGGAGGGCAATGTGTTCTCCTGCAGCGTGATG CACGAGGCCCTGCACAACCACTACACCCAGAAGTCCCTGTCCCTGTCCCTCGGCAAG | 381 |
| Heavy chain-A378 | GAGGTGCAGCTGTTGGAGTCTGGGGGAGGCTTGGTACAGCCTGGGGGGTCCCTGAGACTCTCCTGTGCAGC CTCTGGATTCACCTTTAGCAGCTATGCCATGAGCTGGGTCCGCCAGGCTCCAGGGAAGGGGCTGGAGTGGG TCTCAGCTATTAGTGGTAGTGGTGGTAGCACATACTACGCAGACTCCGTGAAGGGCCGGTTCACCATCTCCA GAGACAATTCCAAGAACACGCTGTATCTGCAAATGAACAGCCTGAGAGCCGAGGACACGGCCGTATATTAC TGTGCGACTAGTAGTGGCCCTAGAATTGATGCTTTTGATATCTGGGGCCAAGGGACAATGGTCACCGTCTCAT CAGCTTCCACCAAGGGCCCCAGCGTGTTCCCCCTGGCCCCTTGTTCCAGGAGCACCTCCGAGAGCACCGCC GCTCTGGGCTGCCTGGTGAAGGACTACTTCCCTGAGCCTGTGACCGTGAGCTGGAACTCCGGCGCTCTGAC CTCCGGCGTGCACACCTTCCCCGCCGTGCTGCAGAGCAGCGGCCTGTACAGCCTGAGCTCCGTGGTGACAG TGCCCAGCTCCAGCCTGGGCACCAAGACCTACACCTGCAATGTGGATCACAAGCCCAGCAATACCAAGGTG GACAAGAGGGTGGAGTCCAAGTACGGCCCTCCCTGCCCCTCCTGTCCTGCTCCTGAGTTCCTGGGCGGCCC TTCCGTGTTCCTGTTCCCCCCCAAGCCCAAGGATACCCTGATGATCAGCCGGACCCCTGAGGTGACATGTGT GGTGGTGGATGTGAGCCAGGAGGACCCTGAGGTGCAGTTCAACTGGTACGTGGATGGCGTGGAGGTGCAC AACGCCAAGACCAAGCCTCGGGAGGAGCAGTTCAATAGCACCTACCGGGTGGTGAGCGTGCTGACCGTGC TGCACCAGGATTGGCTGAACGGCAAGGAGTACAAGTGCAAGGTGAGCAACAAGGGCCTGCCCAGCAGCAT CGAGAAGACCATCTCCAAGGCTAAGGGCCAGCCACGGGAGCCTCAGGTGTACACCCTGCCCCCTAGCCAGG AGGAGATGACCAAGAACCAGGTGAGCCTGACCTGTCTGGTGAAGGGCTTCTACCCCTCCGATATCGCTGTG GAGTGGGAGTCCAACGGCCAGCCCGAGAACAACTATAAGACCACCCCCCCTGTGCTGGACAGCGACGGCA GCTTCTTCCTGTATTCCAGGCTGACCGTGGACAAGAGCCGGTGGCAGGAGGGCAATGTGTTCTCCTGCAGC GTGATGCACGAGGCCCTGCACAACCACTACACCCAGAAGTCCCTGTCCCTGTCCCTCGGCAAG | 382 |
| Heavy chain-A382 | CAGGTGCAGCTGGTACAGTCTGGGGCTGAGGTGAAGAAGCCTGGGGCCTCAGTGAAGGTTTCCTGCAAGG CTTCTGGATACACCTTCACTAGCTATGCTATGCATTGGGTGCGCCAGGCCCCCGGACAAAGGCTTGAGTGGA TGGGATGGATCAACGCTGGCAATGGTAACACAAAATATTCACAGAAGTTCCAGGGCAGAGTCACCATTACC AGGGACACATCCGCGAGCACAGCCTACATGGAGCTGAGCAGCCTGAGATCTGAAGACACGGCTGTGTATTA CTGTGCGACCAGTAAGTCGGTCTGGAATTTTGACTACTGGGGCCAGGGAACCCTGGTCACCGTCTCATCAGC TTCCACCAAGGGCCCCAGCGTGTTCCCCCTGGCCCCTTGTTCCAGGAGCACCTCCGAGAGCACCGCCGCTC TGGGCTGCCTGGTGAAGGACTACTTCCCTGAGCCTGTGACCGTGAGCTGGAACTCCGGCGCTCTGACCTCC GGCGTGCACACCTTCCCCGCCGTGCTGCAGAGCAGCGGCCTGTACAGCCTGAGCTCCGTGGTGACAGTGCC CAGCTCCAGCCTGGGCACCAAGACCTACACCTGCAATGTGGATCACAAGCCCAGCAATACCAAGGTGGACA AGAGGGTGGAGTCCAAGTACGGCCCTCCCTGCCCCTCCTGTCCTGCTCCTGAGTTCCTGGGCGGCCCTTCCG TGTTCCTGTTCCCCCCCAAGCCCAAGGATACCCTGATGATCAGCCGGACCCCTGAGGTGACATGTGTGGTGG TGGATGTGAGCCAGGAGGACCCTGAGGTGCAGTTCAACTGGTACGTGGATGGCGTGGAGGTGCACAACGC CAAGACCAAGCCTCGGGAGGAGCAGTTCAATAGCACCTACCGGGTGGTGAGCGTGCTGACCGTGCTGCACC AGGATTGGCTGAACGGCAAGGAGTACAAGTGCAAGGTGAGCAACAAGGGCCTGCCCAGCAGCATCGAGAA GACCATCTCCAAGGCTAAGGGCCAGCCACGGGAGCCTCAGGTGTACACCCTGCCCCCTAGCCAGGAGGAG ATGACCAAGAACCAGGTGAGCCTGACCTGTCTGGTGAAGGGCTTCTACCCCTCCGATATCGCTGTGGAGTG GGAGTCCAACGGCCAGCCCGAGAACAACTATAAGACCACCCCCCCTGTGCTGGACAGCGACGGCAGCTTCT TCCTGTATTCCAGGCTGACCGTGGACAAGAGCCGGTGGCAGGAGGGCAATGTGTTCTCCTGCAGCGTGATG CACGAGGCCCTGCACAACCACTACACCCAGAAGTCCCTGTCCCTGTCCCTCGGCAAG | 383 |
| Heavy chain-B16 | CAGGTGCAGCTGGTGCAGTCTGGGGGAGGCTTGGTACAGCCTGGGGGGTCCCTGAGACTCTCCTGTGCAGC CTCTGGATTCACCTTTAGCAGCTATGCCATGAGCTGGGTCCGCCAGGCTCCAGGGAAGGGGCTGGAGTGGG TCTCAGCTATTAGTGGTAGTGGTGGTAGCACATACTACGCAGACTCCGTGAAGGGCCGATTCACCATCTCCA GAGACAATCCCAAGAACACCCTGTTCCTGCAAATGACCAGTCTAAGGTCTGAGGACACGGCCATGTATTATT GTGCAAGATCCTATGGTAACTACTTTACTTACTGGGGCCAAGGGACCCTGGTCACCGTCTCATCAGCTTCCA CCAAGGGCCCCAGCGTGTTCCCCCTGGCCCCTTGTTCCAGGAGCACCTCCGAGAGCACCGCCGCTCTGGGC TGCCTGGTGAAGGACTACTTCCCTGAGCCTGTGACCGTGAGCTGGAACTCCGGCGCTCTGACCTCCGGCGT GCACACCTTCCCCGCCGTGCTGCAGAGCAGCGGCCTGTACAGCCTGAGCTCCGTGGTGACAGTGCCCAGCT CCAGCCTGGGCACCAAGACCTACACCTGCAATGTGGATCACAAGCCCAGCAATACCAAGGTGGACAAGAG GGTGGAGTCCAAGTACGGCCCTCCCTGCCCCTCCTGTCCTGCTCCTGAGTTCCTGGGCGGCCCTTCCGTGTT CCTGTTCCCCCCCAAGCCCAAGGATACCCTGATGATCAGCCGGACCCCTGAGGTGACATGTGTGGTGGTGG ATGTGAGCCAGGAGGACCCTGAGGTGCAGTTCAACTGGTACGTGGATGGCGTGGAGGTGCACAACGCCAA GACCAAGCCTCGGGAGGAGCAGTTCAATAGCACCTACCGGGTGGTGAGCGTGCTGACCGTGCTGCACCAG GATTGGCTGAACGGCAAGGAGTACAAGTGCAAGGTGAGCAACAAGGGCCTGCCCAGCAGCATCGAGAAGA CCATCTCCAAGGCTAAGGGCCAGCCACGGGAGCCTCAGGTGTACACCCTGCCCCCTAGCCAGGAGGAGATG ACCAAGAACCAGGTGAGCCTGACCTGTCTGGTGAAGGGCTTCTACCCCTCCGATATCGCTGTGGAGTGGGA GTCCAACGGCCAGCCCGAGAACAACTATAAGACCACCCCCCCTGTGCTGGACAGCGACGGCAGCTTCTTCC TGTATTCCAGGCTGACCGTGGACAAGAGCCGGTGGCAGGAGGGCAATGTGTTCTCCTGCAGCGTGATGCAC GAGGCCCTGCACAACCACTACACCCAGAAGTCCCTGTCCCTGTCCCTCGGCAAG | 384 |
| Heavy chain-B20 | CAGGTGCAGCTGTTGGAGTCTGGCCCGGGACTGGTGAAGCCTTCGGAGACCCTGTCCCTCACCTGCACTGT CTCTGGTGACTCGATCAGTAATTACTTCTGGAGCTGGATCCGGCAGTCCCCAGGGAAGGGCCTGGAGTGGAT TGGGTACATCCATTATAGTGGGACCGTCAGCTATAACCCGTCCCTCAGGAGTCGAGTCACCATATCAATAGAC ACGTCCAAGAACCAATTCTCCCTGAAGCTGAGCTCTGTGACCGCCGCAGACACGGCCGTGTATTACTGTGC GAGGCGAGGGCGTATAGCAGCAGCTGGTACAGGCTACTACTACTACGGTATGGACGTCTGGGGCCAAGGGA CCACGGTCACCGTCTCATCAGCTTCCACCAAGGGCCCCAGCGTGTTCCCCCTGGCCCCTTGTTCCAGGAGCA CCTCCGAGAGCACCGCCGCTCTGGGCTGCCTGGTGAAGGACTACTTCCCTGAGCCTGTGACCGTGAGCTGG AACTCCGGCGCTCTGACCTCCGGCGTGCACACCTTCCCCGCCGTGCTGCAGAGCAGCGGCCTGTACAGCCT GAGCTCCGTGGTGACAGTGCCCAGCTCCAGCCTGGGCACCAAGACCTACACCTGCAATGTGGATCACAAGC CCAGCAATACCAAGGTGGACAAGAGGGTGGAGTCCAAGTACGGCCCTCCCTGCCCCTCCTGTCCTGCTCCT GAGTTCCTGGGCGGCCCTTCCGTGTTCCTGTTCCCCCCCAAGCCCAAGGATACCCTGATGATCAGCCGGACC CCTGAGGTGACATGTGTGGTGGTGGATGTGAGCCAGGAGGACCCTGAGGTGCAGTTCAACTGGTACGTGGA TGGCGTGGAGGTGCACAACGCCAAGACCAAGCCTCGGGAGGAGCAGTTCAATAGCACCTACCGGGTGGTG AGCGTGCTGACCGTGCTGCACCAGGATTGGCTGAACGGCAAGGAGTACAAGTGCAAGGTGAGCAACAAGG GCCTGCCCAGCAGCATCGAGAAGACCATCTCCAAGGCTAAGGGCCAGCCACGGGAGCCTCAGGTGTACACC CTGCCCCCTAGCCAGGAGGAGATGACCAAGAACCAGGTGAGCCTGACCTGTCTGGTGAAGGGCTTCTACCC CTCCGATATCGCTGTGGAGTGGGAGTCCAACGGCCAGCCCGAGAACAACTATAAGACCACCCCCCCTGTGC TGGACAGCGACGGCAGCTTCTTCCTGTATTCCAGGCTGACCGTGGACAAGAGCCGGTGGCAGGAGGGCAAT GTGTTCTCCTGCAGCGTGATGCACGAGGCCCTGCACAACCACTACACCCAGAAGTCCCTGTCCCTGTCCCTC GGCAAG | 385 |
| Heavy chain-B24 | GAGGTGCAGCTGGTGGAGACTGGGGGAGGCCTGGTCAAGCCTGGGGGGTCCCTGAGACTCTCCTGTGCAG CCTCTGGATTCACCTTCAGTAGCTATAGCATGAACTGGGTCCGCCAGGCTCCAGGGAAGGGGCTGGAGTGG GTCTCATCCATTAGTAGTAGTAGTAGTAGTTACATATACTACGCAGACTCAGTGAAGGGCCGATTCACCATCTCCA GAGACAACGCCAAGAACTCACTGTATCTGCAAATGAACAGCCTGAGAGCCGAGGACACGGCTGTGTATTAC TGTGCGAGAGGTCGCAGACGAGACTTTGACTACTGGGGCCAGGGCACCCTGGTCACCGTCTCATCAGCTTC CACCAAGGGCCCCAGCGTGTTCCCCCTGGCCCCTTGTTCCAGGAGCACCTCCGAGAGCACCGCCGCTCTGG GCTGCCTGGTGAAGGACTACTTCCCTGAGCCTGTGACCGTGAGCTGGAACTCCGGCGCTCTGACCTCCGGC GTGCACACCTTCCCCGCCGTGCTGCAGAGCAGCGGCCTGTACAGCCTGAGCTCCGTGGTGACAGTGCCCAG CTCCAGCCTGGGCACCAAGACCTACACCTGCAATGTGGATCACAAGCCCAGCAATACCAAGGTGGACAAGA GGGTGGAGTCCAAGTACGGCCCTCCCTGCCCCTCCTGTCCTGCTCCTGAGTTCCTGGGCGGCCCTTCCGTGT TCCTGTTCCCCCCCAAGCCCAAGGATACCCTGATGATCAGCCGGACCCCTGAGGTGACATGTGTGGTGGTGG ATGTGAGCCAGGAGGACCCTGAGGTGCAGTTCAACTGGTACGTGGATGGCGTGGAGGTGCACAACGCCAA GACCAAGCCTCGGGAGGAGCAGTTCAATAGCACCTACCGGGTGGTGAGCGTGCTGACCGTGCTGCACCAG GATTGGCTGAACGGCAAGGAGTACAAGTGCAAGGTGAGCAACAAGGGCCTGCCCAGCAGCATCGAGAAGA CCATCTCCAAGGCTAAGGGCCAGCCACGGGAGCCTCAGGTGTACACCCTGCCCCCTAGCCAGGAGGAGATG ACCAAGAACCAGGTGAGCCTGACCTGTCTGGTGAAGGGCTTCTACCCCTCCGATATCGCTGTGGAGTGGGA GTCCAACGGCCAGCCCGAGAACAACTATAAGACCACCCCCCCTGTGCTGGACAGCGACGGCAGCTTCTTCC TGTATTCCAGGCTGACCGTGGACAAGAGCCGGTGGCAGGAGGGCAATGTGTTCTCCTGCAGCGTGATGCAC GAGGCCCTGCACAACCACTACACCCAGAAGTCCCTGTCCCTGTCCCTCGGCAAG | 386 |
| Heavy chain-B29 | CAGGTTCAGCTGGTACAGTCTGGGGCTGAGGTGAAGAAGCCTGGGTCCTCGGTAAAGGTCTCCTGCAAGGC TTCTGGAGACACCTTCAGCAGCTTTGCTATCAGCTGGGTGCGACAGGCCCCTGGACAAGGCCTTGAGTGGA TGGGGACAATCATCCCTCTCTTTACTACTCCAAACTATGCACAGAAATTCCAGGGCAGAGTCACGATTACCG CGGACGAATCCACGCGCACAGCCTACATGGAGCTGAGCAGCCTGAGATCTGAGGACACGGCCGTGTATTAC TGTGCGAGAGGGCCCGAACGTATAGCAGCAGCTGGCTACTACTACTACTACGGTATGGACGTCTGGGGCCAA GGGACCACGGTCACCGTCTCATCAGCTTCCACCAAGGGCCCCAGCGTGTTCCCCCTGGCCCCTTGTTCCAG GAGCACCTCCGAGAGCACCGCCGCTCTGGGCTGCCTGGTGAAGGACTACTTCCCTGAGCCTGTGACCGTGA GCTGGAACTCCGGCGCTCTGACCTCCGGCGTGCACACCTTCCCCGCCGTGCTGCAGAGCAGCGGCCTGTAC AGCCTGAGCTCCGTGGTGACAGTGCCCAGCTCCAGCCTGGGCACCAAGACCTACACCTGCAATGTGGATCA CAAGCCCAGCAATACCAAGGTGGACAAGAGGGTGGAGTCCAAGTACGGCCCTCCCTGCCCCTCCTGTCCTG CTCCTGAGTTCCTGGGCGGCCCTTCCGTGTTCCTGTTCCCCCCCAAGCCCAAGGATACCCTGATGATCAGCC GGACCCCTGAGGTGACATGTGTGGTGGTGGATGTGAGCCAGGAGGACCCTGAGGTGCAGTTCAACTGGTAC GTGGATGGCGTGGAGGTGCACAACGCCAAGACCAAGCCTCGGGAGGAGCAGTTCAATAGCACCTACCGGG TGGTGAGCGTGCTGACCGTGCTGCACCAGGATTGGCTGAACGGCAAGGAGTACAAGTGCAAGGTGAGCAA CAAGGGCCTGCCCAGCAGCATCGAGAAGACCATCTCCAAGGCTAAGGGCCAGCCACGGGAGCCTCAGGTG TACACCCTGCCCCCTAGCCAGGAGGAGATGACCAAGAACCAGGTGAGCCTGACCTGTCTGGTGAAGGGCTT CTACCCCTCCGATATCGCTGTGGAGTGGGAGTCCAACGGCCAGCCCGAGAACAACTATAAGACCACCCCCC CTGTGCTGGACAGCGACGGCAGCTTCTTCCTGTATTCCAGGCTGACCGTGGACAAGAGCCGGTGGCAGGAG GGCAATGTGTTCTCCTGCAGCGTGATGCACGAGGCCCTGCACAACCACTACACCCAGAAGTCCCTGTCCCT GTCCCTCGGCAAG | 387 |
| Heavy chain-B44 | CAGGTACAGCTGGTGCAGTCTGGGGCTGAGGTGAAGAAGCCTGGGGCCTCAGTGAAGGTTTCCTGCAAGG CATCTGGATACACCTTCACCAGCTACTATATGCACTGGGTGCGACAGGCCCCTGGACAAGGGCTTGAGTGGA TGGGAATAATCAACCCTAGTGGTGGTAGCACAAGCTACGCACAGAAGTTCCAGGGCAGAGTCACCATGACC AGGGACACGTCCACGAGCACAGTCTACATGGAGCTGAGCAGCCTGAGATCTGAGGACACGGCCGTGTATTA CTGTGCGAGAGGGGTTTCACGAAGGGGTGCTTTTGATATCTGGGGCCAAGGGACAATGGTCACCGTCTCAT CAGCTTCCACCAAGGGCCCCAGCGTGTTCCCCCTGGCCCCTTGTTCCAGGAGCACCTCCGAGAGCACCGCC GCTCTGGGCTGCCTGGTGAAGGACTACTTCCCTGAGCCTGTGACCGTGAGCTGGAACTCCGGCGCTCTGAC CTCCGGCGTGCACACCTTCCCCGCCGTGCTGCAGAGCAGCGGCCTGTACAGCCTGAGCTCCGTGGTGACAG TGCCCAGCTCCAGCCTGGGCACCAAGACCTACACCTGCAATGTGGATCACAAGCCCAGCAATACCAAGGTG GACAAGAGGGTGGAGTCCAAGTACGGCCCTCCCTGCCCCTCCTGTCCTGCTCCTGAGTTCCTGGGCGGCCC TTCCGTGTTCCTGTTCCCCCCCAAGCCCAAGGATACCCTGATGATCAGCCGGACCCCTGAGGTGACATGTGT GGTGGTGGATGTGAGCCAGGAGGACCCTGAGGTGCAGTTCAACTGGTACGTGGATGGCGTGGAGGTGCAC AACGCCAAGACCAAGCCTCGGGAGGAGCAGTTCAATAGCACCTACCGGGTGGTGAGCGTGCTGACCGTGC TGCACCAGGATTGGCTGAACGGCAAGGAGTACAAGTGCAAGGTGAGCAACAAGGGCCTGCCCAGCAGCAT CGAGAAGACCATCTCCAAGGCTAAGGGCCAGCCACGGGAGCCTCAGGTGTACACCCTGCCCCCTAGCCAGG AGGAGATGACCAAGAACCAGGTGAGCCTGACCTGTCTGGTGAAGGGCTTCTACCCCTCCGATATCGCTGTG GAGTGGGAGTCCAACGGCCAGCCCGAGAACAACTATAAGACCACCCCCCCTGTGCTGGACAGCGACGGCA GCTTCTTCCTGTATTCCAGGCTGACCGTGGACAAGAGCCGGTGGCAGGAGGGCAATGTGTTCTCCTGCAGC GTGATGCACGAGGCCCTGCACAACCACTACACCCAGAAGTCCCTGTCCCTGTCCCTCGGCAAG | 388 |
| Heavy chain-B53 | GAGGTGCAGCTGGTGGAGACTGGGGGAGGCGTGGTCCAGCCTGGGAGGTCCCTGAGACTCTCCTGTGCAG CCTCTGGATTCACCTTCAGTAGCTATGCTATGCACTGGGTCCGCCAGGCTCCAGGCAAGGGGCTGGAGTGGG TGGCAGTTATATCATATGATGGAAGTAATAAATACTACGCAGACTCCGTGAAGGGCCGATTCACCATCTCCAG AGACAATTCCAAGAACACGCTGTATCTGCAAATGAACAGCCTGAGAGCTGAGGACACGGCTGTGTATTACT GTGCGAGAAATCGATACGGGGCCACTGACTACTGGGGCCAGGGAACCCTGGTCACCGTCTCATCAGCTTCC ACCAAGGGCCCCAGCGTGTTCCCCCTGGCCCCTTGTTCCAGGAGCACCTCCGAGAGCACCGCCGCTCTGGG CTGCCTGGTGAAGGACTACTTCCCTGAGCCTGTGACCGTGAGCTGGAACTCCGGCGCTCTGACCTCCGGCG TGCACACCTTCCCCGCCGTGCTGCAGAGCAGCGGCCTGTACAGCCTGAGCTCCGTGGTGACAGTGCCCAGC TCCAGCCTGGGCACCAAGACCTACACCTGCAATGTGGATCACAAGCCCAGCAATACCAAGGTGGACAAGAG GGTGGAGTCCAAGTACGGCCCTCCCTGCCCCTCCTGTCCTGCTCCTGAGTTCCTGGGCGGCCCTTCCGTGTT CCTGTTCCCCCCCAAGCCCAAGGATACCCTGATGATCAGCCGGACCCCTGAGGTGACATGTGTGGTGGTGG ATGTGAGCCAGGAGGACCCTGAGGTGCAGTTCAACTGGTACGTGGATGGCGTGGAGGTGCACAACGCCAA GACCAAGCCTCGGGAGGAGCAGTTCAATAGCACCTACCGGGTGGTGAGCGTGCTGACCGTGCTGCACCAG GATTGGCTGAACGGCAAGGAGTACAAGTGCAAGGTGAGCAACAAGGGCCTGCCCAGCAGCATCGAGAAGA CCATCTCCAAGGCTAAGGGCCAGCCACGGGAGCCTCAGGTGTACACCCTGCCCCCTAGCCAGGAGGAGATG ACCAAGAACCAGGTGAGCCTGACCTGTCTGGTGAAGGGCTTCTACCCCTCCGATATCGCTGTGGAGTGGGA GTCCAACGGCCAGCCCGAGAACAACTATAAGACCACCCCCCCTGTGCTGGACAGCGACGGCAGCTTCTTCC TGTATTCCAGGCTGACCGTGGACAAGAGCCGGTGGCAGGAGGGCAATGTGTTCTCCTGCAGCGTGATGCAC GAGGCCCTGCACAACCACTACACCCAGAAGTCCCTGTCCCTGTCCCTCGGCAAG | 389 |
| Heavy chain-B67 | CAGATCACCTTGAAGGAGTCTGGCCCAGGACTGGTGAAGCCTTCGGGGACCCTGTCCCTCACCTGCGCTGT CTCTGGTGGCTCCATCAGCAGTAGTAACTGGTGGAGTTGGGTCCGCCAGCCCCCAGGGAAGGGGCTGGAGT GGATTGGGGAAATCTATCATAGTGGGAGCACCAACTACAACCCGTCCCTCAAGAGTCGAGTCACCATATCAG TAGACAAGTCCAAGAACCAGTTCTCCCTGAAGCTGAGCTCTGTGACCGCCGCGGACACGGCCGTGTATTAC TGTGCGAGAGATGACGGGTGGCGTTATGGTTCGGTCTACTACTACTACATGGACGTCTGGGGCAAAGGGACC ACGGTCACCGTCTCATCAGCTTCCACCAAGGGCCCCAGCGTGTTCCCCCTGGCCCCTTGTTCCAGGAGCACC TCCGAGAGCACCGCCGCTCTGGGCTGCCTGGTGAAGGACTACTTCCCTGAGCCTGTGACCGTGAGCTGGAA CTCCGGCGCTCTGACCTCCGGCGTGCACACCTTCCCCGCCGTGCTGCAGAGCAGCGGCCTGTACAGCCTGA GCTCCGTGGTGACAGTGCCCAGCTCCAGCCTGGGCACCAAGACCTACACCTGCAATGTGGATCACAAGCCC AGCAATACCAAGGTGGACAAGAGGGTGGAGTCCAAGTACGGCCCTCCCTGCCCCTCCTGTCCTGCTCCTGA GTTCCTGGGCGGCCCTTCCGTGTTCCTGTTCCCCCCCAAGCCCAAGGATACCCTGATGATCAGCCGGACCCC TGAGGTGACATGTGTGGTGGTGGATGTGAGCCAGGAGGACCCTGAGGTGCAGTTCAACTGGTACGTGGATG GCGTGGAGGTGCACAACGCCAAGACCAAGCCTCGGGAGGAGCAGTTCAATAGCACCTACCGGGTGGTGAG CGTGCTGACCGTGCTGCACCAGGATTGGCTGAACGGCAAGGAGTACAAGTGCAAGGTGAGCAACAAGGGC CTGCCCAGCAGCATCGAGAAGACCATCTCCAAGGCTAAGGGCCAGCCACGGGAGCCTCAGGTGTACACCCT GCCCCCTAGCCAGGAGGAGATGACCAAGAACCAGGTGAGCCTGACCTGTCTGGTGAAGGGCTTCTACCCCT CCGATATCGCTGTGGAGTGGGAGTCCAACGGCCAGCCCGAGAACAACTATAAGACCACCCCCCCTGTGCTG GACAGCGACGGCAGCTTCTTCCTGTATTCCAGGCTGACCGTGGACAAGAGCCGGTGGCAGGAGGGCAATGT GTTCTCCTGCAGCGTGATGCACGAGGCCCTGCACAACCACTACACCCAGAAGTCCCTGTCCCTGTCCCTCG GCAAG | 390 |
| Heavy chain-B83 | GAGGTGCAGCTGTTGGAGTCTGGGGGAGGCTTGGTACAGCCTGGGGGGTCCCTGAGACTCTCCTGTGCAGC CTCTGGATTCACCTTTAGCAGCTATGCCATGAGCTGGGTCCGCCAGGCTCCAGGGAAGGGGCTGGAGTGGG TCTCAGCTATTAGTGGTAGTGGTGGTAGAACATACTACGCAGACTCCGTGAAGGGCCGGTTCACCATCTCCA GAGACAATTCCAAGAACACGCTGTATCTGCAAATGAACAGCCTGAGAGCCGAGGACACGGCCGTATATTAC TGTGCGAGAGTGTTACAATATAGCGGGACCGACGATTTGGACGTCTGGGGCCGAGGGACCCTGGTCACCGT CTCATCAGCTTCCACCAAGGGCCCCAGCGTGTTCCCCCTGGCCCCTTGTTCCAGGAGCACCTCCGAGAGCA CCGCCGCTCTGGGCTGCCTGGTGAAGGACTACTTCCCTGAGCCTGTGACCGTGAGCTGGAACTCCGGCGCT CTGACCTCCGGCGTGCACACCTTCCCCGCCGTGCTGCAGAGCAGCGGCCTGTACAGCCTGAGCTCCGTGGT GACAGTGCCCAGCTCCAGCCTGGGCACCAAGACCTACACCTGCAATGTGGATCACAAGCCCAGCAATACCA AGGTGGACAAGAGGGTGGAGTCCAAGTACGGCCCTCCCTGCCCCTCCTGTCCTGCTCCTGAGTTCCTGGGC GGCCCTTCCGTGTTCCTGTTCCCCCCCAAGCCCAAGGATACCCTGATGATCAGCCGGACCCCTGAGGTGACA TGTGTGGTGGTGGATGTGAGCCAGGAGGACCCTGAGGTGCAGTTCAACTGGTACGTGGATGGCGTGGAGGT GCACAACGCCAAGACCAAGCCTCGGGAGGAGCAGTTCAATAGCACCTACCGGGTGGTGAGCGTGCTGACC GTGCTGCACCAGGATTGGCTGAACGGCAAGGAGTACAAGTGCAAGGTGAGCAACAAGGGCCTGCCCAGCA GCATCGAGAAGACCATCTCCAAGGCTAAGGGCCAGCCACGGGAGCCTCAGGTGTACACCCTGCCCCCTAGC CAGGAGGAGATGACCAAGAACCAGGTGAGCCTGACCTGTCTGGTGAAGGGCTTCTACCCCTCCGATATCGC TGTGGAGTGGGAGTCCAACGGCCAGCCCGAGAACAACTATAAGACCACCCCCCCTGTGCTGGACAGCGAC GGCAGCTTCTTCCTGTATTCCAGGCTGACCGTGGACAAGAGCCGGTGGCAGGAGGGCAATGTGTTCTCCTG CAGCGTGATGCACGAGGCCCTGCACAACCACTACACCCAGAAGTCCCTGTCCCTGTCCCTCGGCAAG | 391 |
| Heavy chain-B88 | CAGGTGCAGCTGGTGCAATCTGGGGCTGAGGTGAAGAAGCCTGGGTCCTCGGTGAAGGTCTCCTGCAAGG CTTCTGGAGGCACCTTCAGCAGCTATACTATCAGCTGGGTGCGACAGGCCCCTGGACAAGGGCTTGAGTGG ATGGGAAGGATCATCCCTATCCTTGGTATAGCAAACTACGCACAGAAGTTCCAGGGCAGAGTCACGATTACC GCGGACAAATCCACGAGCACAGCCTACATGGAGCTGAGCAGCCTGAGATCTGAGGACACGGCCGTGTATTA CTGTGCGACGGGGGTTGGTATGGTTCGGGGAGACCCTTGGGGCCAGGGAACCCTGGTCACCGTCTCATCAG CTTCCACCAAGGGCCCCAGCGTGTTCCCCCTGGCCCCTTGTTCCAGGAGCACCTCCGAGAGCACCGCCGCT CTGGGCTGCCTGGTGAAGGACTACTTCCCTGAGCCTGTGACCGTGAGCTGGAACTCCGGCGCTCTGACCTC CGGCGTGCACACCTTCCCCGCCGTGCTGCAGAGCAGCGGCCTGTACAGCCTGAGCTCCGTGGTGACAGTGC CCAGCTCCAGCCTGGGCACCAAGACCTACACCTGCAATGTGGATCACAAGCCCAGCAATACCAAGGTGGAC AAGAGGGTGGAGTCCAAGTACGGCCCTCCCTGCCCCTCCTGTCCTGCTCCTGAGTTCCTGGGCGGCCCTTCC GTGTTCCTGTTCCCCCCCAAGCCCAAGGATACCCTGATGATCAGCCGGACCCCTGAGGTGACATGTGTGGTG GTGGATGTGAGCCAGGAGGACCCTGAGGTGCAGTTCAACTGGTACGTGGATGGCGTGGAGGTGCACAACG CCAAGACCAAGCCTCGGGAGGAGCAGTTCAATAGCACCTACCGGGTGGTGAGCGTGCTGACCGTGCTGCAC CAGGATTGGCTGAACGGCAAGGAGTACAAGTGCAAGGTGAGCAACAAGGGCCTGCCCAGCAGCATCGAGA AGACCATCTCCAAGGCTAAGGGCCAGCCACGGGAGCCTCAGGTGTACACCCTGCCCCCTAGCCAGGAGGA GATGACCAAGAACCAGGTGAGCCTGACCTGTCTGGTGAAGGGCTTCTACCCCTCCGATATCGCTGTGGAGT GGGAGTCCAACGGCCAGCCCGAGAACAACTATAAGACCACCCCCCCTGTGCTGGACAGCGACGGCAGCTT CTTCCTGTATTCCAGGCTGACCGTGGACAAGAGCCGGTGGCAGGAGGGCAATGTGTTCTCCTGCAGCGTGA TGCACGAGGCCCTGCACAACCACTACACCCAGAAGTCCCTGTCCCTGTCCCTCGGCAAG | 392 |
| Heavy chain-B90 | GAGGTGCAGCTGTTGGAGTCTGGGGGAGGCCTGGTCAAGCCTGGGGGGTCCCTGAGACTCTCCTGTGCAG CCTCTGGACTCATCTTCAAAAACTATGGCATGAACTGGGTCCGCCTGGCTCCGGGGAAGGGGCTGGAGTGG GTTGCATCCATTAGTGGTAATAGTAACTACATCTCGTATGCAGACTCAGTGAAGGGCCGATTCACCATCTCCA GAGACAACGCCAACAACTCACTGTTTCTGCAGATGAGCAGCCTGAGAGCCGACGACACGGCTGTCTATTAT TGTTCGAGTGGTTATTTCTACTTTGACCTCTGGGGCCCGGGAACCCTGGTCACCGTCTCATCAGCTTCCACCA AGGGCCCCAGCGTGTTCCCCCTGGCCCCTTGTTCCAGGAGCACCTCCGAGAGCACCGCCGCTCTGGGCTGC CTGGTGAAGGACTACTTCCCTGAGCCTGTGACCGTGAGCTGGAACTCCGGCGCTCTGACCTCCGGCGTGCA CACCTTCCCCGCCGTGCTGCAGAGCAGCGGCCTGTACAGCCTGAGCTCCGTGGTGACAGTGCCCAGCTCCA GCCTGGGCACCAAGACCTACACCTGCAATGTGGATCACAAGCCCAGCAATACCAAGGTGGACAAGAGGGT GGAGTCCAAGTACGGCCCTCCCTGCCCCTCCTGTCCTGCTCCTGAGTTCCTGGGCGGCCCTTCCGTGTTCCT GTTCCCCCCCAAGCCCAAGGATACCCTGATGATCAGCCGGACCCCTGAGGTGACATGTGTGGTGGTGGATGT GAGCCAGGAGGACCCTGAGGTGCAGTTCAACTGGTACGTGGATGGCGTGGAGGTGCACAACGCCAAGACC AAGCCTCGGGAGGAGCAGTTCAATAGCACCTACCGGGTGGTGAGCGTGCTGACCGTGCTGCACCAGGATTG GCTGAACGGCAAGGAGTACAAGTGCAAGGTGAGCAACAAGGGCCTGCCCAGCAGCATCGAGAAGACCATC TCCAAGGCTAAGGGCCAGCCACGGGAGCCTCAGGTGTACACCCTGCCCCCTAGCCAGGAGGAGATGACCA AGAACCAGGTGAGCCTGACCTGTCTGGTGAAGGGCTTCTACCCCTCCGATATCGCTGTGGAGTGGGAGTCC AACGGCCAGCCCGAGAACAACTATAAGACCACCCCCCCTGTGCTGGACAGCGACGGCAGCTTCTTCCTGTA TTCCAGGCTGACCGTGGACAAGAGCCGGTGGCAGGAGGGCAATGTGTTCTCCTGCAGCGTGATGCACGAG GCCCTGCACAACCACTACACCCAGAAGTCCCTGTCCCTGTCCCTCGGCAAG | 393 |
| Heavy chain-B109 | GAGGTGCAGCTGTTGGAGTCTGGGGGAGGCCTGGTCAAGCCTGGGGGGTCCCTGAGACTCTCCTGTGCAG CCTCTGGATTCACCTTCAGTAGCTATAGCATGAACTGGGTCCGCCAGGCTCCAGGGAAGGGGCTGGAGTGG GTCTCATCCATTAGTAGTAGTAGTAGTTACATATACTACGCAGACTCAGTGAAGGGCCGATTCACCATCTCCA GAGACAACGCCAAGAACTCACTGTATCTGCAAATGAACAGCCTGAGAGACGGGGACACGGCCATATACTAC TGTGTGAGACGCCTTTGGGAAGCTCCGGGGATTGGGCCTTTTGATGTCTGGGGCCACGGGACAATGGTCAC CGTCTCATCAGCTTCCACCAAGGGCCCCAGCGTGTTCCCCCTGGCCCCTTGTTCCAGGAGCACCTCCGAGA GCACCGCCGCTCTGGGCTGCCTGGTGAAGGACTACTTCCCTGAGCCTGTGACCGTGAGCTGGAACTCCGGC GCTCTGACCTCCGGCGTGCACACCTTCCCCGCCGTGCTGCAGAGCAGCGGCCTGTACAGCCTGAGCTCCGT GGTGACAGTGCCCAGCTCCAGCCTGGGCACCAAGACCTACACCTGCAATGTGGATCACAAGCCCAGCAATA CCAAGGTGGACAAGAGGGTGGAGTCCAAGTACGGCCCTCCCTGCCCCTCCTGTCCTGCTCCTGAGTTCCTG GGCGGCCCTTCCGTGTTCCTGTTCCCCCCCAAGCCCAAGGATACCCTGATGATCAGCCGGACCCCTGAGGTG ACATGTGTGGTGGTGGATGTGAGCCAGGAGGACCCTGAGGTGCAGTTCAACTGGTACGTGGATGGCGTGGA GGTGCACAACGCCAAGACCAAGCCTCGGGAGGAGCAGTTCAATAGCACCTACCGGGTGGTGAGCGTGCTG ACCGTGCTGCACCAGGATTGGCTGAACGGCAAGGAGTACAAGTGCAAGGTGAGCAACAAGGGCCTGCCCA GCAGCATCGAGAAGACCATCTCCAAGGCTAAGGGCCAGCCACGGGAGCCTCAGGTGTACACCCTGCCCCCT AGCCAGGAGGAGATGACCAAGAACCAGGTGAGCCTGACCTGTCTGGTGAAGGGCTTCTACCCCTCCGATAT CGCTGTGGAGTGGGAGTCCAACGGCCAGCCCGAGAACAACTATAAGACCACCCCCCCTGTGCTGGACAGC GACGGCAGCTTCTTCCTGTATTCCAGGCTGACCGTGGACAAGAGCCGGTGGCAGGAGGGCAATGTGTTCTC CTGCAGCGTGATGCACGAGGCCCTGCACAACCACTACACCCAGAAGTCCCTGTCCCTGTCCCTCGGCAAG | 394 |
| Heavy chain-B114 | GAGGTGCAGCTGTTGGAGTCTGGGGGAGGCTTGGTCCAGCCTGGGGGGTCCCTGAAACTCTCCTGTGCAGC CTCTGGGTTCACCTTCAGTGGCTATGCTATGCACTGGGTCCGCCAGGCTCCAGGCAAGGGGCTGGAGTGGG TGGCAGTTATATCATATGATGGAAGTAATAAATACTACGCAGACTCCGTGAAGGGCCGATTCACCATCTCCAG AGACAATTCCAAGAACACGCTGTATCTGCAAATGAACAGCCTGAGAGCTGAGGACACGGCTGTGTATTACT GTGCGAGAGGAAGGGGGAGGGGGGTCTTTGACTACTGGGGCCAGGGAACCCTGGTCACCGTCTCATCAGC TTCCACCAAGGGCCCCAGCGTGTTCCCCCTGGCCCCTTGTTCCAGGAGCACCTCCGAGAGCACCGCCGCTC TGGGCTGCCTGGTGAAGGACTACTTCCCTGAGCCTGTGACCGTGAGCTGGAACTCCGGCGCTCTGACCTCC GGCGTGCACACCTTCCCCGCCGTGCTGCAGAGCAGCGGCCTGTACAGCCTGAGCTCCGTGGTGACAGTGCC CAGCTCCAGCCTGGGCACCAAGACCTACACCTGCAATGTGGATCACAAGCCCAGCAATACCAAGGTGGACA AGAGGGTGGAGTCCAAGTACGGCCCTCCCTGCCCCTCCTGTCCTGCTCCTGAGTTCCTGGGCGGCCCTTCCG TGTTCCTGTTCCCCCCCAAGCCCAAGGATACCCTGATGATCAGCCGGACCCCTGAGGTGACATGTGTGGTGG TGGATGTGAGCCAGGAGGACCCTGAGGTGCAGTTCAACTGGTACGTGGATGGCGTGGAGGTGCACAACGC CAAGACCAAGCCTCGGGAGGAGCAGTTCAATAGCACCTACCGGGTGGTGAGCGTGCTGACCGTGCTGCACC AGGATTGGCTGAACGGCAAGGAGTACAAGTGCAAGGTGAGCAACAAGGGCCTGCCCAGCAGCATCGAGAA GACCATCTCCAAGGCTAAGGGCCAGCCACGGGAGCCTCAGGTGTACACCCTGCCCCCTAGCCAGGAGGAG ATGACCAAGAACCAGGTGAGCCTGACCTGTCTGGTGAAGGGCTTCTACCCCTCCGATATCGCTGTGGAGTG GGAGTCCAACGGCCAGCCCGAGAACAACTATAAGACCACCCCCCCTGTGCTGGACAGCGACGGCAGCTTCT TCCTGTATTCCAGGCTGACCGTGGACAAGAGCCGGTGGCAGGAGGGCAATGTGTTCTCCTGCAGCGTGATG CACGAGGCCCTGCACAACCACTACACCCAGAAGTCCCTGTCCCTGTCCCTCGGCAAG | 395 |
| Heavy chain-B117 | GAGGTGCAGCTGTTGGAGTCTGGGGGAGGCTTGGTACAGCCTGGGGGGTCCCTGAGACTCTCCTGTGCAGC CTCTGGATTCACCTTCAGTAGCTATAGCATGAACTGGGTCCGCCAGGCTCCAGGGAAGGGGCTGGAGTGGG TTTCATACATTAGTAGTAGTAGTAGTACCATATACTACGCAGACTCTGTGAAGGGCCGATTCACCATCTCCAG AGACAATGCCAAGAACTCACTGTATCTGCAAATGAACAGCCTGAGAGACGAGGACACGGCTGTGTATTACT GTGCGAGAGATCGGACGCTGGGCTACTGGGGCCAGGGAACCCTGGTCACCGTCTCATCAGCTTCCACCAAG GGCCCCAGCGTGTTCCCCCTGGCCCCTTGTTCCAGGAGCACCTCCGAGAGCACCGCCGCTCTGGGCTGCCT GGTGAAGGACTACTTCCCTGAGCCTGTGACCGTGAGCTGGAACTCCGGCGCTCTGACCTCCGGCGTGCACA CCTTCCCCGCCGTGCTGCAGAGCAGCGGCCTGTACAGCCTGAGCTCCGTGGTGACAGTGCCCAGCTCCAGC CTGGGCACCAAGACCTACACCTGCAATGTGGATCACAAGCCCAGCAATACCAAGGTGGACAAGAGGGTGG AGTCCAAGTACGGCCCTCCCTGCCCCTCCTGTCCTGCTCCTGAGTTCCTGGGCGGCCCTTCCGTGTTCCTGT TCCCCCCCAAGCCCAAGGATACCCTGATGATCAGCCGGACCCCTGAGGTGACATGTGTGGTGGTGGATGTG AGCCAGGAGGACCCTGAGGTGCAGTTCAACTGGTACGTGGATGGCGTGGAGGTGCACAACGCCAAGACCA AGCCTCGGGAGGAGCAGTTCAATAGCACCTACCGGGTGGTGAGCGTGCTGACCGTGCTGCACCAGGATTGG CTGAACGGCAAGGAGTACAAGTGCAAGGTGAGCAACAAGGGCCTGCCCAGCAGCATCGAGAAGACCATCT CCAAGGCTAAGGGCCAGCCACGGGAGCCTCAGGTGTACACCCTGCCCCCTAGCCAGGAGGAGATGACCAA GAACCAGGTGAGCCTGACCTGTCTGGTGAAGGGCTTCTACCCCTCCGATATCGCTGTGGAGTGGGAGTCCA ACGGCCAGCCCGAGAACAACTATAAGACCACCCCCCCTGTGCTGGACAGCGACGGCAGCTTCTTCCTGTAT TCCAGGCTGACCGTGGACAAGAGCCGGTGGCAGGAGGGCAATGTGTTCTCCTGCAGCGTGATGCACGAGG CCCTGCACAACCACTACACCCAGAAGTCCCTGTCCCTGTCCCTCGGCAAG | 396 |
| Heavy chain-B123 | CAGGTGCAGCTACAGCAGTGGGGCGCAGGACTGTTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACTGCCGCGGACACGGCCGTGTATT ACTGTGCGAGATGGGACCGAGTCAGAGAGGGAGGCCGGTTCTCAAATGCTTTTGATATCTGGGGCCAAGGG ACAATGGTCACCGTCTCATCAGCTTCCACCAAGGGCCCCAGCGTGTTCCCCCTGGCCCCTTGTTCCAGGAGC ACCTCCGAGAGCACCGCCGCTCTGGGCTGCCTGGTGAAGGACTACTTCCCTGAGCCTGTGACCGTGAGCTG GAACTCCGGCGCTCTGACCTCCGGCGTGCACACCTTCCCCGCCGTGCTGCAGAGCAGCGGCCTGTACAGCC TGAGCTCCGTGGTGACAGTGCCCAGCTCCAGCCTGGGCACCAAGACCTACACCTGCAATGTGGATCACAAG CCCAGCAATACCAAGGTGGACAAGAGGGTGGAGTCCAAGTACGGCCCTCCCTGCCCCTCCTGTCCTGCTCC TGAGTTCCTGGGCGGCCCTTCCGTGTTCCTGTTCCCCCCCAAGCCCAAGGATACCCTGATGATCAGCCGGAC CCCTGAGGTGACATGTGTGGTGGTGGATGTGAGCCAGGAGGACCCTGAGGTGCAGTTCAACTGGTACGTGG ATGGCGTGGAGGTGCACAACGCCAAGACCAAGCCTCGGGAGGAGCAGTTCAATAGCACCTACCGGGTGGT GAGCGTGCTGACCGTGCTGCACCAGGATTGGCTGAACGGCAAGGAGTACAAGTGCAAGGTGAGCAACAAG GGCCTGCCCAGCAGCATCGAGAAGACCATCTCCAAGGCTAAGGGCCAGCCACGGGAGCCTCAGGTGTACA CCCTGCCCCCTAGCCAGGAGGAGATGACCAAGAACCAGGTGAGCCTGACCTGTCTGGTGAAGGGCTTCTAC CCCTCCGATATCGCTGTGGAGTGGGAGTCCAACGGCCAGCCCGAGAACAACTATAAGACCACCCCCCCTGT GCTGGACAGCGACGGCAGCTTCTTCCTGTATTCCAGGCTGACCGTGGACAAGAGCCGGTGGCAGGAGGGC AATGTGTTCTCCTGCAGCGTGATGCACGAGGCCCTGCACAACCACTACACCCAGAAGTCCCTGTCCCTGTCC CTCGGCAAG | 397 |
| Heavy chain-B145 | CAGCTGCAGCTGCAGGAGTCGGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACTGCCGCGGACACGGCCGTGTATT ACTGTGCGAGGGGTAAAAGAGATGGCTACAATTACTACTTTGACTACTGGGGCCAGGGAACCCTGGTCACC GTCTCATCAGCTTCCACCAAGGGCCCCAGCGTGTTCCCCCTGGCCCCTTGTTCCAGGAGCACCTCCGAGAG CACCGCCGCTCTGGGCTGCCTGGTGAAGGACTACTTCCCTGAGCCTGTGACCGTGAGCTGGAACTCCGGCG CTCTGACCTCCGGCGTGCACACCTTCCCCGCCGTGCTGCAGAGCAGCGGCCTGTACAGCCTGAGCTCCGTG GTGACAGTGCCCAGCTCCAGCCTGGGCACCAAGACCTACACCTGCAATGTGGATCACAAGCCCAGCAATAC CAAGGTGGACAAGAGGGTGGAGTCCAAGTACGGCCCTCCCTGCCCCTCCTGTCCTGCTCCTGAGTTCCTGG GCGGCCCTTCCGTGTTCCTGTTCCCCCCCAAGCCCAAGGATACCCTGATGATCAGCCGGACCCCTGAGGTGA CATGTGTGGTGGTGGATGTGAGCCAGGAGGACCCTGAGGTGCAGTTCAACTGGTACGTGGATGGCGTGGAG GTGCACAACGCCAAGACCAAGCCTCGGGAGGAGCAGTTCAATAGCACCTACCGGGTGGTGAGCGTGCTGA CCGTGCTGCACCAGGATTGGCTGAACGGCAAGGAGTACAAGTGCAAGGTGAGCAACAAGGGCCTGCCCAG CAGCATCGAGAAGACCATCTCCAAGGCTAAGGGCCAGCCACGGGAGCCTCAGGTGTACACCCTGCCCCCTA GCCAGGAGGAGATGACCAAGAACCAGGTGAGCCTGACCTGTCTGGTGAAGGGCTTCTACCCCTCCGATATC GCTGTGGAGTGGGAGTCCAACGGCCAGCCCGAGAACAACTATAAGACCACCCCCCCTGTGCTGGACAGCG ACGGCAGCTTCTTCCTGTATTCCAGGCTGACCGTGGACAAGAGCCGGTGGCAGGAGGGCAATGTGTTCTCC TGCAGCGTGATGCACGAGGCCCTGCACAACCACTACACCCAGAAGTCCCTGTCCCTGTCCCTCGGCAAG | 398 |
| Heavy chain-B156 | CAGGTACAGCTGCAGCAGTCGGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACCGCCGCAGACACGGCTGTGTATT ACTGTGCGAGACAGAGGCGTGCCAACGGGTATAGCAGCAGCTGGGAATTTGACTACTGGGGCCAGGGAAC CCTGGTCACCGTCTCATCAGCTTCCACCAAGGGCCCCAGCGTGTTCCCCCTGGCCCCTTGTTCCAGGAGCAC CTCCGAGAGCACCGCCGCTCTGGGCTGCCTGGTGAAGGACTACTTCCCTGAGCCTGTGACCGTGAGCTGGA ACTCCGGCGCTCTGACCTCCGGCGTGCACACCTTCCCCGCCGTGCTGCAGAGCAGCGGCCTGTACAGCCTG AGCTCCGTGGTGACAGTGCCCAGCTCCAGCCTGGGCACCAAGACCTACACCTGCAATGTGGATCACAAGCC CAGCAATACCAAGGTGGACAAGAGGGTGGAGTCCAAGTACGGCCCTCCCTGCCCCTCCTGTCCTGCTCCTG AGTTCCTGGGCGGCCCTTCCGTGTTCCTGTTCCCCCCCAAGCCCAAGGATACCCTGATGATCAGCCGGACCC CTGAGGTGACATGTGTGGTGGTGGATGTGAGCCAGGAGGACCCTGAGGTGCAGTTCAACTGGTACGTGGAT GGCGTGGAGGTGCACAACGCCAAGACCAAGCCTCGGGAGGAGCAGTTCAATAGCACCTACCGGGTGGTGA GCGTGCTGACCGTGCTGCACCAGGATTGGCTGAACGGCAAGGAGTACAAGTGCAAGGTGAGCAACAAGGG CCTGCCCAGCAGCATCGAGAAGACCATCTCCAAGGCTAAGGGCCAGCCACGGGAGCCTCAGGTGTACACCC TGCCCCCTAGCCAGGAGGAGATGACCAAGAACCAGGTGAGCCTGACCTGTCTGGTGAAGGGCTTCTACCCC TCCGATATCGCTGTGGAGTGGGAGTCCAACGGCCAGCCCGAGAACAACTATAAGACCACCCCCCCTGTGCT GGACAGCGACGGCAGCTTCTTCCTGTATTCCAGGCTGACCGTGGACAAGAGCCGGTGGCAGGAGGGCAAT GTGTTCTCCTGCAGCGTGATGCACGAGGCCCTGCACAACCACTACACCCAGAAGTCCCTGTCCCTGTCCCTC | 399 |
| | GGCAAG | |
| Heavy chain-B201 | GAGGTACAACTGGTGGAGTCTGGGGGCGGCTTGGTCCAGCCTGGGGGGTCCCTGAGACTCTCCTGTGCAGC CTCTGGATTCACCTTCAGTAGCTATGCTATGCACTGGGTCCGCCAGGCTCCAGGGAAGGGGCTGGAGTGGGT CTCAGCTATTAGTGGTAGTGGTGATAGCACATACTACGCAGACTCCGTGAAGGGCCGGTTCAGCATCTCCAG AGACAATTCCAAGAACACGCTGTATCTGCAAATGAACAGCCTGAGAGCCGAGGACACGGCCGTATATTACT GTGCGAAAGGGAGTAGGAGGAGTGGCTTCAACCTTTTTGACTACTGGGGCCAGGGCACCCTGGTCACCGTC TCATCAGCTTCCACCAAGGGCCCCAGCGTGTTCCCCCTGGCCCCTTGTTCCAGGAGCACCTCCGAGAGCAC CGCCGCTCTGGGCTGCCTGGTGAAGGACTACTTCCCTGAGCCTGTGACCGTGAGCTGGAACTCCGGCGCTC TGACCTCCGGCGTGCACACCTTCCCCGCCGTGCTGCAGAGCAGCGGCCTGTACAGCCTGAGCTCCGTGGTG ACAGTGCCCAGCTCCAGCCTGGGCACCAAGACCTACACCTGCAATGTGGATCACAAGCCCAGCAATACCAA GGTGGACAAGAGGGTGGAGTCCAAGTACGGCCCTCCCTGCCCCTCCTGTCCTGCTCCTGAGTTCCTGGGCG GCCCTTCCGTGTTCCTGTTCCCCCCCAAGCCCAAGGATACCCTGATGATCAGCCGGACCCCTGAGGTGACAT GTGTGGTGGTGGATGTGAGCCAGGAGGACCCTGAGGTGCAGTTCAACTGGTACGTGGATGGCGTGGAGGTG CACAACGCCAAGACCAAGCCTCGGGAGGAGCAGTTCAATAGCACCTACCGGGTGGTGAGCGTGCTGACCG TGCTGCACCAGGATTGGCTGAACGGCAAGGAGTACAAGTGCAAGGTGAGCAACAAGGGCCTGCCCAGCAG CATCGAGAAGACCATCTCCAAGGCTAAGGGCCAGCCACGGGAGCCTCAGGTGTACACCCTGCCCCCTAGCC AGGAGGAGATGACCAAGAACCAGGTGAGCCTGACCTGTCTGGTGAAGGGCTTCTACCCCTCCGATATCGCT GTGGAGTGGGAGTCCAACGGCCAGCCCGAGAACAACTATAAGACCACCCCCCCTGTGCTGGACAGCGACG GCAGCTTCTTCCTGTATTCCAGGCTGACCGTGGACAAGAGCCGGTGGCAGGAGGGCAATGTGTTCTCCTGC AGCGTGATGCACGAGGCCCTGCACAACCACTACACCCAGAAGTCCCTGTCCCTGTCCCTCGGCAAG | 400 |
| Heavy chain-B307 | CAGCTGCAGCTGCAGGAGTCGGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACTGCCGCGGACACGGCCGTGTATT ACTGTGCGAGGATGGGCCGTATAGCAGCATCCAACTACTACGGTATGGACGTCTGGGGCCAAGGGACCACG GTCACCGTCTCATCAGCTTCCACCAAGGGCCCCAGCGTGTTCCCCCTGGCCCCTTGTTCCAGGAGCACCTCC GAGAGCACCGCCGCTCTGGGCTGCCTGGTGAAGGACTACTTCCCTGAGCCTGTGACCGTGAGCTGGAACTC CGGCGCTCTGACCTCCGGCGTGCACACCTTCCCCGCCGTGCTGCAGAGCAGCGGCCTGTACAGCCTGAGCT CCGTGGTGACAGTGCCCAGCTCCAGCCTGGGCACCAAGACCTACACCTGCAATGTGGATCACAAGCCCAGC AATACCAAGGTGGACAAGAGGGTGGAGTCCAAGTACGGCCCTCCCTGCCCCTCCTGTCCTGCTCCTGAGTT CCTGGGCGGCCCTTCCGTGTTCCTGTTCCCCCCCAAGCCCAAGGATACCCTGATGATCAGCCGGACCCCTGA GGTGACATGTGTGGTGGTGGATGTGAGCCAGGAGGACCCTGAGGTGCAGTTCAACTGGTACGTGGATGGCG TGGAGGTGCACAACGCCAAGACCAAGCCTCGGGAGGAGCAGTTCAATAGCACCTACCGGGTGGTGAGCGT GCTGACCGTGCTGCACCAGGATTGGCTGAACGGCAAGGAGTACAAGTGCAAGGTGAGCAACAAGGGCCTG CCCAGCAGCATCGAGAAGACCATCTCCAAGGCTAAGGGCCAGCCACGGGAGCCTCAGGTGTACACCCTGCC CCCTAGCCAGGAGGAGATGACCAAGAACCAGGTGAGCCTGACCTGTCTGGTGAAGGGCTTCTACCCCTCCG ATATCGCTGTGGAGTGGGAGTCCAACGGCCAGCCCGAGAACAACTATAAGACCACCCCCCCTGTGCTGGAC AGCGACGGCAGCTTCTTCCTGTATTCCAGGCTGACCGTGGACAAGAGCCGGTGGCAGGAGGGCAATGTGTT CTCCTGCAGCGTGATGCACGAGGCCCTGCACAACCACTACACCCAGAAGTCCCTGTCCCTGTCCCTCGGCA AG | 401 |
| Heavy chain-A2 (IgG4SP) | CAGGTACAGCTGCAGCAGTCGGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACCGCCGCAGACACGGCTGTGTATT ACTGTGCGAGACAGAGGCGTGCCAACGGGTATAGCAGCAGCTGGGAATTTGACTACTGGGGCCAGGGCAC CCTGGTCACCGTCTCATCAGCTTCCACCAAGGGCCCCAGCGTGTTCCCCCTGGCCCCTTGTTCCAGGAGCAC CTCCGAGAGCACCGCCGCTCTGGGCTGCCTGGTGAAGGACTACTTCCCTGAGCCTGTGACCGTGAGCTGGA ACTCCGGCGCTCTGACCTCCGGCGTGCACACCTTCCCCGCCGTGCTGCAGAGCAGCGGCCTGTACAGCCTG AGCTCCGTGGTGACAGTGCCCAGCTCCAGCCTGGGCACCAAGACCTACACCTGCAATGTGGATCACAAGCC CAGCAATACCAAGGTGGACAAGAGGGTGGAGTCCAAGTACGGCCCTCCCTGCCCCCCCTGTCCTGCTCCTG AGTTCCTGGGCGGCCCTTCCGTGTTCCTGTTCCCCCCCAAGCCCAAGGATACCCTGATGATCAGCCGGACCC CTGAGGTGACATGTGTGGTGGTGGATGTGAGCCAGGAGGACCCTGAGGTGCAGTTCAACTGGTACGTGGAT GGCGTGGAGGTGCACAACGCCAAGACCAAGCCTCGGGAGGAGCAGTTCAATAGCACCTACCGGGTGGTGA GCGTGCTGACCGTGCTGCACCAGGATTGGCTGAACGGCAAGGAGTACAAGTGCAAGGTGAGCAACAAGGG CCTGCCCAGCAGCATCGAGAAGACCATCTCCAAGGCTAAGGGCCAGCCACGGGAGCCTCAGGTGTACACCC TGCCCCCTAGCCAGGAGGAGATGACCAAGAACCAGGTGAGCCTGACCTGTCTGGTGAAGGGCTTCTACCCC TCCGATATCGCTGTGGAGTGGGAGTCCAACGGCCAGCCCGAGAACAACTATAAGACCACCCCCCCTGTGCT GGACAGCGACGGCAGCTTCTTCCTGTATTCCAGGCTGACCGTGGACAAGAGCCGGTGGCAGGAGGGCAAT GTGTTCTCCTGCAGCGTGATGCACGAGGCCCTGCACAACCACTACACCCAGAAGTCCCTGTCCCTGTCCCTC GGCAAG | 402 |
| Heavy chain-A5 (IgG4SP) | CAGGTGCAGCTGGTACAGTCTGGGGGAGGCGTGGTCCAGCCTGGGAACTCCCTGAGACTCTCCTGTGTAGC CTCTGGATTCACCTTCAGTAGGTACACCATCCACTGGGTCCGCCAGGCTCCAGGCAAGGGGCTGGAGTGGG TGGCAGTTATATCATATGATGGAAGCAATAAATACTACGCAGACTCCGTGAAGGGCCGATTCACCATCTCCAG AGACAATTCCAAGAACACGCTGTATCTGCAAATGAACAGCCTGAGAGCTGAGGACACGGCTGTGTATTACT GTGCGAGAGGAGGCCCAACTCCAGACTGGGGCCAGGGCACCCTGGTCACCGTCTCATCAGCTTCCACCAA GGGCCCCAGCGTGTTCCCCCTGGCCCCTTGTTCCAGGAGCACCTCCGAGAGCACCGCCGCTCTGGGCTGCC TGGTGAAGGACTACTTCCCTGAGCCTGTGACCGTGAGCTGGAACTCCGGCGCTCTGACCTCCGGCGTGCAC ACCTTCCCCGCCGTGCTGCAGAGCAGCGGCCTGTACAGCCTGAGCTCCGTGGTGACAGTGCCCAGCTCCAG CCTGGGCACCAAGACCTACACCTGCAATGTGGATCACAAGCCCAGCAATACCAAGGTGGACAAGAGGGTG GAGTCCAAGTACGGCCCTCCCTGCCCCCCCTGTCCTGCTCCTGAGTTCCTGGGCGGCCCTTCCGTGTTCCTG TTCCCCCCCAAGCCCAAGGATACCCTGATGATCAGCCGGACCCCTGAGGTGACATGTGTGGTGGTGGATGTG AGCCAGGAGGACCCTGAGGTGCAGTTCAACTGGTACGTGGATGGCGTGGAGGTGCACAACGCCAAGACCA AGCCTCGGGAGGAGCAGTTCAATAGCACCTACCGGGTGGTGAGCGTGCTGACCGTGCTGCACCAGGATTGG CTGAACGGCAAGGAGTACAAGTGCAAGGTGAGCAACAAGGGCCTGCCCAGCAGCATCGAGAAGACCATCT CCAAGGCTAAGGGCCAGCCACGGGAGCCTCAGGTGTACACCCTGCCCCCTAGCCAGGAGGAGATGACCAA GAACCAGGTGAGCCTGACCTGTCTGGTGAAGGGCTTCTACCCCTCCGATATCGCTGTGGAGTGGGAGTCCA ACGGCCAGCCCGAGAACAACTATAAGACCACCCCCCCTGTGCTGGACAGCGACGGCAGCTTCTTCCTGTAT TCCAGGCTGACCGTGGACAAGAGCCGGTGGCAGGAGGGCAATGTGTTCTCCTGCAGCGTGATGCACGAGG | 403 |
| | CCCTGCACAACCACTACACCCAGAAGTCCCTGTCCCTGTCCCTCGGCAAG | |
| Heavy chain-A13 (IgG4SP) | CAGGTGCAGCTGTTGGAGTCTGGGGGAGGCCTGGTCAAGCCTGGGGGGTCCCTGAGACTCTCCTGTGCAGC CTCTGGATTCACCTTCAGTAGCTATAGCATGAACTGGGTCCGCCAGGCTCCAGGGAAGGGGCTGGAGTGGG TCTCATCCATTAGTAGTGGTAGTAGTTACATATACTACGCAGACTCAGTGAAGGGCCGATTCACCATCTCCAG AGACAACGCCAAGAACTCACTGTATCTGCAAATGAACAGCCTGAGAGCCGAGGACACGGCTGTGTATTACT GTGCGAGAGATCAAGGGGCGTCCGTAACTGGAACCCACACCCACTGGGGCCAGGGAACCCTGGTCACCGT CTCATCAGCTTCCACCAAGGGCCCCAGCGTGTTCCCCCTGGCCCCTTGTTCCAGGAGCACCTCCGAGAGCA CCGCCGCTCTGGGCTGCCTGGTGAAGGACTACTTCCCTGAGCCTGTGACCGTGAGCTGGAACTCCGGCGCT CTGACCTCCGGCGTGCACACCTTCCCCGCCGTGCTGCAGAGCAGCGGCCTGTACAGCCTGAGCTCCGTGGT GACAGTGCCCAGCTCCAGCCTGGGCACCAAGACCTACACCTGCAATGTGGATCACAAGCCCAGCAATACCA AGGTGGACAAGAGGGTGGAGTCCAAGTACGGCCCTCCCTGCCCCCCCTGTCCTGCTCCTGAGTTCCTGGGC GGCCCTTCCGTGTTCCTGTTCCCCCCCAAGCCCAAGGATACCCTGATGATCAGCCGGACCCCTGAGGTGACA TGTGTGGTGGTGGATGTGAGCCAGGAGGACCCTGAGGTGCAGTTCAACTGGTACGTGGATGGCGTGGAGGT GCACAACGCCAAGACCAAGCCTCGGGAGGAGCAGTTCAATAGCACCTACCGGGTGGTGAGCGTGCTGACC GTGCTGCACCAGGATTGGCTGAACGGCAAGGAGTACAAGTGCAAGGTGAGCAACAAGGGCCTGCCCAGCA GCATCGAGAAGACCATCTCCAAGGCTAAGGGCCAGCCACGGGAGCCTCAGGTGTACACCCTGCCCCCTAGC CAGGAGGAGATGACCAAGAACCAGGTGAGCCTGACCTGTCTGGTGAAGGGCTTCTACCCCTCCGATATCGC TGTGGAGTGGGAGTCCAACGGCCAGCCCGAGAACAACTATAAGACCACCCCCCCTGTGCTGGACAGCGAC GGCAGCTTCTTCCTGTATTCCAGGCTGACCGTGGACAAGAGCCGGTGGCAGGAGGGCAATGTGTTCTCCTG CAGCGTGATGCACGAGGCCCTGCACAACCACTACACCCAGAAGTCCCTGTCCCTGTCCCTCGGCAAG | 404 |
| Heavy chain-A61 (IgG4SP) | CAGGTGCAGCTGGTGCAATCTGGGGCTGAGGTGAAGAAGCCTGGGTCCTCGGTGAAGGTCTCCTGCAAGG CTTCTGGAGGCACCTTCAGCAGCTATACTATCAGCTGGGTGCGACAGGCCCCTGGACAAGGGCTTGAGTGG ATGGGAAGGATCATCCCTATCCTTGGTATAGCAAACTACGCACAGAAGTTCCAGGGCAGAGTCACGATTACC GCGGACAAATCCACGAGCACAGCCTACATGGAGCTGAGCAGCCTGAGATCTGAGGACACGGCCGTGTATTA CTGTGCGACGGGGGTTGGTATGGTTCGGGGAGACCCTTGGGGCCAGGGAACCCTGGTCACCGTCTCATCAG CTTCCACCAAGGGCCCCAGCGTGTTCCCCCTGGCCCCTTGTTCCAGGAGCACCTCCGAGAGCACCGCCGCT CTGGGCTGCCTGGTGAAGGACTACTTCCCTGAGCCTGTGACCGTGAGCTGGAACTCCGGCGCTCTGACCTC CGGCGTGCACACCTTCCCCGCCGTGCTGCAGAGCAGCGGCCTGTACAGCCTGAGCTCCGTGGTGACAGTGC CCAGCTCCAGCCTGGGCACCAAGACCTACACCTGCAATGTGGATCACAAGCCCAGCAATACCAAGGTGGAC AAGAGGGTGGAGTCCAAGTACGGCCCTCCCTGCCCCCCCTGTCCTGCTCCTGAGTTCCTGGGCGGCCCTTC CGTGTTCCTGTTCCCCCCCAAGCCCAAGGATACCCTGATGATCAGCCGGACCCCTGAGGTGACATGTGTGGT GGTGGATGTGAGCCAGGAGGACCCTGAGGTGCAGTTCAACTGGTACGTGGATGGCGTGGAGGTGCACAAC GCCAAGACCAAGCCTCGGGAGGAGCAGTTCAATAGCACCTACCGGGTGGTGAGCGTGCTGACCGTGCTGC ACCAGGATTGGCTGAACGGCAAGGAGTACAAGTGCAAGGTGAGCAACAAGGGCCTGCCCAGCAGCATCGA GAAGACCATCTCCAAGGCTAAGGGCCAGCCACGGGAGCCTCAGGTGTACACCCTGCCCCCTAGCCAGGAG GAGATGACCAAGAACCAGGTGAGCCTGACCTGTCTGGTGAAGGGCTTCTACCCCTCCGATATCGCTGTGGA GTGGGAGTCCAACGGCCAGCCCGAGAACAACTATAAGACCACCCCCCCTGTGCTGGACAGCGACGGCAGC TTCTTCCTGTATTCCAGGCTGACCGTGGACAAGAGCCGGTGGCAGGAGGGCAATGTGTTCTCCTGCAGCGT GATGCACGAGGCCCTGCACAACCACTACACCCAGAAGTCCCTGTCCCTGTCCCTCGGCAAG | 405 |
| Heavy chain-A156 (IgG4SP) | CGGCTGCAGCTGCAGGAGTCGGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACTGCCGCGGACACGGCCGTGTATT ACTGTGCGAGGATTCACCATAGTACCGCGGGGCCATACAACAACTGGTACTTTGACTACTGGGGCCAGGGA ACCCTGGTCACCGTCTCATCAGCTTCCACCAAGGGCCCCAGCGTGTTCCCCCTGGCCCCTTGTTCCAGGAGC ACCTCCGAGAGCACCGCCGCTCTGGGCTGCCTGGTGAAGGACTACTTCCCTGAGCCTGTGACCGTGAGCTG GAACTCCGGCGCTCTGACCTCCGGCGTGCACACCTTCCCCGCCGTGCTGCAGAGCAGCGGCCTGTACAGCC TGAGCTCCGTGGTGACAGTGCCCAGCTCCAGCCTGGGCACCAAGACCTACACCTGCAATGTGGATCACAAG CCCAGCAATACCAAGGTGGACAAGAGGGTGGAGTCCAAGTACGGCCCTCCCTGCCCCCCCTGTCCTGCTCC TGAGTTCCTGGGCGGCCCTTCCGTGTTCCTGTTCCCCCCCAAGCCCAAGGATACCCTGATGATCAGCCGGAC CCCTGAGGTGACATGTGTGGTGGTGGATGTGAGCCAGGAGGACCCTGAGGTGCAGTTCAACTGGTACGTGG ATGGCGTGGAGGTGCACAACGCCAAGACCAAGCCTCGGGAGGAGCAGTTCAATAGCACCTACCGGGTGGT GAGCGTGCTGACCGTGCTGCACCAGGATTGGCTGAACGGCAAGGAGTACAAGTGCAAGGTGAGCAACAAG GGCCTGCCCAGCAGCATCGAGAAGACCATCTCCAAGGCTAAGGGCCAGCCACGGGAGCCTCAGGTGTACA CCCTGCCCCCTAGCCAGGAGGAGATGACCAAGAACCAGGTGAGCCTGACCTGTCTGGTGAAGGGCTTCTAC CCCTCCGATATCGCTGTGGAGTGGGAGTCCAACGGCCAGCCCGAGAACAACTATAAGACCACCCCCCCTGT GCTGGACAGCGACGGCAGCTTCTTCCTGTATTCCAGGCTGACCGTGGACAAGAGCCGGTGGCAGGAGGGC AATGTGTTCTCCTGCAGCGTGATGCACGAGGCCCTGCACAACCACTACACCCAGAAGTCCCTGTCCCTGTCC CTCGGCAAG | 406 |
| Heavy chain-A187 (IgG4SP) | CAGGTGCAGCTGTTGGACTCTGGGGGAGGCTTGGTCAAGCCTGGAGGGTCCCTGAGACTCTCCTGTGCAGC CTCTGGATTCACCTTCAGTAGTTATGGCATGCACTGGGTCCGCCAGGCTCCAGGCAAGGGGCTGGAGTGGGT GGCATTTATATCAAATGATGGAAGTAATAAATACTATGCAGACTCCGTGAAGGGCCGATTCACCATCTCCAGA GACAATTCCAAGAACACGTTGTATCTGCAAATGAACAGCCTGAGAGCTGAGGACACGGCTGTGTATTACTGT GCGAGACTTCTCCGTGGACCCTGGAACTTCGATCTCTGGGGCCGTGGCACCCTGGTCACCGTCTCATCAGCT TCCACCAAGGGCCCCAGCGTGTTCCCCCTGGCCCCTTGTTCCAGGAGCACCTCCGAGAGCACCGCCGCTCT GGGCTGCCTGGTGAAGGACTACTTCCCTGAGCCTGTGACCGTGAGCTGGAACTCCGGCGCTCTGACCTCCG GCGTGCACACCTTCCCCGCCGTGCTGCAGAGCAGCGGCCTGTACAGCCTGAGCTCCGTGGTGACAGTGCCC AGCTCCAGCCTGGGCACCAAGACCTACACCTGCAATGTGGATCACAAGCCCAGCAATACCAAGGTGGACAA GAGGGTGGAGTCCAAGTACGGCCCTCCCTGCCCCCCCTGTCCTGCTCCTGAGTTCCTGGGCGGCCCTTCCGT GTTCCTGTTCCCCCCCAAGCCCAAGGATACCCTGATGATCAGCCGGACCCCTGAGGTGACATGTGTGGTGGT GGATGTGAGCCAGGAGGACCCTGAGGTGCAGTTCAACTGGTACGTGGATGGCGTGGAGGTGCACAACGCC AAGACCAAGCCTCGGGAGGAGCAGTTCAATAGCACCTACCGGGTGGTGAGCGTGCTGACCGTGCTGCACC AGGATTGGCTGAACGGCAAGGAGTACAAGTGCAAGGTGAGCAACAAGGGCCTGCCCAGCAGCATCGAGAA GACCATCTCCAAGGCTAAGGGCCAGCCACGGGAGCCTCAGGTGTACACCCTGCCCCCTAGCCAGGAGGAG ATGACCAAGAACCAGGTGAGCCTGACCTGTCTGGTGAAGGGCTTCTACCCCTCCGATATCGCTGTGGAGTG | 407 |
| | GGAGTCCAACGGCCAGCCCGAGAACAACTATAAGACCACCCCCCCTGTGCTGGACAGCGACGGCAGCTTCT TCCTGTATTCCAGGCTGACCGTGGACAAGAGCCGGTGGCAGGAGGGCAATGTGTTCTCCTGCAGCGTGATG CACGAGGCCCTGCACAACCACTACACCCAGAAGTCCCTGTCCCTGTCCCTCGGCAAG | |
| Heavy chain-B88 (IgG4SP) | CAGGTGCAGCTGGTGCAATCTGGGGCTGAGGTGAAGAAGCCTGGGTCCTCGGTGAAGGTCTCCTGCAAGG CTTCTGGAGGCACCTTCAGCAGCTATACTATCAGCTGGGTGCGACAGGCCCCTGGACAAGGGCTTGAGTGG ATGGGAAGGATCATCCCTATCCTTGGTATAGCAAACTACGCACAGAAGTTCCAGGGCAGAGTCACGATTACC GCGGACAAATCCACGAGCACAGCCTACATGGAGCTGAGCAGCCTGAGATCTGAGGACACGGCCGTGTATTA CTGTGCGACGGGGGTTGGTATGGTTCGGGGAGACCCTTGGGGCCAGGGAACCCTGGTCACCGTCTCATCAG CTTCCACCAAGGGCCCCAGCGTGTTCCCCCTGGCCCCTTGTTCCAGGAGCACCTCCGAGAGCACCGCCGCT CTGGGCTGCCTGGTGAAGGACTACTTCCCTGAGCCTGTGACCGTGAGCTGGAACTCCGGCGCTCTGACCTC CGGCGTGCACACCTTCCCCGCCGTGCTGCAGAGCAGCGGCCTGTACAGCCTGAGCTCCGTGGTGACAGTGC CCAGCTCCAGCCTGGGCACCAAGACCTACACCTGCAATGTGGATCACAAGCCCAGCAATACCAAGGTGGAC AAGAGGGTGGAGTCCAAGTACGGCCCTCCCTGCCCCCCCTGTCCTGCTCCTGAGTTCCTGGGCGGCCCTTC CGTGTTCCTGTTCCCCCCCAAGCCCAAGGATACCCTGATGATCAGCCGGACCCCTGAGGTGACATGTGTGGT GGTGGATGTGAGCCAGGAGGACCCTGAGGTGCAGTTCAACTGGTACGTGGATGGCGTGGAGGTGCACAAC GCCAAGACCAAGCCTCGGGAGGAGCAGTTCAATAGCACCTACCGGGTGGTGAGCGTGCTGACCGTGCTGC ACCAGGATTGGCTGAACGGCAAGGAGTACAAGTGCAAGGTGAGCAACAAGGGCCTGCCCAGCAGCATCGA GAAGACCATCTCCAAGGCTAAGGGCCAGCCACGGGAGCCTCAGGTGTACACCCTGCCCCCTAGCCAGGAG GAGATGACCAAGAACCAGGTGAGCCTGACCTGTCTGGTGAAGGGCTTCTACCCCTCCGATATCGCTGTGGA GTGGGAGTCCAACGGCCAGCCCGAGAACAACTATAAGACCACCCCCCCTGTGCTGGACAGCGACGGCAGC TTCTTCCTGTATTCCAGGCTGACCGTGGACAAGAGCCGGTGGCAGGAGGGCAATGTGTTCTCCTGCAGCGT GATGCACGAGGCCCTGCACAACCACTACACCCAGAAGTCCCTGTCCCTGTCCCTCGGCAAG | 408 |
| Heavy chain-B90 (IgG4SP) | GAGGTGCAGCTGTTGGAGTCTGGGGGAGGCCTGGTCAAGCCTGGGGGGTCCCTGAGACTCTCCTGTGCAG CCTCTGGACTCATCTTCAAAAACTATGGCATGAACTGGGTCCGCCTGGCTCCGGGGAAGGGGCTGGAGTGG GTTGCATCCATTAGTGGTAATAGTAACTACATCTCGTATGCAGACTCAGTGAAGGGCCGATTCACCATCTCCA GAGACAACGCCAACAACTCACTGTTTCTGCAGATGAGCAGCCTGAGAGCCGACGACACGGCTGTCTATTAT TGTTCGAGTGGTTATTTCTACTTTGACCTCTGGGGCCCGGGAACCCTGGTCACCGTCTCATCAGCTTCCACCA AGGGCCCCAGCGTGTTCCCCCTGGCCCCTTGTTCCAGGAGCACCTCCGAGAGCACCGCCGCTCTGGGCTGC CTGGTGAAGGACTACTTCCCTGAGCCTGTGACCGTGAGCTGGAACTCCGGCGCTCTGACCTCCGGCGTGCA CACCTTCCCCGCCGTGCTGCAGAGCAGCGGCCTGTACAGCCTGAGCTCCGTGGTGACAGTGCCCAGCTCCA GCCTGGGCACCAAGACCTACACCTGCAATGTGGATCACAAGCCCAGCAATACCAAGGTGGACAAGAGGGT GGAGTCCAAGTACGGCCCTCCCTGCCCCCCCTGTCCTGCTCCTGAGTTCCTGGGCGGCCCTTCCGTGTTCCT GTTCCCCCCCAAGCCCAAGGATACCCTGATGATCAGCCGGACCCCTGAGGTGACATGTGTGGTGGTGGATGT GAGCCAGGAGGACCCTGAGGTGCAGTTCAACTGGTACGTGGATGGCGTGGAGGTGCACAACGCCAAGACC AAGCCTCGGGAGGAGCAGTTCAATAGCACCTACCGGGTGGTGAGCGTGCTGACCGTGCTGCACCAGGATTG GCTGAACGGCAAGGAGTACAAGTGCAAGGTGAGCAACAAGGGCCTGCCCAGCAGCATCGAGAAGACCATC TCCAAGGCTAAGGGCCAGCCACGGGAGCCTCAGGTGTACACCCTGCCCCCTAGCCAGGAGGAGATGACCA AGAACCAGGTGAGCCTGACCTGTCTGGTGAAGGGCTTCTACCCCTCCGATATCGCTGTGGAGTGGGAGTCC AACGGCCAGCCCGAGAACAACTATAAGACCACCCCCCCTGTGCTGGACAGCGACGGCAGCTTCTTCCTGTA TTCCAGGCTGACCGTGGACAAGAGCCGGTGGCAGGAGGGCAATGTGTTCTCCTGCAGCGTGATGCACGAG GCCCTGCACAACCACTACACCCAGAAGTCCCTGTCCCTGTCCCTCGGCAAG | 409 |
| Heavy chain-B156 (IgG4SP) | CAGGTACAGCTGCAGCAGTCGGGCCCAGGACTGGTGAAGCCTTCACAGACCCTGTCCCTCACCTGCACTGT CTCTGGTGGCTCCATCAGCAGTGGTGGTTACTACTGGAGCTGGATCCGCCAGCACCCAGGGAAGGGCCTGG AGTGGATTGGGTACATCTATTACAGTGGGAGCACCTACTACAACCCGTCCCTCAAGAGTCGAGTTACCATATC AGTAGACACGTCTAAGAACCAGTTCTCCCTGAAGCTGAGCTCTGTGACCGCCGCAGACACGGCTGTGTATT ACTGTGCGAGACAGAGGCGTGCCAACGGGTATAGCAGCAGCTGGGAATTTGACTACTGGGGCCAGGGAAC CCTGGTCACCGTCTCATCAGCTTCCACCAAGGGCCCCAGCGTGTTCCCCCTGGCCCCTTGTTCCAGGAGCAC CTCCGAGAGCACCGCCGCTCTGGGCTGCCTGGTGAAGGACTACTTCCCTGAGCCTGTGACCGTGAGCTGGA ACTCCGGCGCTCTGACCTCCGGCGTGCACACCTTCCCCGCCGTGCTGCAGAGCAGCGGCCTGTACAGCCTG AGCTCCGTGGTGACAGTGCCCAGCTCCAGCCTGGGCACCAAGACCTACACCTGCAATGTGGATCACAAGCC CAGCAATACCAAGGTGGACAAGAGGGTGGAGTCCAAGTACGGCCCTCCCTGCCCCCCCTGTCCTGCTCCTG AGTTCCTGGGCGGCCCTTCCGTGTTCCTGTTCCCCCCCAAGCCCAAGGATACCCTGATGATCAGCCGGACCC CTGAGGTGACATGTGTGGTGGTGGATGTGAGCCAGGAGGACCCTGAGGTGCAGTTCAACTGGTACGTGGAT GGCGTGGAGGTGCACAACGCCAAGACCAAGCCTCGGGAGGAGCAGTTCAATAGCACCTACCGGGTGGTGA GCGTGCTGACCGTGCTGCACCAGGATTGGCTGAACGGCAAGGAGTACAAGTGCAAGGTGAGCAACAAGGG CCTGCCCAGCAGCATCGAGAAGACCATCTCCAAGGCTAAGGGCCAGCCACGGGAGCCTCAGGTGTACACCC TGCCCCCTAGCCAGGAGGAGATGACCAAGAACCAGGTGAGCCTGACCTGTCTGGTGAAGGGCTTCTACCCC TCCGATATCGCTGTGGAGTGGGAGTCCAACGGCCAGCCCGAGAACAACTATAAGACCACCCCCCCTGTGCT GGACAGCGACGGCAGCTTCTTCCTGTATTCCAGGCTGACCGTGGACAAGAGCCGGTGGCAGGAGGGCAAT GTGTTCTCCTGCAGCGTGATGCACGAGGCCCTGCACAACCACTACACCCAGAAGTCCCTGTCCCTGTCCCTC GGCAAG | 410 |
| Light chain-A2 | GACATTGTGATGACCCAGTCTCCATCCTCCCTGTCTGCATCTGTAGGAGACAGAGTCACCATCACTTGCCGG GCAAGTCAGGGCATTAGCAGTGCTTTAGCCTGGTATCAGCAGAAACCAGGGAAAGCTCCTAAGCTCCTGAT CTATGATGCCTCCAGTTTGGAAAGTGGGGTCCCATCAAGGTTCAGCGGCAGTGGATCTGGGACAGATTTCAC TCTCACCATCAGCAGCCTGCAGCCTGAAGATTTTGCAACTTATTACTGTCAACAGTTTAATAGTTACCCGCTC ACTTTCGGCGGAGGGACCAAGGTGGAGATCAAAAGGACCGTGGCTGCCCCCAGCGTGTTCATCTTCCCTCC TAGCGACGAGCAGCTGAAGAGCGGCACCGCTAGCGTGGTGTGTCTGCTGAATAACTTCTATCCCAGGGAGG CCAAGGTGCAGTGGAAGGTGGATAACGCCCTGCAGAGCGGCAACTCCCAGGAGTCCGTGACCGAGCAGGA CTCCAAGGACAGCACCTACTCCCTGAGCTCCACCCTGACCCTGTCCAAGGCTGATTATGAGAAGCACAAGG TGTATGCTTGCGAGGTGACACACCAGGGCCTGTCCAGCCCTGTGACCAAGAGCTTCAACCGGGGCGAGTGC | 411 |
| Light chain-A5 | GACATCCAGATGACCCAGTCTCCATCCTCCCTGTCTGCATCTGTAGGAGACAGAGTCACCATCACTTGCCGG GCGAGTCAGGGCATTCGCAATTACTTAGCCTGGTATCAGCAGAAACCAGGGAAAGTTCCTAGGCTCCTGATC TTTGCTGCATCCACTTTACAATCCGGGGTCCCATCTCGTTTCAGTGGCAGTGGATCTGGGACAGATTTCACTC TCACCATCAGCAGCCTGCAGCCTGAAGATGTTGCAACTTATTACTGTCAAAAGTATTACAGTGCCCCCTTCAC TTTCGGCCCTGGGACCAAAGTAGATATCAAAAGGACCGTGGCTGCCCCCAGCGTGTTCATCTTCCCTCCTAG | 412 |
| | CGACGAGCAGCTGAAGAGCGGCACCGCTAGCGTGGTGTGTCTGCTGAATAACTTCTATCCCAGGGAGGCCA AGGTGCAGTGGAAGGTGGATAACGCCCTGCAGAGCGGCAACTCCCAGGAGTCCGTGACCGAGCAGGACTC CAAGGACAGCACCTACTCCCTGAGCTCCACCCTGACCCTGTCCAAGGCTGATTATGAGAAGCACAAGGTGT ATGCTTGCGAGGTGACACACCAGGGCCTGTCCAGCCCTGTGACCAAGAGCTTCAACCGGGGCGAGTGC | |
| Light chain-A8 | GATATTGTGATGACTCAGTCTCCACTCTCCCTGCCCGTCACCCTTGGACAGCCGGCCTCCATCTCCTGCAGGT CTAGTCAAAGCCTCGTACACAGTGATGGAAACACCTACTTGAATTGGTTTCAGCAGAGGCCAGGCCAATCTC CAAGGCGCCTAATTTATAAGGTTTCTAACCGGGACTCTGGGGTCCCAGACAGATTCAGCGGCAGTGGGTCAG GCACTGATTTCACACTGAAAATCAGCAGGGTGGAGGCTGAAGATGTTGGGGTTTATTACTGCATGCAAGGTA CACAGTGGCCGGACACTTTTGGCCAGGGGACCAAGCTGGAGATCAAAAGGACCGTGGCTGCCCCCAGCGT GTTCATCTTCCCTCCTAGCGACGAGCAGCTGAAGAGCGGCACCGCTAGCGTGGTGTGTCTGCTGAATAACTT CTATCCCAGGGAGGCCAAGGTGCAGTGGAAGGTGGATAACGCCCTGCAGAGCGGCAACTCCCAGGAGTCC GTGACCGAGCAGGACTCCAAGGACAGCACCTACTCCCTGAGCTCCACCCTGACCCTGTCCAAGGCTGATTA TGAGAAGCACAAGGTGTATGCTTGCGAGGTGACACACCAGGGCCTGTCCAGCCCTGTGACCAAGAGCTTCA ACCGGGGCGAGTGC | 413 |
| Light chain-A13 | GAAATTGTAATGACACAGTCTCCAGCCACCCTGTCTTTGTCTCCAGGGGAAAGAGCCACCCTCTCCTGCAG GGCCAGTCAGAGTGTTAGCAGCTACTTAGCCTGGTACCAACAGAAACCTGGCCAGGCTCCCAGGCTCCTCA TCTATGATGCATCCAACAGGGCCACTGGCATCCCAGCCAGGTTCAGTGGCAGTGGGTCTGGGACAGACTTC ACTCTCACCATCAGCAGCCTAGAGCCTGAAGATTTTGCAGTTTATTACTGTCAGCAGCGTAGCAACTGGCCT CCGATGTACACTTTTGGCCAGGGGACCAAGCTGGAGATCAAAAGGACCGTGGCTGCCCCCAGCGTGTTCAT CTTCCCTCCTAGCGACGAGCAGCTGAAGAGCGGCACCGCTAGCGTGGTGTGTCTGCTGAATAACTTCTATCC CAGGGAGGCCAAGGTGCAGTGGAAGGTGGATAACGCCCTGCAGAGCGGCAACTCCCAGGAGTCCGTGACC GAGCAGGACTCCAAGGACAGCACCTACTCCCTGAGCTCCACCCTGACCCTGTCCAAGGCTGATTATGAGAA GCACAAGGTGTATGCTTGCGAGGTGACACACCAGGGCCTGTCCAGCCCTGTGACCAAGAGCTTCAACCGGG GCGAGTGC | 414 |
| Light chain-A21 | GACATCCAGTTGACCCAGTCTCCATCTTCTGTGTCTGCATCTGTGGGGGACAGAGTCACCATCACTTGTCGG GCGAGTCAGAATATTGAGAATTGGTTGGCCTGGTATCAACAGAAGCCAGGGAAAGCCCCTAAACTCCTGAT CTATGCAACATCCCGTTTGGAGAATGATGTCCCATCAAGGTTCAGCGGCAGTCGGTCTGGGACAGAGTTCAT TCTCACTATCAGCAGCCTACAGCCTGAGGATTTTGCAACTTATTATTGCCATCAGGCTAATGGTTTCCCGTGG ACGTTCGGCCCAGGGACCAAGGTGGATATCAAAAGGACCGTGGCTGCCCCCAGCGTGTTCATCTTCCCTCCT AGCGACGAGCAGCTGAAGAGCGGCACCGCTAGCGTGGTGTGTCTGCTGAATAACTTCTATCCCAGGGAGGC CAAGGTGCAGTGGAAGGTGGATAACGCCCTGCAGAGCGGCAACTCCCAGGAGTCCGTGACCGAGCAGGAC TCCAAGGACAGCACCTACTCCCTGAGCTCCACCCTGACCCTGTCCAAGGCTGATTATGAGAAGCACAAGGT GTATGCTTGCGAGGTGACACACCAGGGCCTGTCCAGCCCTGTGACCAAGAGCTTCAACCGGGGCGAGTGC | 415 |
| Light chain-A22 | GATGTTGTGATGACTCAGTCTCCACTCTCCCTGCCCGTCACCCTTGGACAGCCGGCCTCCATCTCCTGCAGG TCTAGTCAAAGCCTCGTACACAGTGATGGAAACACCTACTTGAATTGGTTTCAGCAGAGGCCAGGCCAATCT CCAAGGCGCCTAATTTATAAGGTTTCTAACCGGGACTCTGGGGTCCCAGACAGATTCAGCGGCAGTGGGTCA GGCACTGATTTCACACTGAAAATCAGCAGGGTGGAGGCTGAGGATGTTGGGGTTTATTACTGCATGCAAGGT ACACACTGGCCTCCGAGGTACACTTTTGGCCAGGGGACCAAGCTGGAGATCAAAAGGACCGTGGCTGCCC CCAGCGTGTTCATCTTCCCTCCTAGCGACGAGCAGCTGAAGAGCGGCACCGCTAGCGTGGTGTGTCTGCTG AATAACTTCTATCCCAGGGAGGCCAAGGTGCAGTGGAAGGTGGATAACGCCCTGCAGAGCGGCAACTCCCA GGAGTCCGTGACCGAGCAGGACTCCAAGGACAGCACCTACTCCCTGAGCTCCACCCTGACCCTGTCCAAGG CTGATTATGAGAAGCACAAGGTGTATGCTTGCGAGGTGACACACCAGGGCCTGTCCAGCCCTGTGACCAAG AGCTTCAACCGGGGCGAGTGC | 416 |
| Light chain-A23 | AACATCCAGTTGACCCAGTCTCCATCCTCACTGTCTGCATCTGTAGGAGACAGAGTCACCATCACTTGTCGG GCGAGTCAGGACATTAGCAATTATTTAGCCTGGTTTCAGCAGAAACCAGGGAAAGCCCCTAAGTCCCTGATC TATGCTGCATCCAGTTTGCAAAGTGGGGTCCCATCAAGGTTCAGCGGCAGTTGGTCTGGGACAGATTTCACT CTCACCATCAGCAGCCTGCAGCCTGAAGATATTGCAACGTATTACTGCCAACAGTATAAGCGTTACCCTTTCA TTTTTGGCCAGGGGACCAAGCTGGAGATCAAAAGGACCGTGGCTGCCCCCAGCGTGTTCATCTTCCCTCCTA GCGACGAGCAGCTGAAGAGCGGCACCGCTAGCGTGGTGTGTCTGCTGAATAACTTCTATCCCAGGGAGGCC AAGGTGCAGTGGAAGGTGGATAACGCCCTGCAGAGCGGCAACTCCCAGGAGTCCGTGACCGAGCAGGACT CCAAGGACAGCACCTACTCCCTGAGCTCCACCCTGACCCTGTCCAAGGCTGATTATGAGAAGCACAAGGTG TATGCTTGCGAGGTGACACACCAGGGCCTGTCCAGCCCTGTGACCAAGAGCTTCAACCGGGGCGAGTGC | 417 |
| Light chain-A26 | GATATTGTGATGACTCAGTCTCCACTCTCCCTGCCCGTCACCCCTGGAGAGCCGGCCTCCATCTCCTGCAGG TCTAGTCAGAGCCTCCTGCATAGTAATGGACGCAACTATTTGGATTGGTACCTGCAGAAGCCAGGGCAGTCT CCACAGCTCCTGATCTATTTGGGTTCTAATCGGGCCTCCGGGGTCTCTGACAGGTTCAGTGCCAGTGGATCA GGCACAGATTTTACACTGCAAATCAGCAGAGTGGAGGCTGAGGACGTTGGGATTTATTACTGCATGCAAGCT CTACAAACTCCGTGGACGTTCGGCCAAGGGACCAAGGTGGAAGTCAAAAGGACCGTGGCTGCCCCCAGCG TGTTCATCTTCCCTCCTAGCGACGAGCAGCTGAAGAGCGGCACCGCTAGCGTGGTGTGTCTGCTGAATAACT TCTATCCCAGGGAGGCCAAGGTGCAGTGGAAGGTGGATAACGCCCTGCAGAGCGGCAACTCCCAGGAGTC CGTGACCGAGCAGGACTCCAAGGACAGCACCTACTCCCTGAGCTCCACCCTGACCCTGTCCAAGGCTGATT ATGAGAAGCACAAGGTGTATGCTTGCGAGGTGACACACCAGGGCCTGTCCAGCCCTGTGACCAAGAGCTTC AACCGGGGCGAGTGC | 418 |
| Light chain-A44 | GCCATCCAGATGACCCAGTCTCCATCCTCCCTGTCTGCATCTGTAGGAGACAGAGTCACCATCACTTGCCGG GCAAGTCAGGCCCTTACCTATACTTTAGCCTGGTATCAGCAGAAACCAGGCAAAGCTCCCAGGCTCCTGATC CATGATGCCTCCAGTTTGGAAAGTGGAGTCCCTTCAAGGTTCAGCGGCAGTGGATCTGGGACAGATTTCACT CTCACCATCACCAGCCTGCAGCCTGAAGATTTTGCAACCTATTACTGTCAACAGTTCAGGAGTTACCCGCTC ACTTTCGGCGGAGGGACCAAGGTGGAGATCAAAAGGACCGTGGCTGCCCCCAGCGTGTTCATCTTCCCTCC TAGCGACGAGCAGCTGAAGAGCGGCACCGCTAGCGTGGTGTGTCTGCTGAATAACTTCTATCCCAGGGAGG CCAAGGTGCAGTGGAAGGTGGATAACGCCCTGCAGAGCGGCAACTCCCAGGAGTCCGTGACCGAGCAGGA CTCCAAGGACAGCACCTACTCCCTGAGCTCCACCCTGACCCTGTCCAAGGCTGATTATGAGAAGCACAAGG TGTATGCTTGCGAGGTGACACACCAGGGCCTGTCCAGCCCTGTGACCAAGAGCTTCAACCGGGGCGAGTGC | 419 |
| Light chain-A53 | GACATCCAGATGACCCAGTCTCCATCGTCCCTGTCTGCATCTGTAGGAGACAGAGTCACCATCACTTGCCGG GCAAGTCAGAGCATTAACAACTATTTAAATTGGTATCAGGAGAAACCAGGGAAAGCCCCTAAGCTCCTGATC TATGCTGCATCCAGTTTGCAAAGTGGGGTCCCATCAAGGTTCAGTGGCAGTGGATCTGGGACAGACTTCACT CTCACCATCAGCAGTCTGCAACCTGAAGATTTTGCAACTTACTACTGTCAACAGAGTTACAGTTACCCATTC ACTTTCGGCCCTGGGACCAAAGTGGATATCAAAAGGACCGTGGCTGCCCCCAGCGTGTTCATCTTCCCTCCT AGCGACGAGCAGCTGAAGAGCGGCACCGCTAGCGTGGTGTGTCTGCTGAATAACTTCTATCCCAGGGAGGC CAAGGTGCAGTGGAAGGTGGATAACGCCCTGCAGAGCGGCAACTCCCAGGAGTCCGTGACCGAGCAGGAC TCCAAGGACAGCACCTACTCCCTGAGCTCCACCCTGACCCTGTCCAAGGCTGATTATGAGAAGCACAAGGT GTATGCTTGCGAGGTGACACACCAGGGCCTGTCCAGCCCTGTGACCAAGAGCTTCAACCGGGGCGAGTGC | 420 |
| Light chain-A61 | GATATTGTGATGACTCAGTCTCCACTCTCCCTGCCCGTCACCCTTGGACAGCCGGCCTCCATCTCCTGCAGGT CTAGTCAAAGCCTCGTAAAGAGTGATGGAAACACCTACTTGAGTTGGTTTCAGCAGAGGCCAGGCCAATCT CCAAGGCGCCTAATTTATAAGGTTTCTAACCGGGACTCTGGGGTCCCAGACAGATTCAGCGGCAGTGGGTCA GGCACTGACTTCACACTGAAAATCAGCAGGGTGGAGGCTGAGGATGTTGGGGTTTATTACTGCATGCAAGG TACACACTGGCCACCCACTTTCGGCCCTGGGACCAAAGTGGATATCAAAAGGACCGTGGCTGCCCCCAGCG TGTTCATCTTCCCTCCTAGCGACGAGCAGCTGAAGAGCGGCACCGCTAGCGTGGTGTGTCTGCTGAATAACT TCTATCCCAGGGAGGCCAAGGTGCAGTGGAAGGTGGATAACGCCCTGCAGAGCGGCAACTCCCAGGAGTC CGTGACCGAGCAGGACTCCAAGGACAGCACCTACTCCCTGAGCTCCACCCTGACCCTGTCCAAGGCTGATT ATGAGAAGCACAAGGTGTATGCTTGCGAGGTGACACACCAGGGCCTGTCCAGCCCTGTGACCAAGAGCTTC AACCGGGGCGAGTGC | 421 |
| Light chain-A67 | AACATCCAGATGACCCAGTCTCCTTCCTCCCTGTCTGCCTCTGTGGGGGACAGAATCACCATCACTTGCCGG GCAAGTCAGAGCGTCAATAGTTATGTGAATTGGTATCAGCAGACACCAGGGCAAGCCCCTAAACTCCTCATT TACGCTGCATCGAGTGTGCACAATGGGGTCCCAGTGAGGTTCAGTGGCAGTAGATCTGGGACAGAGTTCATT CTCACCATCAGCAGTCTGCAACCTGAAGATTTTGTCACTTATTTTTGTCAACAGAGTTTCACTAGTCCGTGGA CATTCGGCCAGGGGACCAAACTGGAAATGAAAAGGACCGTGGCTGCCCCCAGCGTGTTCATCTTCCCTCCT AGCGACGAGCAGCTGAAGAGCGGCACCGCTAGCGTGGTGTGTCTGCTGAATAACTTCTATCCCAGGGAGGC CAAGGTGCAGTGGAAGGTGGATAACGCCCTGCAGAGCGGCAACTCCCAGGAGTCCGTGACCGAGCAGGAC TCCAAGGACAGCACCTACTCCCTGAGCTCCACCCTGACCCTGTCCAAGGCTGATTATGAGAAGCACAAGGT GTATGCTTGCGAGGTGACACACCAGGGCCTGTCCAGCCCTGTGACCAAGAGCTTCAACCGGGGCGAGTGC | 422 |
| Light chain-A143 | GAAATTGTGTTGACACAGTCTCCAGCCACCCTGTCTTTGTCTCCAGGGGAAAGAGCCACCCTCTCCTGCAG GGCCAGCCAGAGTATTGCCCGCCGCATAGCCTGGTACCAACAGCAACCTGGCCAGGCTCCCAGGCTCCTCA TCTATGATGCATCCAACAGGGCCACTGGCATCCCAGCCAGGTTCAGTGGCAGTGGGTCTGGGACAGACTTC ACTCTCACCATCAGCAGCCTAGAGCCTGAAGATTTTGCAATTTATTACTGTCAACAACGTGCCAACTGGCCT CTCACTTTCGGCCCTGGGACCAAAGTGGATATCAAAAGGACCGTGGCTGCCCCCAGCGTGTTCATCTTCCCT CCTAGCGACGAGCAGCTGAAGAGCGGCACCGCTAGCGTGGTGTGTCTGCTGAATAACTTCTATCCCAGGGA GGCCAAGGTGCAGTGGAAGGTGGATAACGCCCTGCAGAGCGGCAACTCCCAGGAGTCCGTGACCGAGCAG GACTCCAAGGACAGCACCTACTCCCTGAGCTCCACCCTGACCCTGTCCAAGGCTGATTATGAGAAGCACAA GGTGTATGCTTGCGAGGTGACACACCAGGGCCTGTCCAGCCCTGTGACCAAGAGCTTCAACCGGGGCGAGT GC | 423 |
| Light chain-A156 | GCCATCCAGTTGACCCAGTCTCCATCCTCGCTGTCTGCATCGGTCGGAGACAGAGTCACCATCACTTGCCGG GCCAGTCAGGGCATTAGCAGTTATTTAGCCTGGTATCAGCAAAAACCAGGGAAAGCCCCTAAGCTCCTAATC TTTGCTGCATCGCTTTTACAAAGGGGGGTCCCATCAAGGTTCAGCGGCAGTGGATTTGGGACAGATTTCACT CTCACCATCAGCGGCCTGCAGCCTGAAGATTTTGCGACTTATTTCTGTCAACAGTTTAATACTTACCCGATCA CTTTCGGCGGGGGGACCAAGGTGGAGATCAAAAGGACCGTGGCTGCCCCCAGCGTGTTCATCTTCCCTCCT AGCGACGAGCAGCTGAAGAGCGGCACCGCTAGCGTGGTGTGTCTGCTGAATAACTTCTATCCCAGGGAGGC CAAGGTGCAGTGGAAGGTGGATAACGCCCTGCAGAGCGGCAACTCCCAGGAGTCCGTGACCGAGCAGGAC TCCAAGGACAGCACCTACTCCCTGAGCTCCACCCTGACCCTGTCCAAGGCTGATTATGAGAAGCACAAGGT GTATGCTTGCGAGGTGACACACCAGGGCCTGTCCAGCCCTGTGACCAAGAGCTTCAACCGGGGCGAGTGC | 424 |
| Light chain-A187 | GAAATTGTGATGACGCAGTCTCCAGGCACCCTGTCTTTGTCTCCAGGGGAAAGAGCCACCCTCTCCTGCAG GGCCAGTCAGAGTGTTAGCAGCAGCTACTTAGCCTGGTACCAGCAGAAACCTGGCCAGGCTCCCAGGCTCC TCATCTATGGTGCATCCAGCAGGGCCACTGGCATCCCAGACAGGTTCAGTGGCAGTGGGTCTGGGACAGAC TTCACTCTCACCATCAGCAGACTGGAGCCTGAAGATTTTGCAGTGTATTACTGTCAGCAGTATGGTAGCTCAC CTCCGATCACCTTCGGCCAAGGGACACGACTGGAGATTAAAAGGACCGTGGCTGCCCCCAGCGTGTTCATC TTCCCTCCTAGCGACGAGCAGCTGAAGAGCGGCACCGCTAGCGTGGTGTGTCTGCTGAATAACTTCTATCCC AGGGAGGCCAAGGTGCAGTGGAAGGTGGATAACGCCCTGCAGAGCGGCAACTCCCAGGAGTCCGTGACCG AGCAGGACTCCAAGGACAGCACCTACTCCCTGAGCTCCACCCTGACCCTGTCCAAGGCTGATTATGAGAAG CACAAGGTGTATGCTTGCGAGGTGACACACCAGGGCCTGTCCAGCCCTGTGACCAAGAGCTTCAACCGGGG CGAGTGC | 425 |
| Light chain-A378 | AACATCCAGATGACCCAGTCTCCATCCTCCCTGACTGCATCTGTAGGAGACAGAATCACCATCACTTGCCGG GCGAGTCAGGGCATTCGAAACTCTTTAGCCTGGTATCAGCAGAAACCAGGGACAGCCCCTAAGCTCCTGGT CTCTGCTGCATCCAAGTTACAAACTGGGGTCCCTTCCAGGTTCAGTGGCAGCGGATCTGGGACGGATTACAC TCTCACCATCAGCAGCCTGCAGACTGAAGATTTTGCAACTTATTACTGTCAACAATATTACGGAACCCCTCCC ACTTTTGGCCAGGGGACCAAGCTGGAGATCAAAAGGACCGTGGCTGCCCCCAGCGTGTTCATCTTCCCTCC TAGCGACGAGCAGCTGAAGAGCGGCACCGCTAGCGTGGTGTGTCTGCTGAATAACTTCTATCCCAGGGAGG CCAAGGTGCAGTGGAAGGTGGATAACGCCCTGCAGAGCGGCAACTCCCAGGAGTCCGTGACCGAGCAGGA CTCCAAGGACAGCACCTACTCCCTGAGCTCCACCCTGACCCTGTCCAAGGCTGATTATGAGAAGCACAAGG TGTATGCTTGCGAGGTGACACACCAGGGCCTGTCCAGCCCTGTGACCAAGAGCTTCAACCGGGGCGAGTGC | 426 |
| Light chain-A382 | GAAATTGTGTTGACACAGTCTCCAGCCACCCTGTCTTTGTCTCCAGGGGAAAGAGCCACCCTCTCCTGCAG GGCCAGTCAGGGTATTAGCAGCTACTTAGCCTGGTACCAACAGAAACCTGGCCAGGCTCCCAGACTCCTCAT TTATGATGCATCCAATAGGGCCACTGGCATCCCAGCCAGGTTCAGTGGCAGTGGGTCTGGGACAGACTTCAC TCTCACCATCAGCAACCTAGAGCCTGAAGATTTTGCAGTTTATTACTGTCAGCAGCGTAAAAACTGGCCTCT CACTTTCGGCGGAGGGTCCAAGGTGGAGATCAAAAGGACCGTGGCTGCCCCCAGCGTGTTCATCTTCCCTC CTAGCGACGAGCAGCTGAAGAGCGGCACCGCTAGCGTGGTGTGTCTGCTGAATAACTTCTATCCCAGGGAG GCCAAGGTGCAGTGGAAGGTGGATAACGCCCTGCAGAGCGGCAACTCCCAGGAGTCCGTGACCGAGCAGG ACTCCAAGGACAGCACCTACTCCCTGAGCTCCACCCTGACCCTGTCCAAGGCTGATTATGAGAAGCACAAG GTGTATGCTTGCGAGGTGACACACCAGGGCCTGTCCAGCCCTGTGACCAAGAGCTTCAACCGGGGCGAGTG C | 427 |
| Light chain-B16 | GAAATTGTGTTGACACAGTCTCCAGCCACCCTGTCTTTGTCTCCAGGGGAAAGAGCCACCCTCTCCTGCAG GGCCAGTCAGAGTGTTAGCAGCTACTTAGCCTGGTACCAACAGAAACCTGGCCAGGCTCCCAGGCTCCTCA | 428 |
| | TCTATGATGCATCCAACAGGGCCACTGGCATCCCAGCCAGGTTCAGTGGCAGTGGGTCTGGGACAGACTTC ACTCTCACCATCAGCAGCCTAGAGCCTGAAGATTTTGCAGTTTATTACTGTCAGCAGCGTAGCAACTGGCCT CCGGAGCTCACTTTCGGCGGAGGGACCAAGGTGGAGATCAAAAGGACCGTGGCTGCCCCCAGCGTGTTCAT CTTCCCTCCTAGCGACGAGCAGCTGAAGAGCGGCACCGCTAGCGTGGTGTGTCTGCTGAATAACTTCTATCC CAGGGAGGCCAAGGTGCAGTGGAAGGTGGATAACGCCCTGCAGAGCGGCAACTCCCAGGAGTCCGTGACC GAGCAGGACTCCAAGGACAGCACCTACTCCCTGAGCTCCACCCTGACCCTGTCCAAGGCTGATTATGAGAA GCACAAGGTGTATGCTTGCGAGGTGACACACCAGGGCCTGTCCAGCCCTGTGACCAAGAGCTTCAACCGGG GCGAGTGC | |
| Light chain-B20 | GAAATTGTGATGACACAGTCTCCAGCCACCCTGTCTTTGTCTCCAGGGGAAAGAGCCACCCTCTCCTGCAG GGCCAGTCAGAGTGTTAGCAACTACTTAGCCTGGTACCAGCAGAAACCTGGCCAGGCTCCCAGGCTCCTCA TCTATGGTGCATCCAGCAGGGCCACTGGCATCCCAGACAGGTTCAGTGGCAGTGGGTCTGGGACAGACTTC ACTCTCACCATCAGCAGACTGGAGCCTGAAGATTTTGCAGTGTATTACTGTCAGCAGTATGGTAGCTCACTTT GGACGTTCGGCCAAGGGACCAAGGTGGAAATCAAAAGGACCGTGGCTGCCCCCAGCGTGTTCATCTTCCCT CCTAGCGACGAGCAGCTGAAGAGCGGCACCGCTAGCGTGGTGTGTCTGCTGAATAACTTCTATCCCAGGGA GGCCAAGGTGCAGTGGAAGGTGGATAACGCCCTGCAGAGCGGCAACTCCCAGGAGTCCGTGACCGAGCAG GACTCCAAGGACAGCACCTACTCCCTGAGCTCCACCCTGACCCTGTCCAAGGCTGATTATGAGAAGCACAA GGTGTATGCTTGCGAGGTGACACACCAGGGCCTGTCCAGCCCTGTGACCAAGAGCTTCAACCGGGGCGAGT GC | 429 |
| Light chain-B24 | GACATCCAGATGACCCAGTCTCCCTCCTCCCTGCCTGCATCTGTGGGAGACAGAGTCACCATCACTTGCCGG GCCAGTCAGGGCATTGGCACTTTTTTAGCCTGGTATCAGCAACACTTGGGGAAAGCCCCTAAACTCCTGATC TCTGCTAGTTCCACTGTTCAGCGTGGGGTCCCATCCAGGTTCAGCGGCAGTGGATCTGGGACAGAGTTCACT CTCACCATCAACAGCCTGCAGCCTGAAGATTTTGCAACTTATTACTGTCAGCAGCTTAATAATTACCCTCGGA CGTTCGGCCAAGGGACCAAAGTGGAAATCAAGAGGACCGTGGCTGCCCCCAGCGTGTTCATCTTCCCTCCT AGCGACGAGCAGCTGAAGAGCGGCACCGCTAGCGTGGTGTGTCTGCTGAATAACTTCTATCCCAGGGAGGC CAAGGTGCAGTGGAAGGTGGATAACGCCCTGCAGAGCGGCAACTCCCAGGAGTCCGTGACCGAGCAGGAC TCCAAGGACAGCACCTACTCCCTGAGCTCCACCCTGACCCTGTCCAAGGCTGATTATGAGAAGCACAAGGT GTATGCTTGCGAGGTGACACACCAGGGCCTGTCCAGCCCTGTGACCAAGAGCTTCAACCGGGGCGAGTGC | 430 |
| Light chain-B29 | GAAATTGTGATGACGCAGTCTCCAGGCACCCTGTCTTTGTCTCCAGGGCAAAGAGCCACCCTCTCCTGCAG GGCCAGTCAGAGTGTTAGCAGCAGCTCCTTAGCCTGGTATCAACAGAAACCTGGCCAGGCTCCCAGGCTCC TCATCCACGGTGCCTCCAGCAGGGCCACTGGTATCCCAGACAGGTTCAGTGGCAGTGGGTCTGGGACAGAC TTCACTCTCACCATCAGCAGACTGGAGCCTGAAGATTTTGCAGTGTATTACTGTCAGCAGTATGGTACCTCGT GGACGTTCGGCCAAGGGACCAAGGTGGAAATCAAAAGGACCGTGGCTGCCCCCAGCGTGTTCATCTTCCCT CCTAGCGACGAGCAGCTGAAGAGCGGCACCGCTAGCGTGGTGTGTCTGCTGAATAACTTCTATCCCAGGGA GGCCAAGGTGCAGTGGAAGGTGGATAACGCCCTGCAGAGCGGCAACTCCCAGGAGTCCGTGACCGAGCAG GACTCCAAGGACAGCACCTACTCCCTGAGCTCCACCCTGACCCTGTCCAAGGCTGATTATGAGAAGCACAA GGTGTATGCTTGCGAGGTGACACACCAGGGCCTGTCCAGCCCTGTGACCAAGAGCTTCAACCGGGGCGAGT GC | 431 |
| Light chain-B44 | GATGTTGTGATGACTCAGTCTCCACTCTCCCTGCCCGTCACCCCTGGAGAGCCGGCCTCCATCTCCTGCAGG TCTAGTCAGAGCCTCCTGCATAGGAGTGGATACAACTATTTGGATTGGTACGTGCAGAAGCCAGGGCAGTCT CCACAGCTCCTGATCTATTTGGGGTCTATTCGAGCGGTCGGGGTCCCCGACAGGTTCAGTGGCAGCGCATCA GGCACAGATTTTACACTGAGAATCAGCAGAGTGGAGGCTGAGGATGTTGGGATTTATTACTGCATGCAAGCT CTACAAACTCCTCCGGCTTTCGGCGGGGGGACCAAGGTGGAGATCAAAAGGACCGTGGCTGCCCCCAGCG TGTTCATCTTCCCTCCTAGCGACGAGCAGCTGAAGAGCGGCACCGCTAGCGTGGTGTGTCTGCTGAATAACT TCTATCCCAGGGAGGCCAAGGTGCAGTGGAAGGTGGATAACGCCCTGCAGAGCGGCAACTCCCAGGAGTC CGTGACCGAGCAGGACTCCAAGGACAGCACCTACTCCCTGAGCTCCACCCTGACCCTGTCCAAGGCTGATT ATGAGAAGCACAAGGTGTATGCTTGCGAGGTGACACACCAGGGCCTGTCCAGCCCTGTGACCAAGAGCTTC AACCGGGGCGAGTGC | 432 |
| Light chain-B53 | AACATCCAGTTGACCCAGTCTCCATCCTCCCTGTCTGCATCTGTAGGAGACAGAGTCACCATCACTTGTCGG GCGAGTCAGGGTATTAACAGCTGGTTAGCCTGGTATCAGCAGAAACCAGGGAAAGCCCCTAAGCTCCTTATC TATGCTGCATCCACTTTGCAAAGTGGGGTCCCATCAAGATTCAGCGGCAGTGGATCTGGGACAGATTTCACT CTCACCATCAACAGCCTGCAGCCTGAGGATTTTGCAACTTATTATTGTCAGCAGCTTAACAGTTTCCCATTCA CTTTTGGCCCTGGGACCAAAGTGCATATCAAAAGGACCGTGGCTGCCCCCAGCGTGTTCATCTTCCCTCCTA GCGACGAGCAGCTGAAGAGCGGCACCGCTAGCGTGGTGTGTCTGCTGAATAACTTCTATCCCAGGGAGGCC AAGGTGCAGTGGAAGGTGGATAACGCCCTGCAGAGCGGCAACTCCCAGGAGTCCGTGACCGAGCAGGACT CCAAGGACAGCACCTACTCCCTGAGCTCCACCCTGACCCTGTCCAAGGCTGATTATGAGAAGCACAAGGTG TATGCTTGCGAGGTGACACACCAGGGCCTGTCCAGCCCTGTGACCAAGAGCTTCAACCGGGGCGAGTGC | 433 |
| Light chain-B67 | GACATCCAGTTGACCCAGTCTCCATCTTCCCTGTCTGCATCTGTAGGAGACAGAGTCACCATCACTTGCCGG GCAAGTCAGAGCATTGACGACCATTTAAACTGGTATCAGCAAAAGCCAGGGAAAGTCCCCAAGCTCCTCAT AAGTAGTGCATCGACATTGCAGAGTGGGGCCCCATCGAGGTTCAGTGGCAGTGGATATGGGACAGATTTCAC CCTCACCATCAGCAGTCTGCAAGTCGAAGATTTTGCAACTTACTACTGTCAACAGAGTTACAGTCCCCCTCA GACGTTCGGCCAGGGGACCAAGGTGGAAATCAAGAGGACCGTGGCTGCCCCCAGCGTGTTCATCTTCCCTC CTAGCGACGAGCAGCTGAAGAGCGGCACCGCTAGCGTGGTGTGTCTGCTGAATAACTTCTATCCCAGGGAG GCCAAGGTGCAGTGGAAGGTGGATAACGCCCTGCAGAGCGGCAACTCCCAGGAGTCCGTGACCGAGCAGG ACTCCAAGGACAGCACCTACTCCCTGAGCTCCACCCTGACCCTGTCCAAGGCTGATTATGAGAAGCACAAG GTGTATGCTTGCGAGGTGACACACCAGGGCCTGTCCAGCCCTGTGACCAAGAGCTTCAACCGGGGCGAGTG C | 434 |
| Light chain-B83 | GACATCCAGTTGACCCAGTCTCCATCCTCCCTGTCTGCATCTGTAGGAGACAGAGTCACCATCACTTGCCGG GCAAGTCAGAGCATTAGCAGCTATTTAAATTGGTATCAGCAGAAACCAGGGAAAGCCCCTAAGCTCCTGATC TATGCTGCATCCAGTTTGCAAAGTGGGGTCCCATCAAGGTTCAGTGGCAGTGGATCTGGGACAGATTTCACT CTCACCATCAGCAGTCTGCAACCTGAAGATTTTGCAACTTACTACTGTCAACAGAGTTACAGTACCCCTCGG ACCTTCGGCCAAGGGACACGACTGGAGATTAAAAGGACCGTGGCTGCCCCCAGCGTGTTCATCTTCCCTCC TAGCGACGAGCAGCTGAAGAGCGGCACCGCTAGCGTGGTGTGTCTGCTGAATAACTTCTATCCCAGGGAGG CCAAGGTGCAGTGGAAGGTGGATAACGCCCTGCAGAGCGGCAACTCCCAGGAGTCCGTGACCGAGCAGGA CTCCAAGGACAGCACCTACTCCCTGAGCTCCACCCTGACCCTGTCCAAGGCTGATTATGAGAAGCACAAGG TGTATGCTTGCGAGGTGACACACCAGGGCCTGTCCAGCCCTGTGACCAAGAGCTTCAACCGGGGCGAGTGC | 435 |
| Light chain-B88 | GATGTTGTGATGACTCAGTCTCCACTCTCCCTGCCCGTCACCCTTGGACAGCCGGCCTCCATCTCCTGCAGG TCTAGTCAAAGCCTCGTATACAGTGATGGAAACACCTACTTGAATTGGTTTCAGCAGAGGCCAGGCCAATCT CCAAGGCGCCTAATTTATAAGGTTTCTAACCGGGACTCTGGGGTCCCAGACAGATTCAGCGGCAGTGGGTCA GGCACTGATTTCACACTGAAAATCAGCAGGGTGGAGGCTGAGGATGTTGGGGTTTATTACTGCATGCAAGGT ACACACTGGCCTCCCACTTTTGGCCAGGGGACCAAGCTGGAGATCAAAAGGACCGTGGCTGCCCCCAGCGT | 436 |
| | GTTCATCTTCCCTCCTAGCGACGAGCAGCTGAAGAGCGGCACCGCTAGCGTGGTGTGTCTGCTGAATAACTT CTATCCCAGGGAGGCCAAGGTGCAGTGGAAGGTGGATAACGCCCTGCAGAGCGGCAACTCCCAGGAGTCC GTGACCGAGCAGGACTCCAAGGACAGCACCTACTCCCTGAGCTCCACCCTGACCCTGTCCAAGGCTGATTA TGAGAAGCACAAGGTGTATGCTTGCGAGGTGACACACCAGGGCCTGTCCAGCCCTGTGACCAAGAGCTTCA ACCGGGGCGAGTGC | |
| Light chain-B90 | GACATCCAGATGACCCAGTCTCCCTCTATCCTGTCTGCATCTGTAGGAGACAGAGTCACCCTCACTTGCCGG GCCAGTCAAAGTATTAGTTCCTGGTTGGCCTGGTATCAACAGACACCAGGAAAAGCCCCTAGGGTCCTGATT TATGAGGCGTCAACTTTAGAGGTTGGGGTCCCATCAAGGTTCAGCGGCAGTGGATCTGGGACAGACTTCAC TCTCACCATCAGCAGCCTGCAGCCTGAAGATTTTGGAACTTACTTCTGCCAACAATACCATACTGATTCGAG GACTTTTGGACAGGGGACCATCCTAGAGATCAAAAGGACCGTGGCTGCCCCCAGCGTGTTCATCTTCCCTCC TAGCGACGAGCAGCTGAAGAGCGGCACCGCTAGCGTGGTGTGTCTGCTGAATAACTTCTATCCCAGGGAGG CCAAGGTGCAGTGGAAGGTGGATAACGCCCTGCAGAGCGGCAACTCCCAGGAGTCCGTGACCGAGCAGGA CTCCAAGGACAGCACCTACTCCCTGAGCTCCACCCTGACCCTGTCCAAGGCTGATTATGAGAAGCACAAGG TGTATGCTTGCGAGGTGACACACCAGGGCCTGTCCAGCCCTGTGACCAAGAGCTTCAACCGGGGCGAGTGC | 437 |
| Light chain-B109 | GACATCGTGATGACCCAGTCTCCATCCTCCCTGTCTGCATCTGTAGGAGACAGAGTCACCATCACTTGCCGG GCAAGTCAGAGCATTAGCAGCTATTTAAATTGGTATCAGCAGAAACCAGGGAAAGCCCCTAAGCTCCTGATC TATGCTGCATCCAGTTTGCAAAGTGGGGTCCCATCAAGGTTCAGTGGCAGTGGATCTGGGACAGATTTCACT CTCACCATCAGCAGTCTGCAACCTGAAGATTTTGCAACTTACTACTGTCAACAGAGTTACAGTACCCTCACT TTCGGCGGAGGGACCAAGGTGGAGATCAAAAGGACCGTGGCTGCCCCCAGCGTGTTCATCTTCCCTCCTAG CGACGAGCAGCTGAAGAGCGGCACCGCTAGCGTGGTGTGTCTGCTGAATAACTTCTATCCCAGGGAGGCCA AGGTGCAGTGGAAGGTGGATAACGCCCTGCAGAGCGGCAACTCCCAGGAGTCCGTGACCGAGCAGGACTC CAAGGACAGCACCTACTCCCTGAGCTCCACCCTGACCCTGTCCAAGGCTGATTATGAGAAGCACAAGGTGT ATGCTTGCGAGGTGACACACCAGGGCCTGTCCAGCCCTGTGACCAAGAGCTTCAACCGGGGCGAGTGC | 438 |
| Light chain-B114 | GAAATTGTATTGACACAGTCTCCAGGCACCCTGTCTTTGTCTCCAGGGGAAAGAGCCACCCTCTCCTGCAGG GCCAGTCAGAGTGTTAGCAAGTACCTAGCCTGGTACCAACAGAAACCTGGCCAGGCTCCCAGGCTCCTTAT CTATGATGTTTCCAACAGGGCCACTGGCATCCCAGCCAGATTCAGTGGCAGTGGGTCTGGGACAGACTTCAC TCTCACCATCAGCAGCCTAGAGCCTGAAGATTTTGCAGTTTATTACTGTCAGCAGCGGCGCAGCTGGCCTCT CACTTTCGGCGGAGGGACCAAGGTGGAGATCAGAAGGACCGTGGCTGCCCCCAGCGTGTTCATCTTCCCTC CTAGCGACGAGCAGCTGAAGAGCGGCACCGCTAGCGTGGTGTGTCTGCTGAATAACTTCTATCCCAGGGAG GCCAAGGTGCAGTGGAAGGTGGATAACGCCCTGCAGAGCGGCAACTCCCAGGAGTCCGTGACCGAGCAGG ACTCCAAGGACAGCACCTACTCCCTGAGCTCCACCCTGACCCTGTCCAAGGCTGATTATGAGAAGCACAAG GTGTATGCTTGCGAGGTGACACACCAGGGCCTGTCCAGCCCTGTGACCAAGAGCTTCAACCGGGGCGAGTG C | 439 |
| Light chain-B117 | AACATCCAGTTGACCCAGTCTCCATCTTCTGTGTCTGCATCTGTAGGAGACAGAGTCACCATCTCTTGTCGG GCGAGTCAGGGTATTGGCAGATGGTTAGCCTGGTATCAGCAGAAACCAGGGAAAGCCCCTAAACTCCTGAT CTATGCTGCATCCAATTTGCAAAGTGGGGTCCCATCAAGGTTCAGTGGCAGTGGATCTGGGACAGATTTCAC TCTCACCATCAGCAGCCTGCAGCCTGAAGATTTCGCAACTTACTATTGTCAACAGGCTAACAGTTTCCCTCG TACTTTCGGCCCTGGGACCAAAGTGGATATCAAAAGGACCGTGGCTGCCCCCAGCGTGTTCATCTTCCCTCC TAGCGACGAGCAGCTGAAGAGCGGCACCGCTAGCGTGGTGTGTCTGCTGAATAACTTCTATCCCAGGGAGG CCAAGGTGCAGTGGAAGGTGGATAACGCCCTGCAGAGCGGCAACTCCCAGGAGTCCGTGACCGAGCAGGA CTCCAAGGACAGCACCTACTCCCTGAGCTCCACCCTGACCCTGTCCAAGGCTGATTATGAGAAGCACAAGG TGTATGCTTGCGAGGTGACACACCAGGGCCTGTCCAGCCCTGTGACCAAGAGCTTCAACCGGGGCGAGTGC | 440 |
| Light chain-B123 | AACATCCAGTTGACCCAGTCTCCATCCTCCCTGTCTGCATCTGTAGGAGACAGAGTCACCATCACTTGTCGG GCCAGTCAGGGCATTAGCACTTATTTAGCCTGGTATCAGCAAAAACCAGGGTTGGCCCCTAAGCTCCTGATC TATGGTGCATCCACTTTGCAAAGTGGGGTCCCATCAAAATTCAGCGGCTTTGGATCTGGGAGAGATTTCACT CTCACCATCAGCAGCCTGCAGCCTGAAGATATTGCAACTTACTACTGTCAACAACTTAATACTTATCCTCCCA CTTTTGGCCAGGGGACCAAGCTGGAGATCAAAAGGACCGTGGCTGCCCCCAGCGTGTTCATCTTCCCTCCT AGCGACGAGCAGCTGAAGAGCGGCACCGCTAGCGTGGTGTGTCTGCTGAATAACTTCTATCCCAGGGAGGC CAAGGTGCAGTGGAAGGTGGATAACGCCCTGCAGAGCGGCAACTCCCAGGAGTCCGTGACCGAGCAGGAC TCCAAGGACAGCACCTACTCCCTGAGCTCCACCCTGACCCTGTCCAAGGCTGATTATGAGAAGCACAAGGT GTATGCTTGCGAGGTGACACACCAGGGCCTGTCCAGCCCTGTGACCAAGAGCTTCAACCGGGGCGAGTGC | 441 |
| Light chain-B145 | GACATTGTGATGACCCAGTCTCCATCCTCCCTGTCTGCATCTGTAGGAGACAGAGTCACCATCACTTGCCAG GCGAGTCAGGACATTAGCAACTATTTAAATTGGTATCAGCAGAAACCAGGGAAAGCCCCTAAGCTCCTGATC TACGATGCATCCAGTTTGGAAAGCGGGGTCCCATCAAAGTTCAGCGGCAGTAGATCTGGGACAGATTTCACT CTCACCATCAGCAGCCTGCAGCCTGAAGATTCTGCAACTTATTACTGCCAACAGTATAGGAGTTACCCGATC ACTTTCGGCGGAGGGACCAAGGTGGAGATCAAAAGGACCGTGGCTGCCCCCAGCGTGTTCATCTTCCCTCC TAGCGACGAGCAGCTGAAGAGCGGCACCGCTAGCGTGGTGTGTCTGCTGAATAACTTCTATCCCAGGGAGG CCAAGGTGCAGTGGAAGGTGGATAACGCCCTGCAGAGCGGCAACTCCCAGGAGTCCGTGACCGAGCAGGA CTCCAAGGACAGCACCTACTCCCTGAGCTCCACCCTGACCCTGTCCAAGGCTGATTATGAGAAGCACAAGG TGTATGCTTGCGAGGTGACACACCAGGGCCTGTCCAGCCCTGTGACCAAGAGCTTCAACCGGGGCGAGTGC | 442 |
| Light chain-B156 | GACATCCAGTTGACCCAGTCTCCATCCTCCCTGTCTGCATCTATAGGAGACAGAGTCACCATCACTTGCCGG GCCAGTCAGGGCATTAGCAGTTTTTTAGCCTGGTATCAGCAAAAACCAGGGAAAGCCCCACAGCTCCTGAT CTATGCTGCGTCCACTTTGCAAAGTGGGGTCCCATCAAGGTTCAGCGGCAGTGGGTCTGGGACAGAATTCA CTCTCACAATCAGCAGCCTGCAGCCTGAAGATTTTGCAACTTATTACTGTCAACAATTTAAAATTTATCCTCT CACTTTCGGCGGAGGGACCAAGGTGGAGGTCAAAAGGACCGTGGCTGCCCCCAGCGTGTTCATCTTCCCTC CTAGCGACGAGCAGCTGAAGAGCGGCACCGCTAGCGTGGTGTGTCTGCTGAATAACTTCTATCCCAGGGAG GCCAAGGTGCAGTGGAAGGTGGATAACGCCCTGCAGAGCGGCAACTCCCAGGAGTCCGTGACCGAGCAGG ACTCCAAGGACAGCACCTACTCCCTGAGCTCCACCCTGACCCTGTCCAAGGCTGATTATGAGAAGCACAAG GTGTATGCTTGCGAGGTGACACACCAGGGCCTGTCCAGCCCTGTGACCAAGAGCTTCAACCGGGGCGAGTG C | 443 |
| Light chain-B201 | GAAATAGTGATGACGCAGTCTCCAGGCACCCTGTCTTTGTCTCCAGGGGAAAGAGCCACCCTCTCCTGCAG GGCCAGTCAGAGTGTTAGCAGCAGCTACTTAGCCTGGTACCAGCAGAAACCTGGCCAGGCTCCCAGGCTCC TCATCTATGGTGCATCCAGCAGGGCCACTGGCATCCCAGACAGGTTCAGTGGCAGTGGGTCTGGGACAGAC TTCACTCTCACCATCAGCAGACTGGAGCCTGAAGATTTTGCAGTGTATTACTGTCAGCAGTATGGTAGCTCAC CTCCGATCACCTTCGGCCAAGGGACACGACTGGAGATTAAAAGGACCGTGGCTGCCCCCAGCGTGTTCATC TTCCCTCCTAGCGACGAGCAGCTGAAGAGCGGCACCGCTAGCGTGGTGTGTCTGCTGAATAACTTCTATCCC AGGGAGGCCAAGGTGCAGTGGAAGGTGGATAACGCCCTGCAGAGCGGCAACTCCCAGGAGTCCGTGACCG AGCAGGACTCCAAGGACAGCACCTACTCCCTGAGCTCCACCCTGACCCTGTCCAAGGCTGATTATGAGAAG CACAAGGTGTATGCTTGCGAGGTGACACACCAGGGCCTGTCCAGCCCTGTGACCAAGAGCTTCAACCGGGG CGAGTGC | 444 |
| Light chain-B307 | GACATCCAGTTGACCCAGTCTCCATCCTTCCTGTCTGCATCTGTTGGAGACAGAGTCACTATCACTTGCCGG GCCAGTCAGGGCATTAGTAGTCAGTTAGCCTGGTATCAACAAGAGCCAGGGAAAGCCCCTAAGCTCCTGAT CTATGCTGCATCCACTTTGCAAAGTGGGGTCCCATCAAGGTTCAGCGGCAGTGGGTCTGGGACAGAGTTTAC TCTCACAATCAGCAGCCTGCAGCCTGAAGATTTTGCAACTTATTACTGTCAACAGGTCAATGCTTACCCTCTC ACTTTCGGCCCTGGGACCAAAGTGGATATCAAAAGGACCGTGGCTGCCCCCAGCGTGTTCATCTTCCCTCCT AGCGACGAGCAGCTGAAGAGCGGCACCGCTAGCGTGGTGTGTCTGCTGAATAACTTCTATCCCAGGGAGGC CAAGGTGCAGTGGAAGGTGGATAACGCCCTGCAGAGCGGCAACTCCCAGGAGTCCGTGACCGAGCAGGAC TCCAAGGACAGCACCTACTCCCTGAGCTCCACCCTGACCCTGTCCAAGGCTGATTATGAGAAGCACAAGGT GTATGCTTGCGAGGTGACACACCAGGGCCTGTCCAGCCCTGTGACCAAGAGCTTCAACCGGGGCGAGTGC | 445 |

### BRIEF DESCRIPTION OF THE DRAWINGS

The above and/or additional aspects and advantages of the present disclosure will become more apparent and more understandable from the following description of embodiments taken in conjunction with the accompanying drawings.
FIG. 1 is a schematic structural diagram of PLA2R of the present disclosure.
FIG. 2 is a schematic structural diagram of an expression framework in Example 1 of the present disclosure.
FIG. 3 shows a CC1H SDS-PAGE analysis in Example 1 of the present disclosure, in which: lane 1 represents a molecular weight reference; lane 2 represents purified CC1H-Non-reducing; and lane 3 represents purified CC1H-Reducing.
FIG. 4 shows results of binding activity of respective antibodies to CC1h in Example 3 of the present disclosure.
FIG. 5 shows results of binding activity of respective antibodies to CC1h in Example 3 of the present disclosure.
FIG. 6 shows a protein standard curve (y = 0.6849x + 0.6325, R² = 0.9925) determined by the Bradford Assay in Example 4 of the present disclosure.
FIG. 7 is a diagram showing purification effect of CC1H-Biotin detected by SDS-PAGE in Example 4 of the present disclosure, in which: lane 1 represents CC1H-Biotin in a reducing loading buffer containing DTT; and lane 2 represents CC1H-Biotin in a non-reducing loading buffer.
FIG. 8 shows a standard curve prepared by diluting antibody A13 (P59368) and antibody A13SP (P62297) to the equivalent concentration of the standard according to 1 RU/mL = 70 ng/mL in Example 5 of the present disclosure.
FIG. 9 shows a standard curve prepared by diluting 9 antibodies (FIG. 9A) and the SP mutant antibodies thereof (FIG. 9B) to the equivalent concentration of the standard according to 1 RU/mL = 70 ng/mL in Example 5 of the present disclosure.

### DETAILED DESCRIPTION

Embodiments of the present disclosure will be described in detail below. The embodiments described below are exemplary and are only used to explain the present disclosure, and they should not be construed as limiting the present disclosure.

It should be noted that the terms "first" and "second" are only used for descriptive purposes, rather than indicating or implying relative importance or implicitly indicating the number of indicated technical features. Therefore, the features defined with "first" and "second" may include one or more of these features explicitly or implicitly. Further, in the description of the present disclosure, unless otherwise specified, "plurality of" means two or more.

In the present disclosure, endpoints and any value of the ranges shall not be limited to the exact range or value, and those ranges or values should be understood to include values close to those ranges or values. For numerical ranges, endpoints of respective ranges, an endpoint and individual point value of respective ranges, and individual point values can be combined with each other to obtain one or more new numerical ranges, which should be deemed to be specifically disclosed herein.

To make the present disclosure more easily understood, certain technical and scientific terms are specifically defined below. Unless otherwise clearly defined, all other technical and scientific terms used herein have the meanings commonly understood by those skilled in the art to which the present disclosure belongs. The abbreviations for amino acid residues are standard 3-letter and/or 1-letter codes used in the art to refer to one of the 20 commonly used L-amino acids.

As used herein, the term "comprise", "include", "including" or "comprising" is an open expression, that is, including the contents indicated by the present disclosure, but not excluding other aspects.

As used herein, the terms "optionally" or "optional" usually mean that the event or situation described subsequently may happen but not necessarily happen, and the description includes the situation in which the event or situation happens and the situation in which the event or situation does not happen.

As used herein, the term "identity", "homology", or "similarity", for describing an amino acid sequence or a nucleic acid sequence relative to a reference sequence, is a percentage of the same amino acids or nucleotides between two amino acid sequences or nucleic acid sequences determined by a conventional method, for example, see Ausubel et al., ed. (1995), Current Protocols in Molecular Biology, Chapter 19 (Greene Publishing and Wiley-Interscience, New York); and ALIGN program (Dayhoff (1978), Atlas of Protein Sequence and Structure 5: Suppl.3 (National Biomedical Research Foundation, Washington, D.C.). Many algorithms are available for aligning sequences and determining sequence identity, including: the homology alignment algorithm (Needleman et al., (1970) J.Mol.Biol.48:443); local homology algorithm (Smith et al., (1981) Adv. Appl. Math.2: 482); similarity search method (Pearson et al., (1988) Proc.Natl.Acad.Sci.85: 2444); Smith-Waterman algorithm (Meth.Mol.Biol.70: 173-187 (1997); and BLASTP, BLASTN, and BLASTX algorithm (see Altschul et al., (1990) J.Mol.Biol.215: 403-410). The computer programs using these algorithms are also available, including but not limited to: ALIGN or Megalign (DNASTAR) software, or WU-BLAST-2 (Altschul et al., Meth. Enzym., 266: 460-480 (1996)); or GAP, BESTFIT, BLAST Altschul, etc., supra, FASTA and TFASTA, available from Genetics Computing Group (GCG) package, 8th edition, Madison, Wisconsin, USA; and CLUSTAL in PC/Gene program provided by Intelligenetics, Mountain View, California.

Without substantially affecting the activity of the antibody (retaining at least 90% of the activity), those skilled in the art may replace, add, and/or delete one or more (e.g., 1, 2, 3, 4, 5, 6, 7, 8, 9, 10 or more) amino acids in the sequence of the present disclosure to obtain variants of the sequence of the antibody or the antigen-binding fragment thereof, which are all considered to be included within the protection scope of the present disclosure. For example, amino acids with similar properties are substituted in the variable region. The variant sequence of the present disclosure may have at least 90%, 95%, 96%, 97%, 98%, or 99% identity (or homology) to the reference sequence. The sequence identity described in the present disclosure can be measured using sequence analysis software. For example, the computer program BLAST with default parameters is used, especially BLASTP or TBLASTN. The amino acid sequences described in the present disclosure are all shown in an N-terminal to C-terminal manner.

As used herein, the term "at least 80% homology" refers to at least 80% homology to each reference sequence, which can be 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9% homology to each reference sequence. The term "at least 90% homology" refers to at least 90% homology to each reference sequence, which can be 90%, 91%, 92%, 93%, 94%, 95%, 96%, 97%, 98%, 99%, 99.5%, 99.9% homology to each reference sequence.

As used herein, the term "variant" or "mutant" may refer to any naturally occurring or engineered molecule that contains one or more nucleotide or amino acid mutations.

As used herein, the term "expression vector" usually refers to a nucleic acid molecule that can be inserted into a suitable host for self-replication, to transfer the inserted cell or host into and/or between cells or hosts. The expression vector may include a vector mainly configured to insert DNA or RNA into cells, a vector mainly configured to replicate DNA or RNA, and an expression vector mainly configured to transcript and/or translate DNA or RNA. The expression vector further includes vectors with various functions as described above. The expression vector may be a polynucleotide, which can be transcribed and translated into a polypeptide when introduced into a suitable cell or host. Usually, the expression vector can produce a desired expression product by culturing the appropriate cell or host containing the expression vector.

As used herein, the term "recombinant cell" usually refers to a cell with stably inherited unique traits that are obtained by modifying or recombining the genetic material of a host cell by means of genetic engineering technology or cell fusion technology. The term "host cell" refers to a prokaryotic cell or a eukaryotic cell, into which a recombinant expression vector can be introduced. As used herein, the term "transform" or "transfect" refers to the introduction of a nucleic acid (such as a vector) into a cell by various techniques known in the art. Suitable host cells can be transformed or transfected with the DNA sequence of the present disclosure, and can be used for the expression and/or secretion of the target proteins. Examples of suitable host cells that can be used in the present disclosure include immortalized hybridoma cells, NS/0 myeloma cells, 293 cells, Chinese hamster ovary (CHO) cells, HeLa cells, Cap cells (cells derived from human amniotic fluid), and CoS cells.

The present disclosure provides an anti-human PLA2R antibody or an antigen-binding fragment thereof, a nucleic acid molecule, an expression vector, a recombinant cell, a kit and use thereof, which will be described in detail below.

### Antibody or antigen-binding fragment thereof

In an aspect of the present disclosure, the present disclosure provides an antibody or an antigen-binding fragment thereof. According to an embodiment of the present disclosure, the antibody or the antigen-binding fragment thereof includes at least one CDR sequence selected from the following amino acid sequences or amino acid sequences having at least 80% identity thereto: heavy chain variable region CDR sequences: SEQ ID NO: 1 to SEQ ID NO: 105; and light chain variable region CDR sequences: SEQ ID NO: 106 to SEQ ID NO: 210. The antibody or the antigen-binding fragment thereof has a high affinity to a PLA2R protein and can be used as an anti-human PLA2R antibody standard. The antibody or the antigen-binding fragment thereof can be obtained by cell recombinant expression, which has the advantages such as stable and large-scale production. Moreover, the antibody or the antigen-binding fragment thereof can be prevented from being contaminated by pathogenic microorganisms during a preparation process, thereby ensuring the purity and stability of the anti-human PLA2R antibody standard.

As used herein, the term "antibody" is used in the broadest sense, and it may include full-length monoclonal antibodies, multi-specific antibodies, and chimeric antibodies. The specific structure is not limited as long as they exhibit the desired biological activity. The antibody usually includes a light chain with a lighter molecular weight and a heavy chain with a heavier molecular weight. The heavy chain (H chain) and the light chain (L chain) are connected by disulfide bonds to form an antibody molecule. The amino acid sequence of the amino terminal (N terminal) of the peptide chain varies significantly, which is referred to as a variable region (V region), while the carboxyl terminal (C terminal) is relatively stable and varies insignificantly, which is referred to as a constant region (C region). The V region of the L chain and the V region of the H chain are referred to as VL and VH, respectively. Both VL and VH contain a region of main amino acid residues, i.e., Complementarity Determining Region (CDR), that contributes to the binding affinity for the recognized antigen or epitope.

As used herein, the terms "complementarity determining region", "CDR", or "CDRs" refer to highly variable regions of the heavy chain and the light chain of immunoglobulins, and refer to the regions containing one or more or even all of the main amino acid residues of the antibody or the antigen-binding fragment thereof that contribute to the binding affinity for recognized antigen or epitope.

As used herein, heavy chain complementary determining regions (CDR of the heavy chain variable region) are represented by "HCDRs" or "HCDR", which include HCDR1 (also known as CDR-H1), HCDR2 (also known as CDR-H2), and HCDR3 (also known as CDR-H3). As used herein, light chain complementary determining regions (CDR of the light chain variable region) are represented by "LCDRs" or "LCDR", which include LCDR1 (also known as CDR-L1), LCDR2 (also known as CDR-L2), and LCDR3 (also known as CDR-L3). The definition solutions of CDRs commonly used in the art include: Kabat, Chothia, IMGT, Contact, and AbM. As described herein, "Kabat" definition system refers to the definition system described by Kabat et al., U.S. Dept. of Health and Human Services, "Sequence of Proteins of Immunological Interest" (1983). "Chothia" definition system refers to Chothia et al., J Mol Biol 196: 901-917 (1987). As examples, Table A lists the definitions of CDRs. The definitions in different literature may be slightly different. Based on the amino acid sequence of the variable region of a given antibody, those skilled in the art can routinely determine which residues contain a specific CDR. It should be noted that, the CDRs of the present disclosure include, but are not limited to, CDRs defined by a system other than those in Table A, and CDRs determined based on the heavy chain variable region and light chain variable region disclosed in the present disclosure using other rules in the art also fall within the protection scope of the present disclosure.

**[Table A] CDR Definition¹**

| CDR | Kabat | AbM² | Chothia |
|---|---|---|---|
| HCDR1 | H31~H35³ | H26~H35³ | H26~H32..34⁴ |
| HCDR2 | H50~H65 | H50~H58 | H52~H56 |
| HCDR3 | H95~H102 | H95~H102 | H95~H102 |
| LCDR1 | L24~L34 | L24~L34 | L24~L34 |
| LCDR2 | L50~L56 | L50~L56 | L50~L56 |
| LCDR3 | L89~L97 | L89~L97 | L89~L97 |

| | | | |
|---|---|---|---|
| ¹ The numbering of all CDR definitions in Table A is based on the Kabat numbering system (see below), where the amino acid number on the heavy chain is represented by "H+number", and where the amino acid number on the light chain is represented by "L+number". ² "AbM" used in Table A with a lowercase "b" refers to the CDR defined by "AbM" antibody modeling software of Oxford Molecular. ³ If neither H35A nor H35B exists, CDR-H1 ends at position 35; if only H35A exists, CDR-H1 ends at position 35A; and if both H35A and H35B exist simultaneously, CDR-H1 ends at position 35B. ⁴ If neither H35A nor H35B exists, CDR-H1 ends at position 32; if only H35A exists, CDR-H1 ends at position 33; and if both H35A and H35B exist simultaneously, CDR-H1 ends at position 34. | | | |

Kabat *et al.* also define a numbering system applicable to the variable region sequences of any antibody. Those skilled in the art can clearly correspond the Kabat numbering system to any variable region sequence without relying on any experimental data other than the sequence itself. As used herein, "Kabat numbering" refers to the numbering using the numbering system described in Kabat et al., U.S. Dept. of Health and Human Services, "Sequence of Proteins of Immunological Interest" (1983)". The HCDRs and LCDRs of the antibody or the antigen-binding fragment thereof of the present disclosure are numbered using the above-mentioned numbering system, and specific numbering results are shown in Table A.

It should be noted that, the CDRs of the present disclosure are annotated based on domain alignment (refer to "Ehrenmann F, Kaas Q, Lefranc MP. IMGT/3Dstructure-DB and IMGT/DomainGapAlign: a database and a tool for immunoglobulins or antibodies, T cell receptors, MHC, IgSF and MhcSF. Nucleic Acids Res. 2010 Jan;38(Database issue):D301-7. PMID: 19900967." and "Ehrenmann F, Lefranc MP. IMGT/DomainGapAlign: IMGT standardized analysis of amino acid sequences of variable, constant, and groove domains (IG, TR, MH, IgSF, MhSF). Cold Spring Harb Protoc. 2011 Jun 1;2011(6):737-49. PMID: 21632775." for details). However, those skilled in the art are fully capable of converting the heavy chains and light chains in the sequence table into other numbering systems (such as Kabat, Chothia, Contact, and AbM) for definition according to the definition rules in Table A, and the CDRs obtained thereby are all within the protection scope of the present disclosure.

As used herein, the terms "full-length antibody", "full-length monoclonal antibody", or "full-length monoclonal antibody" are all composed of at least two identical light chains and at least two identical heavy chains connected by interchain disulfide bonds, such as immunoglobulin G (IgG), immunoglobulin A (IgA), immunoglobulin M (IgM), immunoglobulin D (IgD), or immunoglobulin E (IgE).

As used herein, the terms "polyclonal antibody" and "multi-specific antibody" are synonymous, and both refer to an antibody capable of recognizing multiple antigenic epitopes, such as an antibody capable of recognizing two antigenic epitopes (bispecific antibody, abbreviated as BsAb), an antibody capable of recognizing three antigenic epitopes, or an antibody capable of recognizing four antigenic epitopes. The terms are understood in a broad sense and specific structures thereof are not limited, as long as multiple antigenic epitopes can be recognized. In the present disclosure, at least one of the multiple antigenic epitopes is derived from cTnI.

As used herein, the term "antigen-binding fragment" refers to a fragment including part or all of an antibody, while lacking at least some of the amino acids present in the full-length chain but is still capable of specifically binding to an antigen. For example, the fragment may include part or all of the CDRs of the antibody. Such fragments are biologically active because they bind to an antigen and can compete with other antigen-binding molecules (including intact antibodies) for binding to a given epitope. Such fragments are selected from Fab, Fv, scFv, or single-domain antibodies. Such fragments can be produced by recombinant nucleic acid technology, or can be produced by enzymatic cleavage or chemical cleavage of antigen-binding molecules (including intact antibodies).

In some optional embodiments of the present disclosure, the above-mentioned antibody or antigen-binding fragment thereof may further include at least one of the following additional technical features.

In some optional embodiments of the present disclosure, the antibody or the antigen-binding fragment thereof includes: a heavy chain variable region CDR1 as set forth in an amino acid sequence selected from any one of SEQ ID NO: 1 to SEQ ID NO: 35 or an amino acid sequence having at least 80% identity thereto; a heavy chain variable region CDR2 as set forth in an amino acid sequence selected from any one of SEQ ID NO: 36 to SEQ ID NO: 70 or an amino acid sequence having at least 80% identity thereto; a heavy chain variable region CDR3 as set forth in an amino acid sequence selected from any one of SEQ ID NO: 71 to SEQ ID NO: 105 or an amino acid sequence having at least 80% identity thereto; a light chain variable region CDR1 as set forth in an amino acid sequence selected from any one of SEQ ID NO: 106 to SEQ ID NO: 140 or an amino acid sequence having at least 80% identity thereto; a light chain variable region CDR2 as set forth in an amino acid sequence selected from any one of SEQ ID NO: 141 to SEQ ID NO: 175 or an amino acid sequence having at least 80% identity thereto; or a light chain variable region CDR3 as set forth in an amino acid sequence selected from any one of SEQ ID NO: 176 to SEQ ID NO: 210 or an amino acid sequence having at least 80% identity thereto.

In some optional embodiments of the present disclosure, the antibody or the antigen-binding fragment thereof includes: heavy chain variable regions selected from any one of the following groups:

| Group No. | Antibody Name | HCDR1 | SEQ ID NO: | HCDR2 | SEQ ID NO: | HCDR3 | SEQ ID NO: |
|---|---|---|---|---|---|---|---|
| 1 | A2 | GGSISSG GY | 1 | YYSGS | 36 | CARQRRANGYSSSWEFDYW | 71 |
| 2 | A5 | GFTFSRY | 2 | SYDGSN | 37 | CARGGPTPDW | 72 |
| 3 | A8 | GGTFSSY | 3 | IPIFGT | 38 | CARDSDFWSGYSSMDVW | 73 |
| 4 | A13 | GFTFSSY | 4 | SSGSSY | 39 | CARDQGASVTGTHTHW | 74 |
| 5 | A21 | GYTFTDY | 5 | DPETGG | 40 | CTTTYGNYWYFDVW | 75 |
| 6 | A22 | GGTFSSY | 6 | IPIFGT | 41 | CAFTSGSYYYFDYW | 76 |
| 7 | A23 | GGTFSSY | 7 | IPIFGT | 42 | CARGGGGRGMTSDW | 77 |
| 8 | A26 | GFTFSSY | 8 | SGSGGS | 43 | CAKGTKMATIRLGYYYYGM DVW | 78 |
| 9 | A44 | GGSISSG GY | 9 | YYSGS | 44 | CARWGRGGSSWRFDIW | 79 |
| 10 | A53 | GGSISSG GY | 10 | YYSGS | 45 | CARDSRGRSYGMDVW | 80 |
| 11 | A61 | GGTFSSY | 11 | IPILGI | 46 | CATGVGMVRGDPW | 81 |
| 12 | A67 | GFTFSNY | 12 | SYDGSK | 47 | CATGYSSPWYGMDVW | 82 |
| 13 | A143 | GFTFSSY | 13 | SYDGSN | 48 | CARAAGHGILDYW | 83 |
| 14 | A156 | GGSISSG GY | 14 | YYSGS | 49 | CARIHHSTAGPYNNWYFDYW | 84 |
| 15 | A187 | GFTFSSY | 15 | SNDGSN | 50 | CARLLRGPWNFDLW | 85 |
| 16 | A378 | GFTFSSY | 16 | SGSGGS | 51 | CATSSGPRIDAFDIW | 86 |
| 17 | A382 | GYTFTSY | 17 | NAGNGN | 52 | CATSKSVWNFDYW | 87 |
| 18 | B16 | GFTFSSY | 18 | SGSGGS | 53 | CARSYGNYFTYW | 88 |
| 19 | B20 | GDSISNY | 19 | HYSGT | 54 | CARRGRIAAAGTGYYYYGM DVW | 89 |
| 20 | B24 | GFTFSSY | 20 | SSSSSY | 55 | CARGRRRDFDYW | 90 |
| 21 | B29 | GDTFSSF | 21 | IPLFTT | 56 | CARGPERIAAAGYYYYYGMD VW | 91 |
| 22 | B44 | GYTFTSY | 22 | NPSGGS | 57 | CARGVSRRGAFDIW | 92 |
| 23 | B53 | GFTFSSY | 23 | SYDGSN | 58 | CARNRYGATDYW | 93 |
| 24 | B67 | GGSISSSN | 24 | YHSGS | 59 | CARDDGWRYGSVYYYYMDV W | 94 |
| 25 | B83 | GFTFSSY | 25 | SGSGGR | 60 | CARVLQYSGTDDLDVW | 95 |
| 26 | B88 | GGTFSSY | 26 | IPILGI | 61 | CATGVGMVRGDPW | 96 |
| 27 | B90 | GLIFKNY | 27 | SGNSNY | 62 | CSSGYFYFDLW | 97 |
| 28 | B109 | GFTFSSY | 28 | SSSSSY | 63 | CVRRLWEAPGIGPFDVW | 98 |
| 29 | B114 | GFTFSGY | 29 | SYDGSN | 64 | CARGRGRGVFDYW | 99 |
| 30 | B117 | GFTFSSY | 30 | SSSSST | 65 | CARDRTLGYW | 100 |
| 31 | B123 | GGSISSG GY | 31 | YYSGS | 66 | CARWDRVREGGRFSNAFDIW | 101 |
| 32 | B145 | GGSISSG GY | 32 | YYSGS | 67 | CARGKRDGYNYYFDYW | 102 |
| 33 | B156 | GGSISSG GY | 33 | YYSGS | 68 | CARQRRANGYSSSWEFDYW | 103 |
| 34 | B201 | GFTFSSY | 34 | SGSGDS | 69 | CAKGSRRSGFNLFDYW | 104 |
| 35 | B307 | GGSISSG GY | 35 | YYSGS | 70 | CARMGRIAASNYYGMDVW | 105 |

and/or, light chain variable regions selected from any one of the following groups:

| Group No. | Antibody Name | LCDR1 | SEQ ID NO: | LCDR2 | SEQ ID NO: | LCDR3 | SEQ ID NO: |
|---|---|---|---|---|---|---|---|
| 1 | A2 | RASQGISSALA | 106 | DASSLES | 141 | CQQFNSYPLTF | 176 |
| 2 | A5 | RASQGIRNYLA | 107 | AASTLQ S | 142 | CQKYYSAPFTF | 177 |
| 3 | A8 | RSSQSLVHSDGNTYLN | 108 | KVSNRD S | 143 | CMQGTQWPDTF | 178 |
| 4 | A13 | RASQSVSSYLA | 109 | DASNRA T | 144 | CQQRSNWPPMYTF | 179 |
| 5 | A21 | RASQNIENWLA | 110 | ATSRLEN | 145 | CHQANGFPWTF | 180 |
| 6 | A22 | RSSQSLVHSDGNTYLN | 111 | KVSNRD S | 146 | CMQGTHWPPRYTF | 181 |
| 7 | A23 | RASQDISNYLA | 112 | AASSLQ S | 147 | CQQYKRYPFIF | 182 |
| 8 | A26 | RSSQSLLHSNGRNYLD | 113 | LGSNRA S | 148 | CMQALQTPWTF | 183 |
| 9 | A44 | RASQALTYTLA | 114 | DASSLES | 149 | CQQFRSYPLTF | 184 |
| 10 | A53 | RASQSINNYLN | 115 | AASSLQ S | 150 | CQQSYSYPFTF | 185 |
| 11 | A61 | RSSQSLVKSDGNTYLS | 116 | KVSNRD S | 151 | CMQGTHWPPTF | 186 |
| 12 | A67 | RASQSVNSYVN | 117 | AASSVH N | 152 | CQQSFTSPWTF | 187 |
| 13 | A143 | RASQSIARRIA | 118 | DASNRA T | 153 | CQQRANWPLTF | 188 |
| 14 | A156 | RASQGISSYLA | 119 | AASLLQ R | 154 | CQQFNTYPITF | 189 |
| 15 | A187 | RASQSVSSSYLA | 120 | GASSRA T | 155 | CQQYGSSPPITF | 190 |
| 16 | A378 | RASQGIRNSLA | 121 | AASKLQ T | 156 | CQQYYGTPPTF | 191 |
| 17 | A382 | RASQGISSYLA | 122 | DASNRA T | 157 | CQQRKNWPLTF | 192 |
| 18 | B16 | RASQSVSSYLA | 123 | DASNRA T | 158 | CQQRSNWPPELTF | 193 |
| 19 | B20 | RASQSVSNYLA | 124 | GASSRA T | 159 | CQQYGSSLWTF | 194 |
| 20 | B24 | RASQGIGTFLA | 125 | ASSTVQ R | 160 | CQQLNNYPRTF | 195 |
| 21 | B29 | RASQSVSSSSLA | 126 | GASSRA T | 161 | CQQYGTSWTF | 196 |
| 22 | B44 | RSSQSLLHRSGYNYLD | 127 | LGSIRAV | 162 | CMQALQTPPAF | 197 |
| 23 | B53 | RASQGINSWLA | 128 | AASTLQ S | 163 | CQQLNSFPFTF | 198 |
| 24 | B67 | RASQSIDDHLN | 129 | SASTLQS | 164 | CQQSYSPPQTF | 199 |
| 25 | B83 | RASQSISSYLN | 130 | AASSLQ S | 165 | CQQSYSTPRTF | 200 |
| 26 | B88 | RSSQSLVYSDGNTYLN | 131 | KVSNRD S | 166 | CMQGTHWPPTF | 201 |
| 27 | B90 | RASQSISSWLA | 132 | EASTLE V | 167 | CQQYHTDSRTF | 202 |
| 28 | B109 | RASQSISSYLN | 133 | AASSLQ S | 168 | CQQSYSTLTF | 203 |
| 29 | B114 | RASQSVSKYLA | 134 | DVSNRA T | 169 | CQQRRSWPLTF | 204 |
| 30 | B117 | RASQGIGRWLA | 135 | AASNLQ S | 170 | CQQANSFPRTF | 205 |
| 31 | B123 | RASQGISTYLA | 136 | GASTLQ S | 171 | CQQLNTYPPTF | 206 |
| 32 | B145 | QASQDISNYLN | 137 | DASSLES | 172 | CQQYRSYPITF | 207 |
| 33 | B156 | RASQGISSFLA | 138 | AASTLQ S | 173 | CQQFKIYPLTF | 208 |
| 34 | B201 | RASQSVSSSYLA | 139 | GASSRA T | 174 | CQQYGSSPPITF | 209 |
| 35 | B307 | RASQGISSQLA | 140 | AASTLQ S | 175 | CQQVNAYPLTF | 210 |

In some optional embodiments of the present disclosure, the antibody or the antigen-binding fragment thereof includes any one of the following groups:

| Group No. | HCDR1 (SEQ ID NO:) | HCDR2 (SEQ ID NO:) | HCDR3 (SEQ ID NO:) | LCDR1 (SEQ ID NO:) | LCDR2 (SEQ ID NO:) | LCDR3 (SEQ ID NO:) |
|---|---|---|---|---|---|---|
| 1 | 1 | 36 | 71 | 106 | 141 | 176 |
| 2 | 2 | 37 | 72 | 107 | 142 | 177 |
| 3 | 3 | 38 | 73 | 108 | 143 | 178 |
| 4 | 4 | 39 | 74 | 109 | 144 | 179 |
| 5 | 5 | 40 | 75 | 110 | 145 | 180 |
| 6 | 6 | 41 | 76 | 111 | 146 | 181 |
| 7 | 7 | 42 | 77 | 112 | 147 | 182 |
| 8 | 8 | 43 | 78 | 113 | 148 | 183 |
| 9 | 9 | 44 | 79 | 114 | 149 | 184 |
| 10 | 10 | 45 | 80 | 115 | 150 | 185 |
| 11 | 11 | 46 | 81 | 116 | 151 | 186 |
| 12 | 12 | 47 | 82 | 117 | 152 | 187 |
| 13 | 13 | 48 | 83 | 118 | 153 | 188 |
| 14 | 14 | 49 | 84 | 119 | 154 | 189 |
| 15 | 15 | 50 | 85 | 120 | 155 | 190 |
| 16 | 16 | 51 | 86 | 121 | 156 | 191 |
| 17 | 17 | 52 | 87 | 122 | 157 | 192 |
| 18 | 18 | 53 | 88 | 123 | 158 | 193 |
| 19 | 19 | 54 | 89 | 124 | 159 | 194 |
| 20 | 20 | 55 | 90 | 125 | 160 | 195 |
| 21 | 21 | 56 | 91 | 126 | 161 | 196 |
| 22 | 22 | 57 | 92 | 127 | 162 | 197 |
| 23 | 23 | 58 | 93 | 128 | 163 | 198 |
| 24 | 24 | 59 | 94 | 129 | 164 | 199 |
| 25 | 25 | 60 | 95 | 130 | 165 | 200 |
| 26 | 26 | 61 | 96 | 131 | 166 | 201 |
| 27 | 27 | 62 | 97 | 132 | 167 | 202 |
| 28 | 28 | 63 | 98 | 133 | 168 | 203 |
| 29 | 29 | 64 | 99 | 134 | 169 | 204 |
| 30 | 30 | 65 | 100 | 135 | 170 | 205 |
| 31 | 31 | 66 | 101 | 136 | 171 | 206 |
| 32 | 32 | 67 | 102 | 137 | 172 | 207 |
| 33 | 33 | 68 | 103 | 138 | 173 | 208 |
| 34 | 34 | 69 | 104 | 139 | 174 | 209 |
| 35 | 35 | 70 | 105 | 140 | 175 | 210 |

In some optional embodiments of the present disclosure, the antibody or the antigen-binding fragment thereof specifically recognizes a PLA2R protein.

In some optional embodiments of the present disclosure, the antibody or the antigen-binding fragment thereof specifically recognizes a PLA2R antigen epitope peptide.

As used herein, the term "PLA2R antigen epitope peptide" refers to a polypeptide whose amino acid sequence is derived from PLA2R. The "PLA2R antigen epitope peptide" includes an antigen epitope that can specifically bind to the PLA2R antibody. For example, the PLA2R antigen epitope peptide includes CysR, FnII, and a PLA2R binding region 1. The term "CysR" refers to a CysR domain in PLA2R, the term "PLA2R binding region 1" refers to CTLD1 (or CTLD domain 1) in PLA2R. The specific structure is illustrated in FIG. 1.

In some optional embodiments of the present disclosure, the antibody or the antigen-binding fragment thereof specifically recognizes a CysR domain, a FNII domain, and a CTLD1 domain in a PLA2R protein.

In some optional embodiments of the present disclosure, the antibody or the antigen-binding fragment thereof further includes at least one of a heavy chain framework region sequence and a light chain framework region sequence. At least a portion of the at least one of the heavy chain framework region sequence and the light chain framework region sequence is derived from at least one of a murine antibody, a humanized antibody, a primate-derived antibody, or a mutant thereof.

As used herein, the "framework region" or "FR" region includes the heavy chain framework region and the light chain framework region, which refer to the regions other than the CDR in the variable region of the heavy chain of the antibody (which can be represented as VH) and the variable region of the light chain of the antibody (which can be represented as VL), respectively. The heavy chain framework region is represented by "HFR" and can be further subdivided into adjacent regions separated by CDRs, including an HFR1 framework region, an HFR2 framework region, an HFR3 framework region, and an HFR4 framework region. The light chain framework region is represented by "LFR" and can be further subdivided into adjacent regions separated by CDRs, including a LFR1 framework region, a LFR2 framework region, a LFR3 framework region, and a LFR4 framework region.

In some optional embodiments of the present disclosure, at least a part of the heavy chain framework region sequence and the light chain framework region sequence is derived from a humanized antibody or a mutant thereof.

In some optional embodiments of the present disclosure, the antibody or the antigen-binding fragment thereof includes a heavy chain variable region with an amino acid sequence as set forth in any one of SEQ ID NO: 211 to SEQ ID NO: 245.

In some optional embodiments of the present disclosure, the antibody or the antigen-binding fragment thereof includes a light chain variable region with an amino acid sequence as set forth in any one of SEQ ID NO: 246 to SEQ ID NO: 280.

In some optional embodiments of the present disclosure, the antibody or the antigen-binding fragment thereof includes any one of the following groups:

| Group No. | VH (SEQ ID NO:) | VL (SEQ ID NO:) | Group No. | VH (SEQ ID NO:) | VL (SEQ ID NO:) |
|---|---|---|---|---|---|
| 1 | 211 | 246 | 19 | 229 | 264 |
| 2 | 212 | 247 | 20 | 230 | 265 |
| 3 | 213 | 248 | 21 | 231 | 266 |
| 4 | 214 | 249 | 22 | 232 | 267 |
| 5 | 215 | 250 | 23 | 233 | 268 |
| 6 | 216 | 251 | 24 | 234 | 269 |
| 7 | 217 | 252 | 25 | 235 | 270 |
| 8 | 218 | 253 | 26 | 236 | 271 |
| 9 | 219 | 254 | 27 | 237 | 272 |
| 10 | 220 | 255 | 28 | 238 | 273 |
| 11 | 221 | 256 | 29 | 239 | 274 |
| 12 | 222 | 257 | 30 | 240 | 275 |
| 13 | 223 | 258 | 31 | 241 | 276 |
| 14 | 224 | 259 | 32 | 242 | 277 |
| 15 | 225 | 260 | 33 | 243 | 278 |
| 16 | 226 | 261 | 34 | 244 | 279 |
| 17 | 227 | 262 | 35 | 245 | 280 |
| 18 | 228 | 263 | | | |

In some optional embodiments of the present disclosure, the antibody includes at least one of a heavy chain constant region and a light chain constant region. At least a portion of the at least one of the heavy chain constant region and the light chain constant region is derived from at least one of a murine antibody, a humanized antibody, a primate-derived antibody, or a mutant thereof.

In some optional embodiments of the present disclosure, the heavy chain constant region includes a heavy chain constant region selected from IgG1, IgG2, IgG3, IgG4, IgA, IgM, IgE, or IgD; or the light chain constant region includes a light chain constant region selected from a κ-type light chain constant region or a λ-type light chain constant region.

In some optional embodiments of the present disclosure, the light chain constant region and the heavy chain constant region are both derived from a humanized IgG4 antibody or a mutant thereof.

In some optional embodiments of the present disclosure, the heavy chain constant region is a wild-type humanized IgG4 or a humanized IgG4 mutant.

In some optional embodiments of the present disclosure, the humanized IgG4 mutant (having the amino acid sequence as set forth in SEQ ID NO: 282) has a mutation at S228P site compared to the heavy chain constant region of the wild-type humanized IgG4 antibody.

It should be noted that, the numbering of the above-mentioned site is based on that the amino acids of the Fc segment of the wild-type human IgG4 (having the amino acid sequence as set forth in SEQ ID NO: 281) is numbered according to the EU numbering system. For example, position 228 refers to the 228-th position numbered according to the EU numbering system; and the "S228P" means that the serine at the 228-th position according to the EU numbering system is replaced by the proline.

In some optional embodiments of the present disclosure, the heavy chain constant region has an amino acid sequence as set forth in SEQ ID NO: 281 or SEQ ID NO: 282.

In some optional embodiments of the present disclosure, the light chain constant region has an amino acid sequence as set forth in SEQ ID NO: 357.

In some optional embodiments of the present disclosure, the antibody or the antigen-binding fragment thereof includes: a heavy chain with an amino acid sequence as set forth in any one of SEQ ID NO: 283 to SEQ ID NO: 317 and SEQ ID NO: 358 to SEQ ID NO: 366; or a light chain with an amino acid sequence as set forth in any one of SEQ ID NO: 318 to SEQ ID NO: 352.

In some optional embodiments of the present disclosure, the antibody or the antigen-binding fragment thereof includes any one of the following groups:

| Group No. | Antibody Name | Heavy Chain (SEQ ID NO:) | Heavy Chain Name | Light Chain (SEQ ID NO:) | Light Chain Name |
|---|---|---|---|---|---|
| 1 | A2 | 283 | Heavy Chain-A2 | 318 | Light Chain-A2 |
| 2 | A5 | 284 | Heavy Chain-A5 | 319 | Light Chain-A5 |
| 3 | A8 | 285 | Heavy Chain-A8 | 320 | Light Chain-A8 |
| 4 | A13 | 286 | Heavy Chain-A13 | 321 | Light Chain-A13 |
| 5 | A21 | 287 | Heavy Chain-A21 | 322 | Light Chain-A21 |
| 6 | A22 | 288 | Heavy Chain-A22 | 323 | Light Chain-A22 |
| 7 | A23 | 289 | Heavy Chain-A23 | 324 | Light Chain-A23 |
| 8 | A26 | 290 | Heavy Chain-A26 | 325 | Light Chain-A26 |
| 9 | A44 | 291 | Heavy Chain-A44 | 326 | Light Chain-A44 |
| 10 | A53 | 292 | Heavy Chain-A53 | 327 | Light Chain-A53 |
| 11 | A61 | 293 | Heavy Chain-A61 | 328 | Light Chain-A61 |
| 12 | A67 | 294 | Heavy Chain-A67 | 329 | Light Chain-A67 |
| 13 | A143 | 295 | Heavy Chain-A143 | 330 | Light Chain-A143 |
| 14 | A156 | 296 | Heavy Chain-A156 | 331 | Light Chain-A156 |
| 15 | A187 | 297 | Heavy Chain-A187 | 332 | Light Chain-A187 |
| 16 | A378 | 298 | Heavy Chain-A378 | 333 | Light Chain-A378 |
| 17 | A382 | 299 | Heavy Chain-A382 | 334 | Light Chain-A382 |
| 18 | B16 | 300 | Heavy Chain-B16 | 335 | Light Chain-B16 |
| 19 | B20 | 301 | Heavy Chain-B20 | 336 | Light Chain-B20 |
| 20 | B24 | 302 | Heavy Chain-B24 | 337 | Light Chain-B24 |
| 21 | B29 | 303 | Heavy Chain-B29 | 338 | Light Chain-B29 |
| 22 | B44 | 304 | Heavy Chain-B44 | 339 | Light Chain-B44 |
| 23 | B53 | 305 | Heavy Chain-B53 | 340 | Light Chain-B53 |
| 24 | B67 | 306 | Heavy Chain-B67 | 341 | Light Chain-B67 |
| 25 | B83 | 307 | Heavy Chain-B83 | 342 | Light Chain-B83 |
| 26 | B88 | 308 | Heavy Chain-B88 | 343 | Light Chain-B88 |
| 27 | B90 | 309 | Heavy Chain-B90 | 344 | Light Chain-B90 |
| 28 | B109 | 310 | Heavy Chain-B109 | 345 | Light Chain-B109 |
| 29 | B114 | 311 | Heavy Chain-B114 | 346 | Light Chain-B114 |
| 30 | B117 | 312 | Heavy Chain-B117 | 347 | Light Chain-B117 |
| 31 | B123 | 313 | Heavy Chain-B123 | 348 | Light Chain-B123 |
| 32 | B145 | 314 | Heavy Chain-B145 | 349 | Light Chain-B145 |
| 33 | B156 | 315 | Heavy Chain-B156 | 350 | Light Chain-B156 |
| 34 | B201 | 316 | Heavy Chain-B201 | 351 | Light Chain-B201 |
| 35 | B307 | 317 | Heavy Chain-B307 | 352 | Light Chain-B307 |
| 36 | A2 (IgG4SP) | 358 | Heavy Chain-A2 (IgG4SP) | 318 | Light Chain-A2 |
| 37 | A5 (IgG4SP) | 359 | Heavy Chain-A5 (IgG4SP) | 319 | Light Chain-A5 |
| 38 | A13 (IgG4SP) | 360 | Heavy Chain-A13 (IgG4SP) | 321 | Light Chain-A13 |
| 39 | A61 (IgG4SP) | 361 | Heavy Chain-A61 (IgG4SP) | 328 | Light Chain-A61 |
| 40 | A156 (IgG4SP) | 362 | Heavy Chain-A156 (IgG4SP) | 331 | Light Chain-A156 |
| 41 | A187 (IgG4SP) | 363 | Heavy Chain-A187 (IgG4SP) | 332 | Light Chain-A187 |
| 42 | B88 (IgG4SP) | 364 | Heavy Chain-B88 (IgG4SP) | 343 | Light Chain-B88 |
| 43 | B90 (IgG4SP) | 365 | Heavy Chain-B90 (IgG4SP) | 344 | Light Chain-B90 |
| 44 | B156 (IgG4SP) | 366 | Heavy Chain-B156 (IgG4SP) | 350 | Light Chain-B156 |

In some optional embodiments of the present disclosure, the antibody is selected from at least one of a monoclonal antibody, a polyclonal antibody, a multimeric antibody, and a CDR-grafted antibody.

In some optional embodiments of the present disclosure, the antibody includes at least one selected from a single-chain antibody, a Fab antibody, a Fab' antibody, a F(ab')₂ antibody, a Fv antibody, a single-chain antibody, a single-domain antibody, and a minimum recognition unit.

In some optional embodiments of the present disclosure, the antigen-binding fragment of the antibody includes at least one selected from a Fab fragment, a Fab' fragment, a F(ab)₂ fragment, a F(ab')₂ fragment, a Fv fragment, a scFv fragment, a scFv-Fc fusion protein, a scFv-Fv fusion protein, a Fv fragment, and a minimum recognition unit.

The antigen-binding fragments of the above-mentioned antibodies usually have the same binding specificity as the antibodies from which they are derived. Those skilled in the art can easily understand based on the contents of the disclosure that the antigen-binding fragments of the above-mentioned antibodies can be obtained by methods such as enzymatic digestion (including pepsin or papain) and/or by a method of chemically reducing and cleaving disulfide bonds. Based on the structure of the intact antibody of the present disclosure, those skilled in the art can easily obtain the above-mentioned antigen-binding fragments.

The antigen-binding fragments of the above-mentioned antibodies can also be obtained through recombinant genetics techniques known to those skilled in the art or by synthesis using, for example, an automatic peptide synthesizer, such as the automatic peptide synthesizer sold by Applied BioSystems, etc.

As used herein, the terms "polyclonal antibody" and "multi-specific antibody" are synonymous, and they both refer to an antibody capable of recognizing multiple antigenic epitopes, such as an antibody capable of recognizing two antigenic epitopes (abbreviated as BsAb), an antibody capable of recognizing three antigenic epitopes, or an antibody capable of recognizing four antigenic epitopes. The terms are understood in a broad sense, and specific structures are not limited as long as multiple antigenic epitopes can be recognized.

As used herein, the terms "CDR-grafted antibody" and "modified antibody" both refer to the transplantation of the CDR of a monoclonal antibody from one species to the variable region of an antibody from another species. For example, the CDRs of a mouse monoclonal antibody can be transplanted into the variable region of a human antibody to replace the CDRs of the humanized antibody, allowing the humanized antibody to obtain the antigen binding specificity of the murine monoclonal antibody while reducing its heterology. It should be noted that, both polyclonal antibodies and monoclonal antibodies of the present disclosure may be CDR-grafted antibodies.

As used herein, the terms "single domain antibody", "nanobody", and "VHH antibody" are used interchangeably. The terms were originally described as the antigen-binding immunoglobulin (variable) domain (Hamers-Casterman C, AtarhouchT, Muyldermans S, Robinson G, Hamers C, Songa EB, Bendahman N, Hamers R .:"Naturallyoccurring antibodies devoid of light chains"; Nature 363 ,446-448(1993)) of a "heavy chain antibody" (i.e. "an antibody lacking a light chain"), which includes a heavy chain variable region (VH) and conventional CH2 and CH3 regions, and specifically binds to an antigenic protein (such as cTnI) through the variable region of the heavy chain.

As used herein, the term "Fab antibody" or "Fab fragment" usually refers to an antibody or fragment containing only Fab molecules, which is composed of VH and CH1 of the heavy chain and the intact light chain. The light chain and the heavy chain are connected by a disulfide bond.

As used herein, the term "F(ab')₂ antibody" or "F(ab')₂ fragment" has two antigen-binding F(ab') parts linked together by disulfide bonds.

As used herein, the term "Fv antibody" or "Fv fragment" usually refers to an antibody or a fragment only composed of the light chain variable region (VL) and the heavy chain variable region (VH) connected by non-covalent bonds, and it is the smallest functional fragment of the antibody that retains the complete antigen-binding site.

As used herein, the terms "single-chain antibody" and "scFv fragment" refer to antibodies or fragments composed of the heavy chain variable region and the light chain variable region of the antibody that are connected by a short peptide.

As used herein, the terms "minimum recognition unit" and "MRU" both refer to antibodies or fragments composed of only one CDR, with an extremely small molecular weight that accounts for only about 1% of the complete antibody.

### Nucleic acid molecule, expression vector, recombinant cell, kit, and uses thereof

In a process of preparing or obtaining the above-mentioned antibodies or the antigen-binding fragments thereof, nucleic acid molecules expressing these antibodies or antigen-binding fragments can be used, connected to different vectors, and then expressed in different cells to obtain the corresponding antibodies or the antigen-binding fragments thereof.

In another aspect of the present disclosure, the present disclosure provides a nucleic acid molecule. According to an embodiment of the present disclosure, the nucleic acid molecule encodes the above-mentioned antibody or antigen-binding fragment thereof. The nucleic acid molecule according to the embodiment of the present disclosure can encode and obtain the above-mentioned antibody or antigen-binding fragment thereof.

In some optional embodiments of the present disclosure, the nucleic acid molecule is DNA.

It should be noted that, for the nucleic acid molecules mentioned herein, those skilled in the art should understand that they actually include any one or two of the complementary double strands. In the present disclosure, for convenience, only one strand is given in most cases, but the other strand complementary to the one strand is actually also disclosed. In addition, the molecular sequences of the present disclosure include a DNA form or an RNA form, the disclosure of one of which implies the disclosure of the other.

In yet another aspect of the present disclosure, the present disclosure provides an expression vector. According to an embodiment of the present disclosure, the expression vector carries the above-mentioned nucleic acid molecule. When the above-mentioned nucleic acid molecule is connected to the vector, the nucleic acid molecule can be directly or indirectly connected to control elements on the expression vector, as long as these control elements can control the translation and expression of the nucleic acid molecule and so on. These control elements may be directly derived from the expression vector itself, or they may be exogenous, i.e., derived from the expression vector itself. The nucleic acid molecule is operably connected to the control elements. As used herein, "operably connected" means that an exogenous gene is connected to the vector, in such a manner that the control elements in the vector, such as transcription control sequences and translation control sequences, can play their expected functions of regulating the transcription and translation of the exogenous gene. Common vectors may be, for example, plasmids, bacteriophages, etc. Once the vector according to some specific embodiments of the present disclosure is introduced into suitable recipient cells, the expression of the above-mentioned antibodies or the antigen-binding fragments thereof can be effectively achieved under the mediation of the regulatory system, thereby obtaining a large amount of the antibodies or the antigen-binding fragments thereof *in vitro.*

In some optional embodiments of the present disclosure, the expression vector is a eukaryotic expression vector or a prokaryotic expression vector.

In some optional embodiments of the present disclosure, the expression vector is a plasmid expression vector.

In yet another aspect of the present disclosure, the present disclosure provides a recombinant cell. According to the embodiments of the present disclosure, the recombinant cell carries the above-mentioned nucleic acid molecule or expresses the above-mentioned antibody or antigen-binding fragment thereof. Under suitable conditions, this cell can be used to effectively express the above-mentioned antibody or antigen-binding fragment thereof in the cell.

In some optional embodiments of the present disclosure, the recombinant cell is obtained by introducing the above-mentioned expression vector into a host cell.

In some optional embodiments of the present disclosure, the recombinant cell is a eukaryotic cell.

In some optional embodiments of the present disclosure, the recombinant cell is a mammalian cell.

It should be noted that, the cell of the present disclosure is not particularly limited and may be a prokaryotic cell, a eukaryotic cell, or a bacteriophage. The prokaryotic cell may be *Escherichia coli, Bacillus subtilis, Streptomyces,* or *Proteus mirabilis,* etc. The eukaryotic cell include fungi such as *Pichia pastoris, Saccharomyces cerevisiae, Schizosaccharomyces pombe, Trichoderma spp.,* insect cells such as *Spodoptera frugiperd,* plant cells such as tobacco, and mammalian cells such as BHK cells, CHO cells, COS cells, and myeloma cells.

In an optional embodiment of the present disclosure, the recombinant cell is the mammalian cell, including BHK cells, CHO cells, NSO cells, or COS cells, and does not include animal germ cells, fertilized eggs, or embryonic stem cells.

It should be noted that, the "suitable conditions" described in the present disclosure refer to conditions suitable for the expression of the antibody or the antigen-binding fragment thereof described in the present disclosure. Those skilled in the art can understand that the conditions suitable for the expression of the antibody or the antigen-binding fragment thereof include, but are not limited to, appropriate transformation or transfection methods, appropriate transformation or transfection conditions, healthy cell states, appropriate cell density, suitable cell culture environment, and suitable cell culture time. The "suitable conditions" are not particularly limited, and those skilled in the art can optimize the most suitable conditions for the expression of the antibody or the antigen-binding fragment thereof according to the specific environment of the laboratory.

In yet another aspect of the present disclosure, the present disclosure provides use of the above-mentioned antibody or antigen-binding fragment thereof, the above-mentioned nucleic acid molecule, the above-mentioned expression vector, or the above-mentioned recombinant cell as a PLA2R antibody standard. The method according to some specific embodiments of the present disclosure can effectively obtain the antibody or the antigen-binding fragment thereof in large quantities.

Based on the amino acid sequence of the antibody or the antigen-binding fragment thereof in the present disclosure, those skilled in the art can think of using genetic engineering technology or other technologies (chemical synthesis, recombinant expression) to prepare the antibody or the antigen-binding fragment thereof. For example, the antibody or the antigen-binding fragment thereof can be isolated and purified from a culture product of a recombinant cell capable of recombinantly expressing the antibody or the antigen-binding fragment thereof described in any one of the above, which is easy to achieve for those skilled in the art. Based on this, the antibody or the antigen-binding fragment thereof prepared with any technology in the present disclosure shall fall within the protection scope of the present disclosure.

In yet another aspect of the present disclosure, the present disclosure provides a kit. According to an embodiment of the present disclosure, the kit includes: the above-mentioned antibody or antigen-binding fragment thereof; the above-mentioned nucleic acid molecule; the above-mentioned expression vector; or the above-mentioned recombinant cell. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the kit containing the above-mentioned antibody or antigen-binding fragment thereof has the advantages such as high accuracy in the detection of the PLA2R antibody.

In an optional embodiment of the present disclosure, the kit may also include a reagent for detecting the PLA2R antibody.

In yet another aspect of the present disclosure, the present disclosure provides use of the above-mentioned antibody or antigen-binding fragment thereof, the above-mentioned nucleic acid molecule, the above-mentioned expression vector, or the above-mentioned recombinant cell in the preparation of a kit for detecting a PLA2R antibody or diagnosing PLA2R antibody-positive membranous nephropathy. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the kit containing the above-mentioned antibody or antigen-binding fragment thereof has the advantages such as high accuracy in the detection of the PLA2R antibody.

In yet another aspect of the present disclosure, the present disclosure provides use of the above-mentioned antibody or antigen-binding fragment thereof, the above-mentioned nucleic acid molecule, the above-mentioned expression vector, or the above-mentioned recombinant cell in the detection of a PLA2R antibody or in the diagnosis of PLA2R antibody-positive membranous nephropathy. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the kit containing the above-mentioned antibody or antigen-binding fragment thereof has the advantages such as high accuracy in the detection of the PLA2R antibody.

In yet another aspect of the present disclosure, the present disclosure provides the above-mentioned antibody or antigen-binding fragment thereof, the above-mentioned nucleic acid molecule, the above-mentioned expression vector, or the above-mentioned recombinant cell for use in the detection of a PLA2R antibody or the diagnosis of PLA2R antibody-positive membranous nephropathy. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the kit containing the above-mentioned antibody or antigen-binding fragment thereof has the advantages such as high accuracy in the detection of the PLA2R antibody.

### Method

In yet another aspect of the present disclosure, the present disclosure provides a method for detecting a PLA2R antibody. According to an embodiment of the present disclosure, the method includes: determining, based on a detection result of the PLA2R antibody, a content of the PLA2R antibody in a sample to be tested by using the above-mentioned antibody or antigen-binding fragment thereof as a PLA2R antibody standard. In this way, the accuracy of PLA2R antibody detection can be improved. As mentioned above, the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard. In this way, the method of the present disclosure has the advantages such as high accuracy in the detection of the PLA2R antibody.

In yet another aspect of the present disclosure, the present disclosure provides a method for diagnosing PLA2R antibody-positive membranous nephropathy. According to an embodiment of the present disclosure, the method includes: determining, based on a detection result of the PLA2R antibody in a sample to be tested, a content of the PLA2R antibody in the sample to be tested by using the above-mentioned antibody or antigen-binding fragment thereof as a PLA2R antibody standard; and determining, based on the content of the PLA2R antibody, whether a patient corresponding to the sample to be tested suffers from the PLA2R antibody-positive membranous nephropathy. In this way, the accuracy of PLA2R antibody detection can be improved. As mentioned above the above-mentioned antibody or antigen-binding fragment thereof has a high affinity to a PLA2R protein and can be produced stably and in large scale, and the contamination of pathogenic microorganisms can be eliminated. Thus, the above-mentioned antibody or antigen-binding fragment thereof can be used as an anti-human PLA2R antibody standard, and can accurately detect the content of the PLA2R antibody. In this way, the method of the present disclosure has a high diagnostic accuracy for the PLA2R antibody-positive membranous nephropathy.

The solutions of the present disclosure will be explained with examples. Those skilled in the art can understand that the following examples are only used to illustrate the present disclosure and should not be regarded as limitations of the scope of the present disclosure. If the specific technologies or conditions are not specified in the examples, they shall be carried out according to the technologies or conditions as described in the literature in the art or according to the product instructions. The reagents and instruments used without indicating the manufacturer are all conventional products that can be purchased commercially.

### Example 1: Preparation of Major Antigenic Region CC1H of PLA2R

### 1.1 Construction Method of Recombinant Proteins of Main Antigenic Region of PLA2R

The plasmid vector used was a pcDNA3.4 transient expression vector, which contained a native full-length CMV promoter and a WPRE element downstream of the cloning sites. The expression level of the vector based on CMV promoter was usually good for the transient expression system of the CHO cells. The WPRE element is located downstream of the multiple cloning sites and can effectively improve the transcription and expression of genes. *Escherichia coli* has an ampicillin resistance gene. Then, the vector gene pcDNA3.4 was amplified by means of PCR technology and connected to a synthetic gene, which is referred to as a gene sequence connecting the gene of interest and the signal peptide. The PCR template was obtained by gene synthesis, the pcDNA3.4 vector fragment and the synthetic gene fragment were connected by using a homologous recombination kit. A single clone was obtained by transformation in DH5α competent cells. The single clone was amplified and sequenced. The plasmid, after being confirmed based on the sequencing, was extracted to obtain the synthetic gene recombinant expression plasmid, in which the synthetic gene was cloned between the CMV promoter and the WPRE gene of the plasmid pcDNA3.4, as illustrated in FIG. 2 for details.

The amino acid sequence of the signal peptide was MLLSPSLLLLLLLGAPRGCA (SEQ ID NO: 353).

The nucleotide sequence of the signal peptide was

ATGCTGCTGTCGCCGTCGCTGCTGCTGCTGCTGCTGGGGGCGCCGCGGGGCT
GCGCC (SEQ ID NO: 354).

FIG. 1 illustrates a schematic structural diagram of PLA2R. The gene of interest was the amino acid sequences 1 to 367 (Glu) of the PLA2R fragment. The constructed transient expression plasmid was named pcDNA3.4-CC1H.

The amino acid sequence of the synthetic gene (the major antigenic region of PLA2R) was:

MLLSPSLLLLLLLGAPRGCAEGVAAALTPERLLEWQDKGIFVIQSESLKKCIQAGKSVL
TLENCKQANKHMLWKWVSNHGLFNIGGSGCLGLNFSAPEQPLSLYECDSTLVSLRWR
CNRKMITGPLQYSVQVAHDNTVVASRKYIHKWISYGSGGGDICEYLHKDLHTIKGNT
HGMPCMFPFQYNHQWHHECTREGREDDLLWCATTSRYERDEKWGFCPDPTSAEVG
CDTIWEKDLNSHICYQFNLLSSLSWSEAHSSCQMQGGTLLSITDETEENFIREHMSSKT
VEVWMGLNQLDEHAGWQWSDGTPLNYLNWSPEVNFEPFVEDHCGTFSSFMPSAWR
SRDCESTLPYICKKYLNHIDHEIVEGGHHHHHH (SEQ ID NO: 355).

The nucleotide sequence of the synthetic gene (the major antigenic region of PLA2R) was:

ATGCTGCTGTCGCCGTCGCTGCTGCTGCTGCTGCTGGGGGCGCCGCGGGGCT
GCGCCGAGGGTGTGGCGGCGGCGCTTACCCCCGAGCGGCTCCTGGAGTGGCAGG
ATAAAGGAATATTTGTTATCCAAAGTGAGAGTCTCAAGAAATGCATTCAAGCAGGT
AAATCGGTTCTGACCCTGGAGAACTGCAAGCAAGCAAACAAGCACATGCTGTGGA
AATGGGTTTCAAACCATGGCCTCTTTAACATAGGAGGCAGTGGTTGCCTGGGCCTG
AATTTCTCCGCCCCAGAGCAGCCATTAAGCTTATATGAATGTGACTCCACCCTCGTT
TCCTTACGGTGGCGCTGTAACAGGAAGATGATCACAGGCCCGCTGCAGTACTCTGT
CCAGGTGGCGCATGACAACACAGTGGTGGCCTCACGGAAGTATATTCATAAGTGG
ATTTCTTATGGGTCAGGTGGTGGAGACATTTGTGAATATCTACACAAAGATTTGCAT
ACAATCAAAGGGAACACCCACGGGATGCCGTGTATGTTTCCCTTCCAGTATAACCA
TCAGTGGCATCATGAATGTACCCGTGAAGGTCGGGAAGATGACTTACTGTGGTGTG
CCACGACAAGCCGTTATGAAAGAGATGAAAAGTGGGGATTTTGCCCTGATCCCAC
CTCTGCAGAAGTAGGTTGTGATACTATTTGGGAGAAGGACCTCAATTCACACATTT
GCTACCAGTTCAACCTGCTTTCATCTCTCTCTTGGAGTGAGGCACATTCTTCATGCC
AGATGCAAGGAGGTACGCTGTTAAGTATTACAGATGAAACTGAAGAAAATTTCATA
AGGGAGCACATGAGCAGTAAAACAGTGGAGGTGTGGATGGGCCTCAATCAGCTG
GATGAACACGCTGGCTGGCAGTGGTCTGATGGAACGCCGCTCAACTATCTGAATTG
GAGCCCAGAGGTAAATTTTGAGCCATTTGTTGAAGATCACTGTGGAACATTTAGTT
CATTTATGCCAAGTGCCTGGAGGAGTCGGGATTGTGAGTCCACCTTGCCATATATAT
GTAAAAAATATCTAAACCACATTGATCATGAAATAGTTGAAGGAGGTCATCACCAT
CACCATCAC (SEQ ID NO: 356).

### 1.2 Transient Expression of CC1H

About 24 hours before transfection, FreeStyle^{™} CHO-S^{®} cells (with a cell density of 5 to 6×10⁵/mL, from the FreeStyle^{™} MAX CHO expression system of Gibco, USA, catalog number: K900020) were taken and cultured under the conditions of 37°C, 5% CO₂, and 120 rpm/min to 135 rpm/min.

On the day of transfection, the cells were diluted to a cell density of 1×10⁶/mL, and 10 mL of cells were added to each shake flask (the cell survival rate was above 95%).

FreeStyle^{™} MAX Reagent was mixed gently and carefully several times, without causing swirl.

12.5 µg of pcDNA3.4-CC1H was taken and added with OptiPRO^{™} SFM to a total volume of 0.2 mL, and the mixture was mixed gently.

12.5 µL of FreeStyle^{™} MAX Reagent was taken and added with Opti-Pro^{™} SFM to a total volume of 0.2 mL, and the mixture was mixed gently. After mixing evenly, the mixture was added to the plasmid mixture and mixed gently to obtain 0.4 mL of DNA-FreeStyle^{™} MAX mixture, and incubated at room temperature for 10 minutes to form a complex.

0.4 mL of the DNA-FreeStyle^{™} MAX mixture was slowly added into the cells, while the flask was rotated slowly.

The cells were cultured under the conditions of 37°C, 5% CO₂, and 120 rpm/min to 135 rpm/min, without replacing or supplementing the culture medium, and the cell culture supernatant was collected after 7 days.

### 1.3 Purification and Identification of CC1H

Sample: 25 mL of the cell culture supernatant in step 1.2 was taken, centrifuged at 4°C and 7,000 rpm for 10 min, and filtered through a syringe filter (0.22 µm). The filtered sample was loaded onto a Ni sepharose 6FF column at 2 mL/min. The column was eluted with 20 mM phosphate buffer, 500 mM NaCl, and 20 mM imidazole at pH 6.8 to equilibrate the column. Then, the column was eluted at pH 6.8 with a volume equivalent to 5 times the column volume of a linear gradient from 0% to 100% of a solution containing 20 mM phosphate buffer, 1 M NaCl, and 500 mM imidazole. The eluent was collected in fractions of 2 mL per tube.

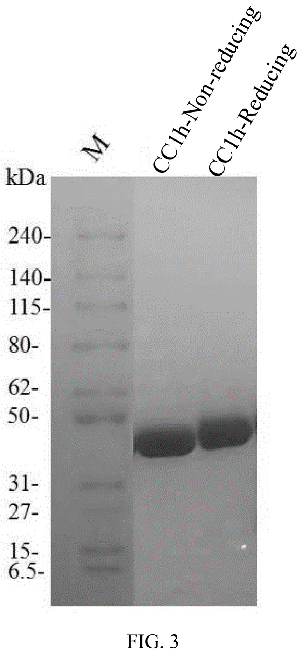

The elution peak was ultra-filtrated for 3 times in a 30 kD ultrafiltration centrifuge tube, and the solution was replaced with 50 mM phosphate buffer and 150 mM NaCl, at pH 7.4. The expression level of CC1H was quantified with the Bradford Assay and measured to be 37 mg/L. SDS-PAGE analysis was performed on CC1H, and the analysis results were illustrated in FIG. 3.

### Example 2: Biotin labeling of CC1H and Screening of Humanized Phage Library

The biotin labeling of CC1H and the screening of the humanized phage library were completed by Sanyou Biopharmaceuticals (Shanghai) Co., Ltd. Firstly, CC1H was labeled with biotin, and ELISA was used to confirm that CC1H was indeed labeled with biotin. Subsequently, the humanized recombinant antibody library constructed by Sanyou Biopharmaceuticals (Shanghai) Co., Ltd. was screened by a method of solid-phase and liquid-phase cross-screening using immune tubes and a magnetic bead screening instrument. The specific Fab antibodies against CC1H were enriched by trypsin elution. The enrichment of different output sets was detected by ELISA, and in this screening, most of the output sets with good enrichment were obtained at the ELISA level. Based on the initial screening by ELISA, a total of 1,748 clones were selected, and 834 positive clones that specifically bound to CC1H were obtained, of which 98 were molecules with unique sequences. 34 molecules (see P59365 to P59376, P59378 to P59399 in Table 1 for details) were selected for full-length construction. These 34 molecules were of IgG4 subtype.

Nine antibodies with better affinity and expression levels were selected at the phage level to construct antibodies of the IgG4SP subtype (see P62295 to P62298, P62300 to P62304 in Table 1 for details, abbreviated as SP mutants).

A total of 43 antibodies were selected for expression (their amino acid sequences and nucleotide sequences were illustrated in the "Amino Acid Sequence Table of the Present Disclosure and the Nucleotide Sequence Table of the Present Disclosure" in the summary of the present disclosure). The corresponding heavy chains and light chains were cloned between the CMV promoter and WPRE gene of pcDNA3.4. CHO cells were transiently transfected and the expression level was detected (ExpiCHO Expression System Kit, ThermoFisher, catalog number A29133). The antibody numbers and expression levels were illustrated in Table 1.

**[Table 1] Antibody Numbers and Expression Levels**

| Antibody No. | Antibody name | Amino acid sequence/nucleotide sequence of heavy chains (SEQ ID NO:) | Amino acid sequence/nucleotide sequence of light chains (SEQ ID NO:) | Subtypes of constant region | Concentration (µg/mL) |
|---|---|---|---|---|---|
| P59365 | A2 | 283/367 | 318/ 411 | IgG4 | 48.5 |
| P59366 | A5 | 284/ 368 | 319/ 412 | IgG4 | 13.5 |
| P59367 | A8 | 285/ 369 | 320/ 413 | IgG4 | 24.3 |
| P59368 | A13 | 286/ 370 | 321/ 414 | IgG4 | 20.8 |
| P59369 | A21 | 287/ 371 | 322/ 415 | IgG4 | 4.06 |
| P59370 | A22 | 288/ 372 | 323/ 416 | IgG4 | 44.2 |
| P59371 | A23 | 289/ 373 | 324/ 417 | IgG4 | 27.9 |
| P59372 | A26 | 290/ 374 | 325/ 418 | IgG4 | 43.1 |
| P59373 | A44 | 291/ 375 | 326/ 419 | IgG4 | 32.5 |
| P59374 | A53 | 292/ 376 | 327/ 420 | IgG4 | 51.4 |
| P59375 | A61 | 293/ 377 | 328/ 421 | IgG4 | 32.1 |
| P59376 | A67 | 294/ 378 | 329/ 422 | IgG4 | 37.1 |
| P59378 | A156 | 296/ 380 | 331/ 424 | IgG4 | 40.8 |
| P59379 | A187 | 297/ 381 | 332/ 425 | IgG4 | 50.2 |
| P59380 | A378 | 298/ 382 | 333/ 426 | IgG4 | 49.3 |
| P59381 | A382 | 299/ 383 | 334/ 427 | IgG4 | 38.6 |
| P59382 | B16 | 300/ 384 | 335/ 428 | IgG4 | 32.9 |
| P59383 | B20 | 301/ 385 | 336/ 429 | IgG4 | 47.8 |
| P59384 | B24 | 302/ 386 | 337/ 430 | IgG4 | 13 |
| P59385 | B29 | 303/ 387 | 338/ 431 | IgG4 | 12.9 |
| P59386 | B44 | 304/ 388 | 339/ 432 | IgG4 | 0.117 |
| P59387 | B53 | 305/ 389 | 340/ 433 | IgG4 | 17.7 |
| P59388 | B67 | 306/ 390 | 341/ 434 | IgG4 | 30.3 |
| P59389 | B83 | 307/ 391 | 342/ 435 | IgG4 | 33.3 |
| P59390 | B88 | 308/ 392 | 343/ 436 | IgG4 | 23.6 |
| P59391 | B90 | 309/ 393 | 344/ 437 | IgG4 | 8.4 |
| P59392 | B109 | 310/ 394 | 345/ 438 | IgG4 | 30.2 |
| P59393 | B114 | 311/ 395 | 346/ 439 | IgG4 | 18.2 |
| P59394 | B117 | 312/ 396 | 347/ 440 | IgG4 | 15.6 |
| P59395 | B123 | 313/ 397 | 348/ 441 | IgG4 | 30.9 |
| P59396 | B145 | 314/ 398 | 349/ 442 | IgG4 | 0.0917 |
| P59397 | B156 | 315/ 399 | 350/ 443 | IgG4 | 4.66 |
| P59398 | B201 | 316/ 400 | 351/ 444 | IgG4 | 11.3 |
| P59399 | B307 | 317/ 401 | 352/ 445 | IgG4 | 28.5 |
| P62295 | A2 (IgG4SP) | 358/ 402 | 318/ 411 | IgG4SP* | 38.5 |
| P62296 | A5 (IgG4SP) | 359/403 | 319/ 412 | IgG4SP | 17.5 |
| P62297 | A13 (IgG4SP) | 360/404 | 321/ 414 | IgG4SP | 20.9 |
| P62298 | A61 (IgG4SP) | 361/ 405 | 328/ 421 | IgG4SP | 31.3 |
| P62300 | A156 (IgG4SP) | 362/ 406 | 331/ 424 | IgG4SP | 9.44 |
| P62301 | A187 (IgG4SP) | 363/ 407 | 332/ 425 | IgG4SP | 30.4 |
| P62302 | B88 (IgG4SP) | 364/ 408 | 343/ 436 | IgG4SP | 19.9 |
| P62303 | B90 (IgG4SP) | 365/ 409 | 344/ 437 | IgG4SP | 7.18 |
| P62304 | B156 (IgG4SP) | 366/ 410 | 350/443 | IgG4SP | 35 |

| | | | | | |
|---|---|---|---|---|---|
| Note: * IgG4SP is a point mutation of IgG4, and has an S228P mutation site in the hinge region, which can enhance the stability of the antibody. | | | | | |

### Example 3: ELISA Detection of Human Anti-human PLA2R Antibody

The human anti-human PLA2R antibody after phage screening and expression in Example 2 was measured in terms of the ability of binding to CClh using the indirect ELISA method.

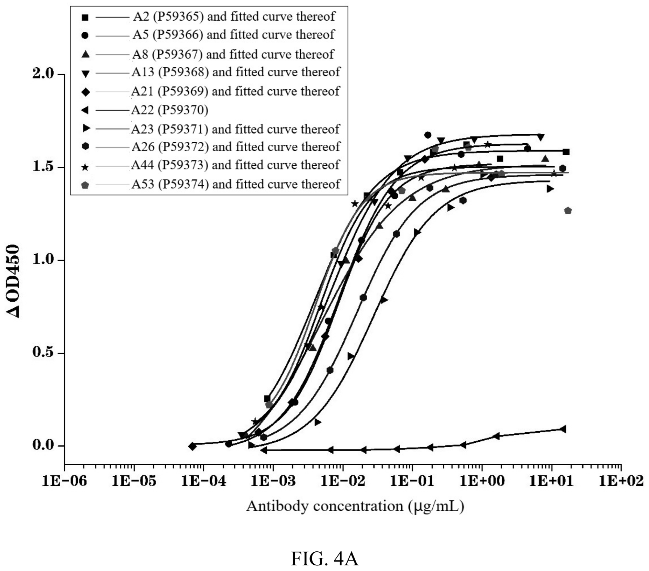

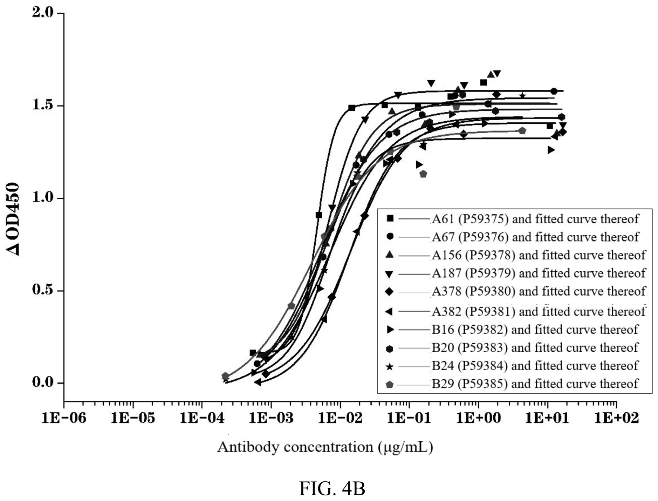

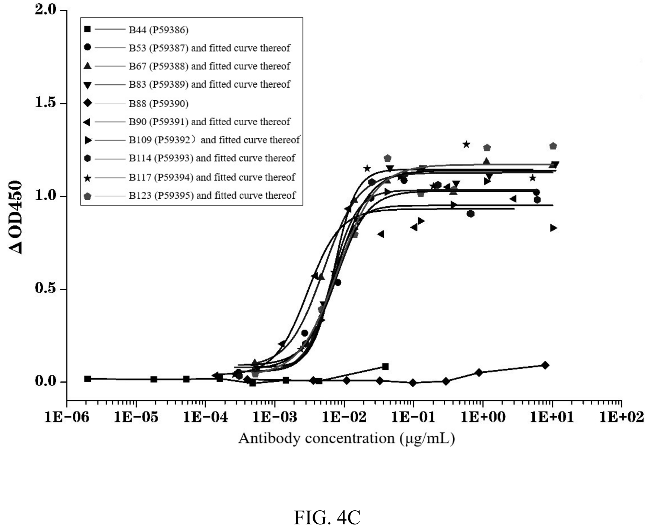

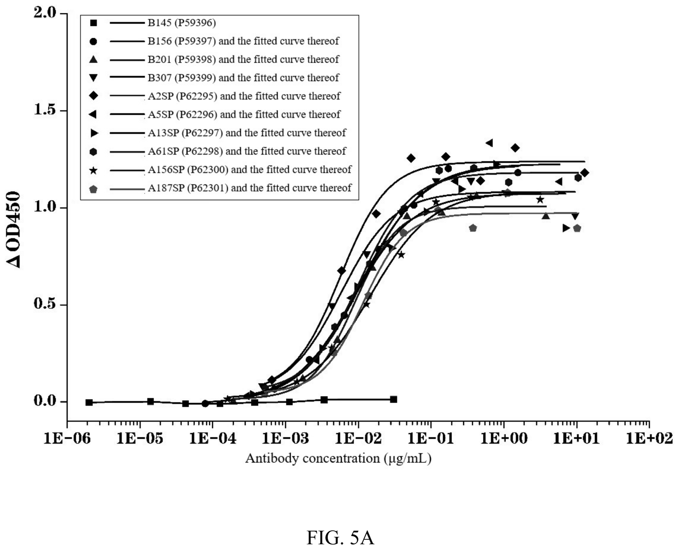

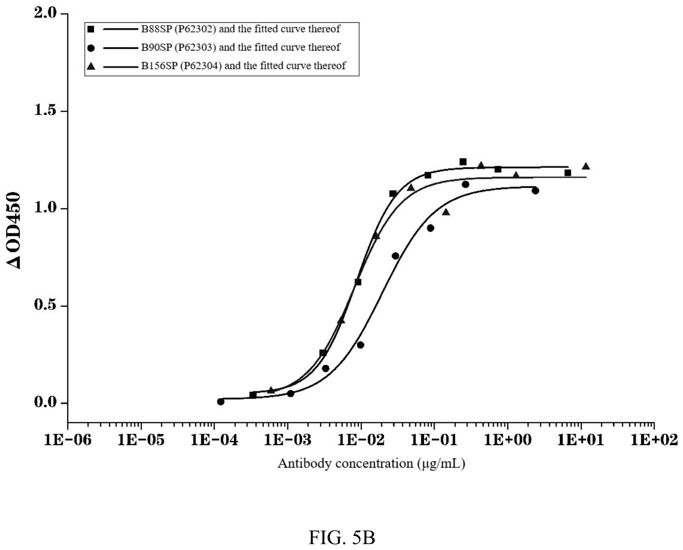

30 µL of CClh (2 µg/mL, diluted with 1*PBS) was added to the reaction wells of the ELISA plate, and the plate was sealed and incubated at 4°C overnight. The plate was washed with PBST for 3 times, and the liquid in the wells was discarded. 5% PBSM was added to block the ELISA plate at room temperature for 2 hours. The plate was washed with PBST for 3 times by adding 30 µL of Abs (diluted with 1 *PBS) to each well of the ELISA plate, and the plate was incubated at room temperature for 60 min. The plate was washed with PBST for 3 times, 30 µL of anti-human IgG Fc HRP (diluted with 1% PBSM at a ratio of 1:8000) was added to each well, and incubated at room temperature for 60 min. TMB was added and the reaction was stopped with 2M blocking solution and the OD value was detected at 450 nm. Recombinant Ipilimumab (Sanyou Biopharmaceuticals Co., Ltd.) was used as a negative control. The test results were illustrated in Table 2 as well as FIG. 4 and FIG. 5.

**[Table 2] Binding Activity of Each Antibody to CC1h**

| Protein Name | EC₅₀ (ng/mL) | Protein Name | EC₅₀ (ng/mL) |
|---|---|---|---|
| A2 (P59365) | 3.94 | B67 (P59388) | 5.02 |
| A5 (P59366) | 8.81 | B83 (P59389) | 7.71 |
| A8 (P59367) | 4.81 | B88 (P59390) | - |
| A13 (P59368) | 6.49 | B90 (P59391) | 2.96 |
| A21 (P59369) | 8.49 | B109 (P59392) | 6.38 |
| A22 (P59370) | - | B114 (P59393) | 6.52 |
| A23 (P59371) | 27.57 | B117 (P59394) | 7.17 |
| A26 (P59372) | 15.97 | B123 (P59395) | 8.09 |
| A44 (P59373) | 4.79 | B145 (P59396) | - |
| A53 (P59374) | 4.03 | B156 (P59397) | 10.48 |
| A61 (P59375) | 4.6 | B201 (P59398) | 8.88 |
| A67 (P59376) | 6.41 | B307 (P59399) | 5.74 |
| A156 (P59378) | 6.9 | A2SP (P62295) | 5.66 |
| A187 (P59379) | 6.4 | A5SP (P62296) | 11.15 |
| A378 (P59380) | 14.06 | A13SP (P62297) | 8.69 |
| A382 (P59381) | 13.24 | A61SP (P62298) | 10.26 |
| B16 (P59382) | 6.67 | A156SP (P62300) | 14.65 |
| B20 (P59383) | 5.36 | A187SP (P62301) | 11.52 |
| B24 (P59384) | 7.03 | B88SP (P62302) | 8.76 |
| B29 (P59385) | 3.85 | B90SP (P62303) | 19.5 |
| B44 (P59386) | - | B156SP (P62304) | 8.12 |
| B53 (P59387) | 7.67 | | |

The results of the ELISA indicated that four antibodies, i.e., A22 (as illustrated in FIG. 4A), B44 (as illustrated in FIG. 4C), B88 (as illustrated in FIG. 4C), and B145 (as illustrated in FIG. 5A), failed to exhibit significant binding to CC1h. The positivity exhibited in the phage library screening process may be false positives. The remaining antibodies all exhibited respective binding strengths to CC1h, with EC₅₀ ranging from 3.85 ng/mL (B29) to 27.6 ng/mL (A23). The results revealed that most antibodies specifically bound to CC1h.

### Example 4: Kinetic and thermodynamic analysis of the interaction between antibodies and CC1H

The interactions between proteins and biomolecules were detected using the Sartorius Octet ^{®} N1 molecular interaction instrument based on bio-layer interferometry (BLI), i.e., the association constant (Ka), dissociation constant (Kd), and affinity constant (KD) of the interactions between the 43 human anti-PLA2R antibodies in Example 2 and the biotin-labeled CC1H.

The specific experimental steps were as follows.

### 4.1 Biotin Labeling of CC1H

The purified CC1H protein in Example 1 was subjected to biotin labeling using the EZ-Link Sulfo-NHS-LC-Biotin (Thermo Scientific, 21335) kit, and the specific steps were as follows.

The kit was equilibrated to room temperature.

A 10 mM biotin reagent solution was prepared. Then, 90 µL of sterile double distilled water was added to fully dissolve every 0.5 mg of biotin reagent.

20 times the molar number of biotin reagent solution was added to the CC1H protein solution.

The system was incubated on ice for 2 hours, and inverted for several times to mix thoroughly.

### 4.2 Purification of CC1H-Biotin

The Biotin-labeled CC1H-Biotin was purified using a G-25 pre-packed desalting column (Borgron, EG001).

A 20 mM PBS buffer at pH 7.2 was prepared according to Table 3.

**[Table 3] Preparation Table of the PBS Buffer**

| | | | | | | |
|---|---|---|---|---|---|---|
| 200 mM PBS | | | | | | |
| Volume: | 1 L | | Preparation method | | | |
| Solvent: | Ultrapure water | | Reagents were weighed and placed in a PP beaker and added with 950 mL of ultrapure water to be completely dissolved, the pH was 7.28 and the volume was made up to 1 L. | | | |
| Storage temperature: | RT | | | | | |
| Storage period: | 1 day | | | | | |
| pH: | 7.28 | | | | | |

| Concentration | | Ingredient | | Reagent | Molecular weight | Weight |
|---|---|---|---|---|---|---|
| 3.8 mM | | Sodium Dihydrogen Phosphate | | NaH2POH2O | 138 | 0.524g |
| 16 mM | | Disodium Hydrogen Phosphate | | Na2HPO4 | 142 | 2.3g |
| 150 mM | | NaCl | | NaCl | 58.5 | 8.77g |

Equilibration: the PBS buffer was taken using a 10 mL syringe and connected to the column and syringe "drop by drop" each time to prevent bubbles; the outlet end was cut off; and a total of 25 mL of the PBS buffer passed at 5 mL/min (120 drops/min) to remove the ethanol in the column, and the effluent from the column was discarded.

Sample loading: 1.5 mL of the sample (if the volume is less than 1.5 mL, the sample was made up with the PBS buffer) was loaded using a 3 mL syringe at 5 mL/min, and the effluent from the column was discarded; and 3 mL of the PBS buffer was continuously injected, and about 2 mL in total was collected.

Column cleaning and storage: the PBS buffer passed through the column using a 10 mL syringe, then 25 mL of water passed through the column, and the effluent from the column was discarded; 25 mL of 20% ethanol passed through the column using a 10 mL syringe, and the column was stored at 4°C.

Sample storage: the collected samples were sterilized by filtration through a filter with a pore size of 0.2 µm, and stored in aliquots.

### 4.3 Determination of Protein Concentration of CC 1H-Biotin by the Bradfod Method (Beyotime, P0060)

The protein standard (5 mg/ml BSA) was completely thawed and mixed evenly, then diluted with the PBS buffer to prepare protein standards with concentrations of 0 mg/ml, 0.125 mg/ml, 0.25 mg/ml, 0.5 mg/ml, 0.75 mg/ml, 1 mg/ml, and 1.5 mg/ml and mix them thoroughly.

5 µL of protein standards with different concentrations was taken and added to the standard wells of a 96-well immunoplate (Thermo Scientific, 468667).

5 µL of the sample was taken and added to the sample wells of the 96-well immunoplate (if the volume is less than 5 µL, the volume was made up to 5 µL with the PBS buffer).

250 µL of G250 staining solution was added to each well.

A595 was measured with a microplate reader.

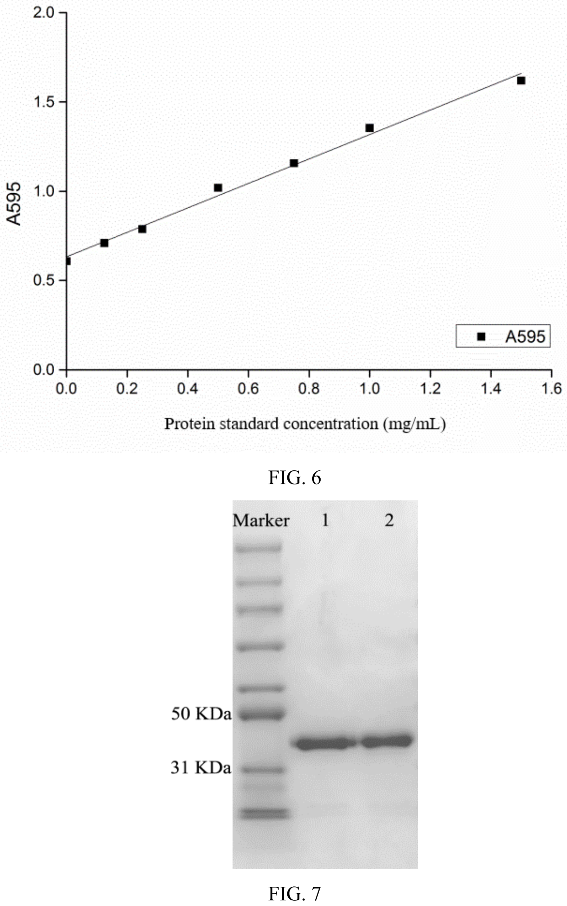

The protein concentration in the sample was calculated according to the standard curve. The standard curve was illustrated in FIG. 6, where y=0.6849x+0.6325, R²=0.9925.

Based on calculation, the protein concentration of CC1H-Biotin was 2.36 mg/mL.

### 4.4 Identification of Purity and Concentration of the Sample by Reducing/Non-reducing SDS-PAGE

The purity and concentration of the sample were identified by reducing/non-reducing SDS-PAGE. The purification effect of CC1H-Biotin was illustrated in FIG. 7, in which the lanes from left to right were CC1H-Biotin with the reducing loading buffer containing DTT and CC1H-Biotin with the non-reducing loading buffer in sequence.

The molecular weight of CC1H-Biotin was about 40 KDa, and the purity was greater than 98%.

### 4.5 Detection of Biotin Incorporation Level Using Pierce^{™} Biotin Quantitation Kit (HABA assay) (Thermo Scientific^{™}, 28005)

The ABA/Avidin premix was equilibrated to room temperature.

100 µL of ultrapure water was added to the HABA/Avidin premix tube and blew evenly with a pipette tip.

160 µL of the PBS buffer was added to the wells of a 96-well immunoplate (Thermo Scientific, 468667).

20 µL of the HABA/Avidin premix solution was added to the wells containing the PBS buffer, and A500 was measured after shaking and mixing the solution evenly.

20 µL of the biotinylated sample was added to the wells containing HABA/Avidin, and A500 was measured and kept constant for at least 15 seconds after shaking and mixing the sample evenly.

The biotin incorporation level was calculated according to the Beer's law. The results were illustrated in Table 4. After calculation, the biotin incorporation level of CC1H-Biotin was 1.80 mol Biotin/ mol protein.

**[Table 4] Biotin Incorporation Levels**

| Molar Ratio | CC1H-Biotin |
|---|---|
| Biotin/Protein | 1.80±0.33 |

### 4.6 Determination of Interaction between CC1H-Biotin and the Human Anti-PLA2R Antibody Screened from the Phage Library

Sartorius Octet ^{®} N1 molecular interaction instrument was used, the SA sensor (Sartorius, Octet^{®} Streptavidin (SA) Biosensor, 18-5019) was selected.

The sensor was immersed in a PBSTB buffer (0.02% Tween-20 and 0.1% BSA were added in the PBS buffer) to be pre-wetted for 10 min or longer, and loaded onto the molecular interaction instrument.

The sensor was successively immersed in different buffers and detected using a 5-step method: Baseline 1 (300 µL of the PBSTB buffer, for 1 min) -> Solidification (CC1H-biotin, 20 µg/mL, 300 µL, 2 min) -> Baseline 2 (PBSTB buffer, 3 min) -> Binding (300 µL of the human anti-PLA2R antibodies prepared in Example 2, which were gradient-diluted with the PBSTB buffer to 76.6 nM ~ 8.5 nM, 2 min) -> Dissociation (300 µL of the PBSTB buffer, 5 min) (as the human antibody sample contained ExpiCHO^{™} expression medium, the PBSTB buffer for the Baseline 2 and Dissociation contained the corresponding concentration of ExpiCHO^{™} expression medium (Gibco, A2910001)).

The association constant (Ka), dissociation constant (Kd), and affinity constant (KD) were calculated and illustrated in Table 5 for details.

**[Table 5] Ka, Kd, and KD values of the interaction between CC1H-Biotin and the human anti-PLA2R antibody**

| Protein No. | Protein Name | KD (M) | ka (1/Ms) | kd (1/s) | R² |
|---|---|---|---|---|---|
| P59365 | A2 | 1.04E-10 | 7.55E+05 | 7.86E-05 | 0.992 |
| P59366 | A5 | 4.55E-10 | 5.77E+05 | 2.63E-04 | 0.999 |
| P59368 | A13 | 5.80E-10 | 4.80E+05 | 2.78E-04 | 0.998 |
| P59375 | A61 | 1.78E-11 | 1.06E+06 | 1.89E-05 | 0.995 |
| P59378 | A156 | 1.58E-10 | 6.44E+05 | 1.02E-04 | 0.996 |
| P59379 | A187 | 2.04E-09 | 1.94E+05 | 3.95E-04 | 0.999 |
| P59390 | B88 | 2.11E-02 | 5.91E+04 | 1.25E+03 | 0.000 |
| P59391 | B90 | 8.407E-10 | 6.08E+05 | 5.12E-04 | 0.991 |
| P59397 | B156 | 2.42E-10 | 5.16E+05 | 1.25E-04 | 0.999 |
| P62295 | A2SP | <1E-12 | 8.72E+05 | <1E-7 | 0.989 |
| P62296 | A5SP | 1.56E-09 | 3.04E+05 | 4.75E-04 | 0.999 |
| P62297 | A13SP | 3.587E-10 | 4.06E+05 | 1.46E-04 | 0.998 |
| P62298 | A61SP | 1.26E-10 | 6.48E+05 | 8.17E-05 | 0.999 |
| P62300 | A156SP | 4.56E-10 | 3.71E+05 | 1.69E-04 | 0.999 |
| P62301 | A187SP | 1.36E-09 | 1.67E+05 | 2.27E-04 | 0.999 |
| P62302 | B88SP | 1.448E-10 | 5.56E+05 | 8.04E-05 | 0.998 |
| P62303 | B90SP | 6.594E-09 | 9.70E+04 | 6.39E-04 | 0.981 |
| P62304 | B156SP | <1E-12 | 6.54E+05 | <1E-7 | 0.997 |

As can be seen from the above table, except for P59390 (B88), other antibodies for detecting molecular interactions all have good ability of binding to CC1H and can be used as the PLA2R antibody standards.

### Example 5: Calibration of PLA2R Antibody Standard

5.1 Measurement of Conversion Relationship

The titers of A13 (P59368) and A13SP (P62297) prepared in Example 2 at different dilutions were detected using the Euroimmun anti-PLA2R (IgG) kit (Euroimmun, EA 1254-9601 G (96)). The average values of the titers were used as the conversion relationship between RU and ng, as illustrated in Table 6.

**[Table 6] Detection results of the Titers A13 and A13SP at Different Dilutions**

| Name | Dilution factor | Concentration of A13 | ΔOD450 | Converted concentration (RU/mL) | RU/ng | Mean of RU/ng |
|---|---|---|---|---|---|---|
| | | (ng/mL) | | | | |
| A13 | 5X | 525 | 3.077 | 60493.8 | 86.8 | 73.0 |
| | 25X | 105 | 1.759 | 15333.7 | 68.5 | |
| | 125X | 21 | 0.667 | 3302.4 | 63.6 | |

| | Dilution factor | Concentration of A13SP | ΔOD450 | Converted concentration (RU/mL) | RU/ng | Mean of RU/ng |
|---|---|---|---|---|---|---|
| | | (ng/mL) | | | | |
| A13SP | 5X | 525 | 3.236 | 72317.0 | 72.6 | 72.3 |
| | 25X | 105 | 1.770 | 15518.5 | 67.7 | |
| | 125X | 21 | 0.582 | 2741.9 | 76.6 | |

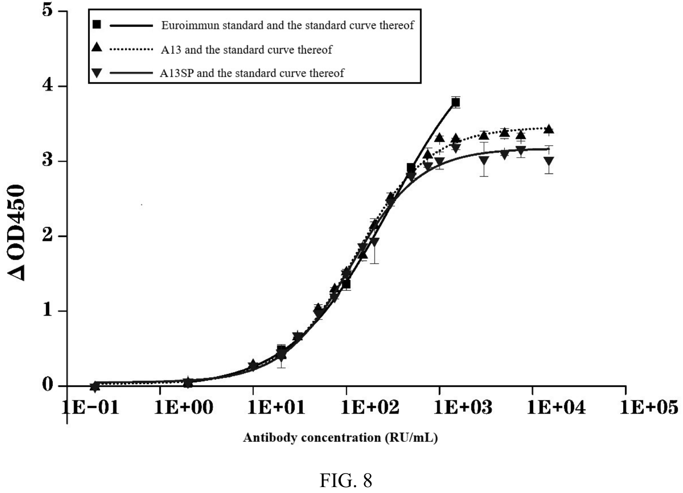

The obtained quantitative conversion relationship of A13/A13SP was about 1 RU=70 ng. A standard curve of A13/A13SP was drawn according to 1 RU=70 ng and compared with the Euroimmun standard, as illustrated in FIG. 8.

As illustrated in FIG. 8, in a very high concentration range (greater than 750 RU/mL), the A13/A13SP standard curve was slightly different from the Euroimmun standard. This may be attributed to that the kit standard was a polyclonal antibody, which was a mixture of multiple antibodies with significantly different affinities. Therefore, the ELISA curve performance of the kit standard was different from the screened monoclonal antibody.

### 2. Comparison of Standard Curves

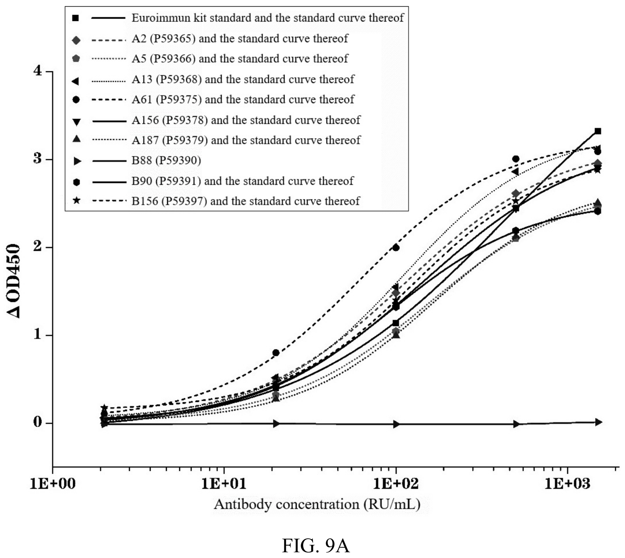

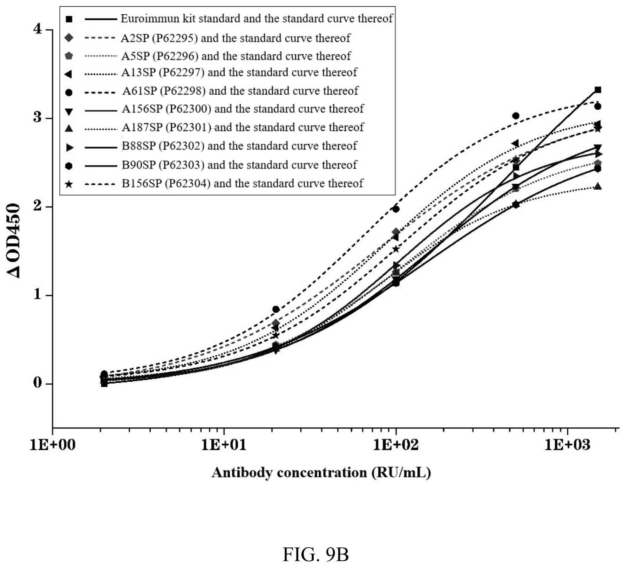

The standard curves of 9 antibodies and SP mutants thereof prepared in Example 2 obtained by screening were detected using the Euroimmun ELISA kit and compared with the kit standard (as illustrated in FIG. 9 for details).

The experimental steps were as follows.

The standards 1 to 5, human antibody samples, and negative control were added at 100 µL per well. The plate was sealed and incubated at room temperature (25°C) for 30 min.

The liquid in the wells was discarded, washed with the accompanying plate washing solution for 4 times on a plate washer (Bio-Rad, ImmunoWash^{™} 1575 plate washer), centrifuged at 600 g for 1 min on a plate shaker (Hettich, Universal 16R), and patted dry for several times.

100 µL of the accompanying enzyme conjugate was added to each well. The plate was sealed, and incubated at room temperature (25°C) for 30 min.

The plate was repeatedly washed.

100 µL of the accompanying substrate chromogenic solution was added to each well, and incubated at room temperature (25°C) in the dark for 15 min. Then, 100 µL of the accompanying stop solution was added to each well and the absorbance at 450 nm (A450) was measured.

The detection results were illustrated in FIG. 9. The concentration units of the screened antibodies in FIG. 9 was converted to RU according to 1 RU=70 ng.

As illustrated in FIG. 9A, P59390 (B88) cannot be detected by the commercial kit, which was consistent with the results of CC1h ELISA and BLI analysis. The performance of other antibodies and the SP mutants thereof was close to the standard curve of the Euroimmun ELISA kit. The screened antibody was different from the kit standard in terms of the curve shape. This may be attributed to that the kit standard was a polyclonal antibody, which was a mixture of multiple antibodies with significantly different affinities. Therefore, the ELISA curve performance of the kit standard was different from that of the screened monoclonal antibody.

Reference throughout this specification to "an embodiment", "some embodiments", "an example", "a specific example", or "some examples" means that a particular feature, structure, material, or characteristic described in connection with the embodiment or example is included in at least one embodiment or example of the present disclosure. The appearances of the above phrases in various places throughout this specification are not necessarily referring to the same embodiment or example. Further, the particular features, structures, materials, or characteristics may be combined in any suitable manner in one or more embodiments or examples. In addition, those skilled in the art can combine the different embodiments or examples and the features of the different embodiments or examples described in this specification without contradicting each other.

Although embodiments of the present disclosure have been illustrated and described above, it should be understood that the above embodiments are merely exemplary, and cannot be construed to limit the present disclosure. For those skilled in the art, changes, modifications, substitutions, and variations can be made to the embodiments without departing from the scope of the present disclosure.

## Claims

1. An antibody or an antigen-binding fragment thereof, comprising at least one CDR sequence selected from the following amino acid sequences or amino acid sequences having at least 80% identity thereto:
heavy chain variable region CDR sequences: SEQ ID NO: 1 to SEQ ID NO: 105; and
light chain variable region CDR sequences: SEQ ID NO: 106 to SEQ ID NO: 210.

2. The antibody or the antigen-binding fragment thereof according to claim 1, comprising:
a heavy chain variable region CDR1 as set forth in an amino acid sequence selected from any one of SEQ ID NO: 1 to SEQ ID NO: 35 or an amino acid sequence having at least 80% identity thereto;
a heavy chain variable region CDR2 as set forth in an amino acid sequence selected from any one of SEQ ID NO: 36 to SEQ ID NO: 70 or an amino acid sequence having at least 80% identity thereto;
a heavy chain variable region CDR3 as set forth in an amino acid sequence selected from any one of SEQ ID NO: 71 to SEQ ID NO: 105 or an amino acid sequence having at least 80% identity thereto;
a light chain variable region CDR1 as set forth in an amino acid sequence selected from any one of SEQ ID NO: 106 to SEQ ID NO: 140 or an amino acid sequence having at least 80% identity thereto;
a light chain variable region CDR2 as set forth in an amino acid sequence selected from any one of SEQ ID NO: 141 to SEQ ID NO: 175 or an amino acid sequence having at least 80% identity thereto; or
a light chain variable region CDR3 as set forth in an amino acid sequence selected from any one of SEQ ID NO: 176 to SEQ ID NO: 210 or an amino acid sequence having at least 80% identity thereto.

3. The antibody or the antigen-binding fragment thereof according to claim 1, comprising:
heavy chain variable regions selected from any one of the following groups:
| Group No. | HCDR1 (SEQ ID NO:) | HCDR2 (SEQ ID NO:) | HCDR3(SEQ ID NO:) |
|---|---|---|---|
| 1 | 1 | 36 | 71 |
| 2 | 2 | 37 | 72 |
| 3 | 3 | 38 | 73 |
| 4 | 4 | 39 | 74 |
| 5 | 5 | 40 | 75 |
| 6 | 6 | 41 | 76 |
| 7 | 7 | 42 | 77 |
| 8 | 8 | 43 | 78 |
| 9 | 9 | 44 | 79 |
| 10 | 10 | 45 | 80 |
| 11 | 11 | 46 | 81 |
| 12 | 12 | 47 | 82 |
| 13 | 13 | 48 | 83 |
| 14 | 14 | 49 | 84 |
| 15 | 15 | 50 | 85 |
| 16 | 16 | 51 | 86 |
| 17 | 17 | 52 | 87 |
| 18 | 18 | 53 | 88 |
| 19 | 19 | 54 | 89 |
| 20 | 20 | 55 | 90 |
| 21 | 21 | 56 | 91 |
| 22 | 22 | 57 | 92 |
| 23 | 23 | 58 | 93 |
| 24 | 24 | 59 | 94 |
| 25 | 25 | 60 | 95 |
| 26 | 26 | 61 | 96 |
| 27 | 27 | 62 | 97 |
| 28 | 28 | 63 | 98 |
| 29 | 29 | 64 | 99 |
| 30 | 30 | 65 | 100 |
| 31 | 31 | 66 | 101 |
| 32 | 32 | 67 | 102 |
| 33 | 33 | 68 | 103 |
| 34 | 34 | 69 | 104 |
| 35 | 35 | 70 | 105 |
; and/or
light chain variable regions selected from any one of the following groups:
| Group No. | LCDR1 (SEQ ID NO:) | LCDR2 (SEQ ID NO:) | LCDR3 (SEQ ID NO:) |
|---|---|---|---|
| 1 | 106 | 141 | 176 |
| 2 | 107 | 142 | 177 |
| 3 | 108 | 143 | 178 |
| 4 | 109 | 144 | 179 |
| 5 | 110 | 145 | 180 |
| 6 | 111 | 146 | 181 |
| 7 | 112 | 147 | 182 |
| 8 | 113 | 148 | 183 |
| 9 | 114 | 149 | 184 |
| 10 | 115 | 150 | 185 |
| 11 | 116 | 151 | 186 |
| 12 | 117 | 152 | 187 |
| 13 | 118 | 153 | 188 |
| 14 | 119 | 154 | 189 |
| 15 | 120 | 155 | 190 |
| 16 | 121 | 156 | 191 |
| 17 | 122 | 157 | 192 |
| 18 | 123 | 158 | 193 |
| 19 | 124 | 159 | 194 |
| 20 | 125 | 160 | 195 |
| 21 | 126 | 161 | 196 |
| 22 | 127 | 162 | 197 |
| 23 | 128 | 163 | 198 |
| 24 | 129 | 164 | 199 |
| 25 | 130 | 165 | 200 |
| 26 | 131 | 166 | 201 |
| 27 | 132 | 167 | 202 |
| 28 | 133 | 168 | 203 |
| 29 | 134 | 169 | 204 |
| 30 | 135 | 170 | 205 |
| 31 | 136 | 171 | 206 |
| 32 | 137 | 172 | 207 |
| 33 | 138 | 173 | 208 |
| 34 | 139 | 174 | 209 |
| 35 | 140 | 175 | 210 |

4. The antibody or the antigen-binding fragment thereof according to claim 1, comprising any one of the following groups:
| Group No. | HCDR1 (SEQ ID NO:) | HCDR2 (SEQ ID NO:) | HCDR3 (SEQ ID NO:) | LCDR1 (SEQ ID NO:) | LCDR2 (SEQ ID NO:) | LCDR3 (SEQ ID NO:) |
|---|---|---|---|---|---|---|
| 1 | 1 | 36 | 71 | 106 | 141 | 176 |
| 2 | 2 | 37 | 72 | 107 | 142 | 177 |
| 3 | 3 | 38 | 73 | 108 | 143 | 178 |
| 4 | 4 | 39 | 74 | 109 | 144 | 179 |
| 5 | 5 | 40 | 75 | 110 | 145 | 180 |
| 6 | 6 | 41 | 76 | 111 | 146 | 181 |
| 7 | 7 | 42 | 77 | 112 | 147 | 182 |
| 8 | 8 | 43 | 78 | 113 | 148 | 183 |
| 9 | 9 | 44 | 79 | 114 | 149 | 184 |
| 10 | 10 | 45 | 80 | 115 | 150 | 185 |
| 11 | 11 | 46 | 81 | 116 | 151 | 186 |
| 12 | 12 | 47 | 82 | 117 | 152 | 187 |
| 13 | 13 | 48 | 83 | 118 | 153 | 188 |
| 14 | 14 | 49 | 84 | 119 | 154 | 189 |
| 15 | 15 | 50 | 85 | 120 | 155 | 190 |
| 16 | 16 | 51 | 86 | 121 | 156 | 191 |
| 17 | 17 | 52 | 87 | 122 | 157 | 192 |
| 18 | 18 | 53 | 88 | 123 | 158 | 193 |
| 19 | 19 | 54 | 89 | 124 | 159 | 194 |
| 20 | 20 | 55 | 90 | 125 | 160 | 195 |
| 21 | 21 | 56 | 91 | 126 | 161 | 196 |
| 22 | 22 | 57 | 92 | 127 | 162 | 197 |
| 23 | 23 | 58 | 93 | 128 | 163 | 198 |
| 24 | 24 | 59 | 94 | 129 | 164 | 199 |
| 25 | 25 | 60 | 95 | 130 | 165 | 200 |
| 26 | 26 | 61 | 96 | 131 | 166 | 201 |
| 27 | 27 | 62 | 97 | 132 | 167 | 202 |
| 28 | 28 | 63 | 98 | 133 | 168 | 203 |
| 29 | 29 | 64 | 99 | 134 | 169 | 204 |
| 30 | 30 | 65 | 100 | 135 | 170 | 205 |
| 31 | 31 | 66 | 101 | 136 | 171 | 206 |
| 32 | 32 | 67 | 102 | 137 | 172 | 207 |
| 33 | 33 | 68 | 103 | 138 | 173 | 208 |
| 34 | 34 | 69 | 104 | 139 | 174 | 209 |
| 35 | 35 | 70 | 105 | 140 | 175 | 210 |

5. The antibody or the antigen-binding fragment thereof according to any one of claims 1 to 4, the antibody or the antigen-binding fragment thereof specifically recognizing a PLA2R protein.

6. The antibody or the antigen-binding fragment thereof according to any one of claims 1 to 4, the antibody or the antigen-binding fragment thereof specifically recognizing a CysR domain, a FNII domain, and a CTLD1 domain in a PLA2R protein.

7. The antibody or the antigen-binding fragment thereof according to any one of claims 1 to 4, further comprising:
at least one of a heavy chain framework region sequence and a light chain framework region sequence,
wherein at least a portion of the at least one of the heavy chain framework region sequence and the light chain framework region sequence is derived from at least one of a murine antibody, a humanized antibody, a primate-derived antibody, or a mutant thereof.

8. The antibody or the antigen-binding fragment thereof according to claim 7, wherein at least a portion of the heavy chain framework region sequence and the light chain framework region sequence is derived from a humanized antibody or a mutant thereof.

9. The antibody or the antigen-binding fragment thereof according to any one of claims 1 to 4, comprising:
a heavy chain variable region with an amino acid sequence as set forth in any one of SEQ ID NO: 211 to SEQ ID NO: 245; and/or
a light chain variable region with an amino acid sequence as set forth in any one of SEQ ID NO: 246 to SEQ ID NO: 280.

10. The antibody or antigen-binding fragment thereof according to claim 9, comprising any one of the following groups:
| Group No. | VH (SEQ ID NO:) | VL (SEQ ID NO:) | Group No. | VH (SEQ ID NO:) | VL (SEQ ID NO:) |
|---|---|---|---|---|---|
| 1 | 211 | 246 | 19 | 229 | 264 |
| 2 | 212 | 247 | 20 | 230 | 265 |
| 3 | 213 | 248 | 21 | 231 | 266 |
| 4 | 214 | 249 | 22 | 232 | 267 |
| 5 | 215 | 250 | 23 | 233 | 268 |
| 6 | 216 | 251 | 24 | 234 | 269 |
| 7 | 217 | 252 | 25 | 235 | 270 |
| 8 | 218 | 253 | 26 | 236 | 271 |
| 9 | 219 | 254 | 27 | 237 | 272 |
| 10 | 220 | 255 | 28 | 238 | 273 |
| 11 | 221 | 256 | 29 | 239 | 274 |
| 12 | 222 | 257 | 30 | 240 | 275 |
| 13 | 223 | 258 | 31 | 241 | 276 |
| 14 | 224 | 259 | 32 | 242 | 277 |
| 15 | 225 | 260 | 33 | 243 | 278 |
| 16 | 226 | 261 | 34 | 244 | 279 |
| 17 | 227 | 262 | 35 | 245 | 280 |
| 18 | 228 | 263 | | | |

11. The antibody or the antigen-binding fragment thereof according to any one of claims 1 to 4, wherein the antibody comprises at least one of a heavy chain constant region and a light chain constant region, at least a portion of the at least one of the heavy chain constant region and the light chain constant region being derived from at least one of a murine antibody, a humanized antibody, a primate-derived antibody, or a mutant thereof.

12. The antibody or antigen-binding fragment thereof according to claim 11, wherein:
the heavy chain constant region comprises a heavy chain constant region selected from IgG1, IgG2, IgG3, IgG4, IgA, IgM, IgE, or IgD; or
the light chain constant region comprises a light chain constant region selected from a κ-type light chain constant region or a λ-type light chain constant region.

13. The antibody or the antigen-binding fragment thereof according to claim 11, wherein the light chain constant region and the heavy chain constant region are both derived from a humanized IgG4 antibody or a mutant thereof.

14. The antibody or the antigen-binding fragment thereof according to claim 13, wherein the heavy chain constant region is a wild-type humanized IgG4 or a humanized IgG4 mutant.

15. The antibody or the antigen-binding fragment thereof according to claim 14, wherein the humanized IgG4 mutant has a mutation at S228P site compared to the heavy chain constant region of the wild-type humanized IgG4 antibody.

16. The antibody or the antigen-binding fragment thereof according to claim 11, wherein:
the heavy chain constant region has an amino acid sequence as set forth in SEQ ID NO: 281 or SEQ ID NO: 282; and/or
the light chain constant region has an amino acid sequence as set forth in SEQ ID NO: 357.

17. The antibody or the antigen-binding fragment thereof according to any one of claims 1 to 4, comprising:
a heavy chain with an amino acid sequence as set forth in any one of SEQ ID NO: 283 to SEQ ID NO: 317 and SEQ ID NO: 358 to SEQ ID NO: 366; or
a light chain with an amino acid sequence as set forth in any one of SEQ ID NO: 318 to SEQ ID NO: 352.

18. The antibody or the antigen-binding fragment thereof according to claim 17, comprising any one of the following groups:
| Group No. | Heavy Chain (SEQ ID NO:) | Light Chain (SEQ ID NO:) | Group No. | Heavy Chain (SEQ ID NO:) | Light Chain (SEQ ID NO:) |
|---|---|---|---|---|---|
| 1 | 283 | 318 | 23 | 305 | 340 |
| 2 | 284 | 319 | 24 | 306 | 341 |
| 3 | 285 | 320 | 25 | 307 | 342 |
| 4 | 286 | 321 | 26 | 308 | 343 |
| 5 | 287 | 322 | 27 | 309 | 344 |
| 6 | 288 | 323 | 28 | 310 | 345 |
| 7 | 289 | 324 | 29 | 311 | 346 |
| 8 | 290 | 325 | 30 | 312 | 347 |
| 9 | 291 | 326 | 31 | 313 | 348 |
| 10 | 292 | 327 | 32 | 314 | 349 |
| 11 | 293 | 328 | 33 | 315 | 350 |
| 12 | 294 | 329 | 34 | 316 | 351 |
| 13 | 295 | 330 | 35 | 317 | 352 |
| 14 | 296 | 331 | 36 | 358 | 318 |
| 15 | 297 | 332 | 37 | 359 | 319 |
| 16 | 298 | 333 | 38 | 360 | 321 |
| 17 | 299 | 334 | 39 | 361 | 328 |
| 18 | 300 | 335 | 40 | 362 | 331 |
| 19 | 301 | 336 | 41 | 363 | 332 |
| 20 | 302 | 337 | 42 | 364 | 343 |
| 21 | 303 | 338 | 43 | 365 | 344 |
| 22 | 304 | 339 | 44 | 366 | 350 |

19. The antibody or the antigen-binding fragment thereof according to any one of claims 1 to 4, wherein the antibody is selected from at least one of a monoclonal antibody, a polyclonal antibody, a multimeric antibody, and a CDR-grafted antibody.

20. The antibody or the antigen-binding fragment thereof according to any one of claims 1 to 4, wherein:
the antibody comprises at least one selected from a single-chain antibody, a Fab antibody, a Fab' antibody, a F(ab')₂ antibody, a Fv antibody, a single-chain antibody, a single-domain antibody, and a minimum recognition unit; and/or
the antigen-binding fragment of the antibody comprises at least one selected from a Fab fragment, a Fab' fragment, a F(ab)₂ fragment, a F(ab')₂ fragment, a Fv fragment, a scFv fragment, a scFv-Fc fusion protein, a scFv-Fv fusion protein, a Fv fragment, and a minimum recognition unit.

21. A nucleic acid molecule, encoding the antibody or the antigen-binding fragment thereof according to any one of claims 1 to 20,
optionally, the nucleic acid molecule is DNA.

22. An expression vector, carrying the nucleic acid molecule according to claim 21,
optionally, the expression vector is a eukaryotic expression vector or a prokaryotic expression vector, and preferably, the expression vector is a plasmid expression vector.

23. A recombinant cell,
carrying the nucleic acid molecule according to claim 21; or
expressing the antibody or the antigen-binding fragment thereof according to any one of claims 1 to 20,
optionally, the recombinant cell is obtained by introducing the expression vector according to claim 22 into a host cell,
optionally, the recombinant cell is a eukaryotic cell, and preferably, the recombinant cell is a mammalian cell.

24. Use of the antibody or the antigen-binding fragment thereof according to any one of claims 1 to 20, the nucleic acid molecule according to claim 21, the expression vector according to claim 22, or the recombinant cell according to claim 13 as a PLA2R antibody standard.

25. A kit, comprising:
the antibody or the antigen-binding fragment thereof according to any one of claims 1 to 20;
the nucleic acid molecule according to claim 21;
the expression vector according to claim 22; or
the recombinant cell according to claim 23.

26. Use of the antibody or the antigen-binding fragment thereof according to any one of claims 1 to 20, the nucleic acid molecule according to claim 21, the expression vector according to claim 22, or the recombinant cell according to claim 23 in the preparation of a kit for detecting the PLA2R antibody or diagnosing PLA2R antibody-positive membranous nephropathy.

27. Use of the antibody or the antigen-binding fragment thereof according to any one of claims 1 to 20, the nucleic acid molecule according to claim 21, the expression vector according to claim 22, or the recombinant cell according to claim 23 in the detection of the PLA2R antibody or in the diagnosis of PLA2R antibody-positive membranous nephropathy.

28. The antibody or the antigen-binding fragment thereof according to any one of claims 1 to 20, the nucleic acid molecule according to claim 21, the expression vector according to claim 22, or the recombinant cell according to claim 23, for use in the detection of the PLA2R antibody or the diagnosis of PLA2R antibody-positive membranous nephropathy.

29. A method for detecting the PLA2R antibody or diagnosing PLA2R antibody-positive membranous nephropathy, the method comprising:
determining, based on a detection result of the PLA2R antibody in a sample to be tested, a content of the PLA2R antibody in the sample to be tested by using the antibody or the antigen-binding fragment thereof according to any one of claims 1 to 20 as a PLA2R antibody standard; and
determining, based on the content of the PLA2R antibody, whether a patient corresponding to the sample to be tested suffers from the PLA2R antibody-positive membranous nephropathy.

30. A method for detecting a PLA2R antibody, the method comprising:
determining, based on a detection result of the PLA2R antibody, a content of the PLA2R antibody in a sample to be tested by using the antibody or the antigen-binding fragment thereof according to any one of claims 1 to 20 as a PLA2R antibody standard.
